# EUROPEAN PATENT SPECIFICATION

(11) **EP 1 625 403 B1**
(45) Date of publication and mention of the grant of the patent: **20.02.2008**
(21) Application number: 04730881.2
(22) Date of filing: 03.05.2004
(51) Int. Cl.: G01N 33/68

(54) **METHOD FOR THE PREDICTION, DIAGNOSIS AND DIFFERENTIAL DIAGNOSIS OF ALZHEIMER S DISEASE**
VERFAHREN ZUR VORAUSSAGE, ZUR DIAGNOSE UND ZUR VERGLEICHENDEN DIAGNOSE VON ALZHEIMER
METHODE DE PREDICTION, DIAGNOSTIC ET DIAGNOSTIC DIFFERENTIAL DE LA MALADIE D'ALZHEIMER

(30) Priority: 22.05.2003 EP 03447120; 11.06.2003 US 477621 P
(43) Date of publication of application: 15.02.2006
(73) Proprietor: Innogenetics N.V., 9052 Gent (BE)
(72) Inventor: VANDERSTICHELE, Hugo, B-9000 Ghent (BE); VANMECHELEN, Eugeen, B-9810 Nazareth - Eke (BE); BLENNOW, Kaj, S-416 79 Göteborg (SE); DE MEYER, Geert, B-9000 Ghent (BE); KOSTANJEVECKI, Vesna, B-9051 Sint Denijs-Westrem (BE)
(86) International application number: PCT/EP2004/050684
(87) International publication number: WO 2004/104597

(56) References cited:
- EP-A- 0 683 234
- WO-A-00/72880
- WO-A-02/46237
- KIM, K. S. ET AL: "Detection and quantitation of amyloid B-peptide with 2 monoclonal antibodies" NEUROSCIENCE RESEARCH COMMUNICATIONS, vol. 7, no. 2, 1990, pages 113-122, XP009017745 cited in the application
- WANG RONG ET AL: "The profile of soluble amyloid beta protein in cultured cell media. Detection and quantification of amyloid beta protein and variants by immunoprecipitation-mass spectrometry" JOURNAL OF BIOLOGICAL CHEMISTRY, AMERICAN SOCIETY OF BIOLOGICAL CHEMISTS, BALTIMORE, MD, US, vol. 271, no. 50, 13 December 1996 (1996-12-13), pages 31894-31902, XP002247951 ISSN: 0021-9258
- TEKIRIAN T.L. ET AL: "N-terminal heterogeneity of parenchymal and cerebrovascular Abeta deposits" JOURNAL OF NEUROPATHOLOGY AND EXPERIMENTAL NEUROLOGY, vol. 57, no. 1, January 1998 (1998-01), pages 76-94, XP009017746 cited in the application
- JENSEN M ET AL: "QUANTIFICATION OF ALZHEIMER AMYLOID BETA PEPTIDES ENDING AT RESIDUES 40 AND 42 BY NOVEL ELISA SYSTEMS" MOLECULAR MEDICINE, BLACKWELL SCIENCE, CAMBRIDGE, MA, US, vol. 6, no. 4, April 2000 (2000-04), pages 291-302, XP008011178 ISSN: 1076-1551
- WILTFANG, J. ET AL: "Elevation of beta-Amyloid peptide 2-42 in sporadic and familial Alzheimer's disease and its generation in PS1 knockout cells" THE JOURNAL OF BIOLOGICAL CHEMISTRY, vol. 276, no. 46, 16 November 2001 (2001-11-16), pages 42645-42657, XP001154588 cited in the application

## Description

### FIELD OF THE INVENTION

The present invention relates to the prediction, diagnosis and differential diagnosis of Alzheimer's disease. More particularly, the present invention provides a method to determine whether a subject, that does not show any clinical signs of Alzheimer's disease, has a likelihood to develop Alzheimer's disease. The present invention further provides a method for the diagnosis of AD and/or for the differential diagnosis of Alzheimer's disease versus other dementias such as dementia with Lewy bodies.

### BACKGROUND ART

Dementia is a serious, common, and rapidly growing worldwide problem associated with increased healthcare utilization. It is a major predictor of morbidity and mortality in the elderly. The occurrence of the more than 100 known diseases that produce this condition depends on age, as well as genetic factors linked to geography, race, and ethnicity. Dementia can be defined as a chronic deterioration in multiple cognitive abilities (memory, attention, judgment, etc.) that impairs the previously successful performance of activities of daily living. Its clinical profile and degree of severity are affected not only by the total quantity of neuronal loss, but by the specific locations of the underlying lesions. By far the most common forms of dementia are Alzheimer's disease (40-60% of the cases), dementia with Lewy bodies (10-20% of the cases), vascular dementia (25% and possibly contributing in up to 40% of the cases), and frontotemporal dementia (for which prevalence remains unclear) (Lowe, 2001; Leys et al., 2002; McKetih, 2002; Knopman et al., 2002). More than 33% of women and 20% of men over the age of 65 will develop dementia or milder forms of cognitive impairment in their lifetime (Yaffe and Gregg, 2002).

Alzheimer's disease (AD), the principle form and prototype of dementia, may be classified according to different criteria. From the genetic point of view, the disease can be categorized into two types: (i) less frequent, inherited familial forms (ranging from <5% for early-onset to 10-15% for late-onset forms when all genetic predisposition factors are included), and (ii) the far more common sporadic type for which no obvious inheritance patterns have been established. The sporadic form generally emerges after 65 years of age, and is thought to be multifactorial in nature.

The definitive diagnosis of AD is based on the finding of disruptively large amounts of senile plaques and neurofibrillary tangles in the affected areas of the neocortex at autopsy. Along with massive gray matter atrophy, these two types of abnormal structures are the hallmarks of the disease.

Neurofibrillary tangles consist of abnormal collections of twisted threads found inside the nerve cells. The chief components of these tangles are abnormal aggregations of a protein called tau. In the central nervous system, normal tau proteins bind and stabilize microtubules that are key constituents of the cell's internal structure. In AD, however, tau is hyperphosphorylated and twists itself into paired helical filaments: two threads of tau wound around each other. These filaments aggregate to form the telltale neurofibrillary tangles (Goedert, 1996).

The second hallmark of AD, senile plaques, consist largely of an insoluble peptide called β-amyloid (Aβ) that is surrounded by a variety of neuronal and glial processes. This amorphous, acellular material is found in the spaces between the brain's nerve cells. Aβ is a small peptide found mainly in two sizes, 40 (Aβ₄₀) and 42 (Aβ₄₂) amino acids, and in minor amounts in other sizes (see further). Aβ is known to be metabolised from the proteolytic cleavage of APP (Saido, 2000) along two pathways. In the first non-pathogenic route, the cleavage of the APP molecule by secretase enzymes leads to shorter non-amyloidogenic proteins (Aβ₃₉ or Aβ₄₀). In the second disease-related pathway, the APP cleavage yields the longer and potentially amyloidogenic Aβ₄₂ fragment that tends to misfold and aggregate into polymer chains that not only seed the plaques, but may ultimately cause neuronal damage (Selkoe, 1991).

Amyloid found in senile plaque cores is primarily Aβ₄₂ (Roher et al., 1993; Miller et al., 1993). Amyloid deposits are sparsely found in different regions of the normal aging brain, but become increasingly more abundant in the initial and subsequent stages of Alzheimer's disease. Secreted soluble Aβ is a product of normal cell metabolism and is found in various body fluids including plasma and CSF. In biological fluids, Aβ₄₀ and Aβ₄₂ are generally believed to be the major amyloid species (Seubert et al., 1992; Shoji et al., 1992; Vigo-Pelfrey et al., 1993; Ida et al., 1996). In addition to Aβ₄₀ and Aβ₄₂, however, Aβ_{37/38/39} has also been found in CSF from AD and normal subjects (Wiltfang et al., 2002).

The N-terminus is the most heterogeneous part of Aβ, being subject to truncation, racemization and isomerization. Since the discovery of Aβ as the major constituent of amyloid deposits in AD (Glenner and Wong, 1984), different N-terminally truncated and/or modified Aβ peptides have been identified in plaques of AD patients (Masters et al., 1985; Mori et al., 1992; Näslund et al., 1994; Saido et al., 1995; 1996; Iwatsubo et al., 1996; Russo et al., 1997; Tekirian et al., 1998; Lamer, 1999; Thal et al., 1999, Harigaya et al., 2000; Wiltfang et al., 2001; Kalback et al., 2002; Sergeant et al., 2003). N-terminally truncated and/or modified Aβ peptides were also identified in CSF of AD and normal subjects (Seubert et al., 1992; Vigo-Pelfrey et al., 1993; Ida et al., 1996; Lewczuk et al., 2003; 2004).

"WO 200072880 disclose sandwich ELISA using monoclonal antibody 266, specific for 13-28 Aβ and the antibody 3D6 specific for 1-5 Aβ. WO 200246237, apart from disclosing monoclonals 266 and 3D6 also further discloses the use of another monoclonal antibody 21F12 specific for 33-42 Aβ."

The second most common cause of primary dementia, after Alzheimer's disease, is dementia with Lewy bodies (DLB) accounting for no less than 10% to 20% of all cases of dementia (Lowe, 2001; McKeith, 2002). The disease-defining Lewy bodies are neuronal inclusions composed of abnormally phosphorylated neurofilaments, ubiquitin, and alpha-synuclein. These abnormalities are thought to contribute to neurological dysfunction resulting in clinical symptoms which, depending on the brain region affected, may partially resemble those associated with Alzheimer's and Parkinson's disease. Indeed, many cases of DLB are still erroneously misdiagnosed as Alzheimer's disease. However, differentiation of DLB from Alzheimer's disease is important. This is because certain neuroleptic agents, extensively prescribed for the treatment of psychotic symptoms and behavioral disturbances common in dementia, may result in severe (potentially lethal) hypersensitivity reactions in the case of DLB (Mc Keith, 2002). In addition, the pathological mechanisms that cause DLB may be fundamentally different from those in AD and, accordingly, the differentiation of DLB from AD might be of relevance for disease-modifying treatment aimed at these pathological mechanisms.

Recent clinical research has identified a transitional state between the cognitive decline found in normal aging and dementia. This prodromal state has been termed mild cognitive impairment (MCI). Persons with MCI experience consistent memory deficits earlier and to a greater extent than one would expect at their age, but remain functionally independent. Therefore, they fall short of fulfilling the accepted criteria for a positive diagnosis of one of the primary dementias (Petersen et al., 2001). Individuals who present clinically mild cognitive symptoms belong to a heterogeneous group and may not share the same fate ultimately. Some may go on to develop AD, while others may progress to another form of dementia. It is possible that some of the subjects will never progress beyond the state of MCI. Nevertheless, depending on the cohort source and definition, between 19% and 50% of individuals diagnosed with MCI progress to dementia (usually Alzheimer's disease) (Chertkow, 2002).

A very significant effort is underway to test a large number of therapeutic options for AD and other dementias. These approaches include numerous agents such as acetycholinesterase inhibitors, nonsteroidal anti-inflammatory drugs (NSAIDS), estrogen, neurotrophic agents, and even vitamins (Sramek and Cutler, 2000; Thal, 2000) as well as the reduction of senile plaque formation by the identification of secretase inhibitors and the development of an immunization model for the prevention of amyloid deposition (Conde, 2002). Since disease modifying therapy is likely to be most effective early in the course of the disease, MCI might be the optimal stage for therapeutic intervention (Chertkow, 2002). While no treatments are recommended for MCI currently, clinical trials regarding potential therapies are under way. Early diagnosis is therefore highly desirable before neurodegeneration becomes severe and widespread. Therefore, for the physician, a key challenge is to assess whether the patient has crossed the thin line between normal aging and MCI. Indeed, incipient dementia must be clearly distinguished from benign memory problems associated with age, anxiety, lack of attention, co-existent medical problems, or depression. In addition, as some of the forms of dementia are partially or even completely reversible upon treatment (e.g. dementia due to vitamin deficiency, drug intoxication, alcoholism, endocrine disorders, etc.), the importance of timeliness and accuracy in clinical diagnosis and differential diagnosis is clear.

Diagnosis of dementias such as AD, is currently based on a broad, comprehensive work-up that consists of (i) a thorough clinical evaluation (incl. physical exam, anamnesis with patient and family, medication review); (ii) a neurological examination involving neuropsychological tests and radiology; and (iii) laboratory testing (e.g., vitamin B12, folic acid, thyroid function, complete blood chemistry and blood count, etc.) (Marin et al., 2002) and exclusion of all other forms of dementia. However, ultimately, only confirmation by autopsy can unequivocally differentiate between the various dementing disorders.

The value of neuropsychological tests to help identify the presence of early memory and cognitive impairments - and quantify the degree to which the patient is affected - has been well documented (Welsh et al., 1991 ; Petersen et al., 1994; Masur et al., 1994). However, in the very early stages of the disease, delineating disease process from "normal aging" remains difficult. Even in later stages of the disease, diagnosis of AD and distinguishing AD from a number of neurodegenerative diseases associated with dementia, and especially from DLB, may also be difficult.

Diagnostic procedures need to be improved so that they can identify AD at pre-dementia stage and differentiate AD from other causes of cognitive impairment or dementia. Some aspects of the pathological cascade of AD are reflected in altered protein concentrations in body fluids. Such proteins (biomarkers or biological markers) which reflect the central pathogenic processes associated with the disease, namely the degeneration of neurons and their synapses as reflected in their defining characteristic lesions - senile plaques and neurofibrillary tangles (The Ronald and Nancy Reagan Research Institute of the Alzheimer's Association of the National Institute on Aging Working Group, 1998), can add to the accuracy of this early and differential diagnosis. Since the entire brain is in direct contact with the CSF and AD and related disorders are considered as brain diseases, the chance of finding significant differences is likely to be in this body fluid.

Two such mainstream biomarkers are CSF-Aβ protein ending at amino acid 42 (Aβ₄₂) and CSF-tau protein. For its part, the concentration of CSF-Aβ₄₂ appears to be linked with the deposition of β-amyloid into extracellular senile plaques (Motter et al., 1995; Andreasen et al., 1999a). For CSF-tau, the levels of this protein are thought to reflect neuronal and axonal degeneration (Blennow et al., 1995; Andreasen et al., 1998) or the possible formation of neurofibrillary tangles (Tapiola et al., 1997). Significantly increased CSF-tau levels (Motter et al., 1995; Vigo-Pelfrey et al., 1995) and markedly reduced CSF-Aβ₄₂ concentrations (Blennow et al., 1995; Tapiola et al., 1997) were both observed in patients with Alzheimer's disease compared to non-demented controls. Indeed, large, well-designed, multicenter studies in the United States (Galasko et al., 1998) and Japan (Kanai et al., 1998), and an international multicenter study (Hulstaert et al., 1999) consistently found that the combined use of CSF-tau and CSF-Aβ₄₂ markers resulted in a high sensitivity and specificity, and met the requirements for discriminating Alzheimer's disease from normal aging and specific neurological disorders (Sunderland et al., 2003). This clearly places their joint use well within the consensus guidelines.

Another potential breakthrough for the improved diagnosis of dementia is linked to the observation of the relative absence of abnormally phosphorylated tau protein in the brain cells of patients with non-tau dementias (eg. Parkinsons's disease, DLB) versus the high amounts found in those with Alzheimer's disease (Harrington et al., 1994). Significant differences were shown in CSF-phospho-tau levels between Alzheimer's disease and other dementias, especially DLB (Parnetti et al., 2001; Vanmechelen et al., 2001).

Wiltfang and coworkers (2001) also observed an elevated level of Aβ₍₂₋₄₂₎ in CSF from AD patients. Both CSF-Aβx-42(43) and Aβ1-42(43) levels were significantly lower in AD patients than in a control group, whereas neither CSF-Aβx-40 nor CSF-Aβ1-40 levels showed any differences between the two groups (Tamaoka et al., 1997). It was, however, not specified which Aβx-42 peptides were measured in this study.

In addition, none of the above studies assessed the behaviour of these biomarkers in cognitive impaired patients or their value in the prediction of progression of MCI patients to dementia.

Because memory impairment and MCI may be clinically and pathologically heterogeneous, biomarkers may be particularly useful for identifying subtypes. At present, there are no definite data on the usefulness of biomarkers in classifying MCI and predicting whether a patient with MCI will develop a primary dementia such as Alzheimer's disease. The measurement of CSF-tau might be used effectively for identifying incipient AD among patients diagnosed clinically as having MCI (Sunderland et al., 1999; Riemenschneider et al., 2002). However, Buerger et al. (2002) observed that high CSF-phospho-tau levels, but not CSF-tau levels correlated with cognitive decline and conversion from MCI to AD. Arai et al. (2000) observed elevated tau and phospho-tau in the CSF of patients with MCI who went on to develop AD. Okamura et al. (2002) used the CSF-CBF index to discriminate MCI that progressed to AD from MCI that did not progress to AD. The CSF-CBF index is based on CSF-tau levels divided by regional cerebral blood flow (CBF) in the posterior cingulate cortex. Several studies report on the use of CSF-Aβ₄₂ (in combination with CSF-tau and/or CSF-phospho-tau) as a biological marker for MCI progressing to AD (Andreasen et al. 1999b; Riemenschneider et al., 2002; Andreasen et al., 2003). Non-demented individuals who developed dementia during a follow-up period of three years already had lower Aβ₍₁₋₄₂₎ levels (but not Aβ₍₁₋₄₀₎) than those who remained non-demented (Skoog et al., 2003). The level of β-amyloid forms seems to decrease constantly from the onset of AD. Although the ratio Aβ₄₀/Aβ₄₂ showed an increase with the progression of AD, its increase already had started before the appearance of the clinical symptoms of AD (Kanai et al. 1998).

The above shows that certain biomarkers are abnormally altered before conversion to clinical dementia and they could be promising as potential early markers to identify MCI patients that will develop primary dementia (especially Alzheimer's disease).

Such findings point towards the future combined use of such biomarkers, together with other appropriate tests, as part of a broad diagnostic work-up for MCI, Alzheimer's disease, and other dementias. It is therefore of extreme importance that additional biomarkers are identified that are already altered in patients before their conversion to clinical dementia and that could aid in the diagnosis of AD and prediction of cognitive impaired patients that will progress to dementias such as AD.

### SUMMARY OF THE INVENTION

The present invention provides methods and diagnostic kits for the prediction, diagnosis and differential diagnosis of Alzheimer's disease (AD). More particularly, the present invention provides a method and a diagnostic kit to determine whether a subject that at the time of sampling does not show any clinical signs of AD, has a likelihood to develop AD. The methods and diagnostic kits of the present invention are based on the determination of x/y ratios in body fluid samples obtained from the subjects under diagnosis whereby:
- x is the level of Aβ peptides capable of forming an immunological complex with an antibody that recognizes an epitope of the Aβ peptide containing the first amino acid (D; aspartic acid), the second amino acid (A; alanine), and/or the third amino acid (E; glutamic acid) of the Aβ peptide;
- y is the level of Aβ peptides capable of forming an immunological complex with an antibody that recognizes an epitope of the Aβ peptide not containing the first amino acid (D; aspartic acid), the second amino acid (A; alanine), and/or the third amino acid (E; glutamic acid) of the Aβ peptide;

The x/y ratios in body fluid samples obtained from subjects that, at the time of sampling did not show any clinical signs of AD and that later developed AD, appeared to be significantly altered compared to the x/y ratios in body fluid samples obtained from subjects that at the time of sampling did not show any clinical signs of AD and that did not develop AD. Accordingly, the present invention provides a method and a diagnostic kit to determine whether a subject has a likelihood to develop AD, comprising the following steps:
(a) Determining, in a body fluid sample obtained from said subject, the ratio x/y, wherein:
   - x is the level of Aβ peptides capable of forming an immunological complex with an antibody that recognizes an epitope of the Aβ peptide containing the first amino acid (D; aspartic acid), the second amino acid (A; alanine), and/or the third amino acid (E; glutamic acid) of the Aβ peptide;
   - y is the level of Aβ peptides capable of forming an immunological complex with an antibody that recognizes an epitope of the Aβ peptide not containing the first amino acid (D; aspartic acid), the second amino acid (A; alanine), and/or the third amino acid (E; glutamic acid) of the Aβ peptide;
(b) Comparing the ratio x/y obtained in (a) with a range of x/y ratios previously defined as characteristic for body fluid samples obtained from subjects that at the time of sampling did not show clinical signs of AD and that later developed AD, and with a range of x/y ratios previously defined as characteristic for body fluid samples obtained from subjects that at the time of sampling did not show clinical signs of AD and that did not develop AD;
(c) Determining, from the comparison in step (b), whether the subject has a likelihood to develop AD, whereby a ratio x/y in a range previously defined as characteristic for body fluid samples obtained from subjects that at the time of sampling did not show clinical signs of AD and that later developed AD, is an indication that said subject has a likelihood to develop AD; and whereby a ratio x/y in a range previously defined as characteristic for body fluid samples obtained from subjects that at the time of sampling did not show clinical signs of AD and that did not develop AD, is an indication that said subject does not have a likelihood to develop AD.

The method and diagnostic kit of the present invention can be carried out on body fluid samples obtained from subjects that do not show any clinical signs of memory impairment, MCI, or dementia. In a preferred embodiment, the method of the present invention is carried out on body fluid samples obtained from subjects with memory impairment or subjects that are clinically diagnosed as having MCI.

The present invention also provides methods and diagnostic kits for the diagnosis of subjects suffering from AD and/or for the differential diagnosis of subjects suffering from AD versus subjects suffering from other dementias such as DLB. The methods and diagnostic kits of the present invention are based on the finding that the x/y ratios in a body fluid samples obtained from subjects suffering from AD are significantly altered compared to the x/y ratios in body fluid samples obtained from control subjects and subjects suffering from DLB. Accordingly, the present invention provides methods and diagnostic kits for the diagnosis of a subject suffering from AD and/or for the differential diagnosis of a subject suffering from AD versus a subject suffering from another dementia such as DLB, comprising the following steps:
(a) Determining, in a body fluid sample obtained from said subject, the ratio x/y, wherein:
   - x is the level of Aβ peptides capable of forming an immunological complex with an antibody that recognizes an epitope of the Aβ peptide containing the first amino acid (D; aspartic acid), the second amino acid (A; alanine), and/or the third amino acid (E; glutamic acid) of the Aβ peptide;
   - y is the level of Aβ peptides capable of forming an immunological complex with an antibody that recognizes an epitope of the Aβ peptide not containing the first amino acid (D; aspartic acid), the second amino acid (A; alanine), and/or the third amino acid (E; glutamic acid) of the Aβ peptide;
(b) Comparing the ratio x/y obtained in (a) with a range of x/y ratios previously defined as characteristic for body fluid samples obtained from subjects diagnosed as suffering from AD, with a range of x/y ratios previously defined as characteristic for body fluid samples obtained from control subjects, and with a range of x/y ratios previously defined as characteristic for body fluid samples obtained from subjects diagnosed as suffering from another dementia such as DLB;
(c) Determining, from the comparison in step (b), whether or not the subject is suffering from AD or from another dementia such as DLB, whereby a ratio x/y in a range previously defined as characteristic for body fluid samples obtained from subjects diagnosed as suffering from AD is an indication that said subject is suffering from AD; whereby a ratio x/y in a range previously defined as characteristic for body fluid samples obtained from control subjects is an indication that said subject is not suffering from AD; and whereby a ratio x/y in a range previously defined as characteristic for body fluid samples obtained from subjects diagnosed as suffering from another dementia such as DLB is an indication that said subject is suffering from another dementia such as DLB.

The present invention provides for use of a set of antibodies for immunologically determining the ratio of X/Y in the methods as described above comprising at least a first antibody that specifically recognizes an epitope of the Aβ peptide containing the first amino acid (D; aspartic acid), the second amino acid, (A; alanine) and/or the third amino acid (E; glutamic acid) of the Aβ peptide for detecting X and a second antibody that recognizes an epitope of the A peptide not containing the first amino acid (D; aspartic acid), the second amino acid (A; alanine), and/or the third amino acid (E; glutamic acid) of the Aβ peptide for determining Y.

In a preferred embodiment of the present invention, x is the level of Aβ peptides capable of forming an immunological complex with an antibody that recognizes an epitope of the Aβ peptide containing the first amino acid (D; aspartic acid) of the Aβ peptide. Even more preferably, x is the level of Aβ peptides capable of forming an immunological complex with the monoclonal antibody 3D6, BAN-50, and/or Anti-NI (D). In another preferred embodiment of the present invention, y is the level of Λβ peptides capable of forming an immunological complex with an antibody that recognizes an epitope of the Aβ peptide not containing the first amino acid (D; aspartic acid) of the Aβ peptide. Even more preferably, y is the level of Aβ peptides capable of forming an immunological complex with an antibody that recognizes an epitope different from the 3D6, BAN-50, and/or Anti-N1(D) epitope, such as the monoclonal antibody 4G8, the monoclonal antibody 6E10, and/or the monoclonal antibody 10H3. In another preferred embodiment, x is the level of Aβ_{(1-C)} peptides and y is the level of Aβ_{(N-C)} peptides. In another preferred embodiment, x is the level of Aβ₍₁₋₄₂₎ and/or Aβ₍₁₋₄₃₎ peptides and y is the level of Aβ_{(N-42)} and/or Aβ_{(N-43)} peptides. In another preferred embodiment, x is the level of Aβ₍₁₋₄₂₎ peptides and y is the level of Aβ_{(N-42)} peptides.

The methods and diagnostic kits of the present invention can be used on any body fluid sample obtained from a subject. In a preferred embodiment, the body fluid sample is a cerebrospinal fluid sample or a plasma or serum sample.

The methods and diagnostic kits of the present invention can also be used in the treatment follow up of a subject that has a likelihood to develop AD or of a subject that is diagnosed as suffering from AD.

The present invention further provides for use of a diagnostic kit comprising a first antibody that specifically recognizes an epitope of the Aβ peptide containing the first amino acid (D; aspartic acid), the second amino acid (A; alanine), and/or the third amino acid (E; glutamic acid) of the Aβ peptide and a second antibody that recognizes an epitope of the Aβ peptide not containing the first amino acid (D; aspartic acid), the second amino acid (A; alanine), and/or the third amino acid (E; glutamic acid) of the Aβ peptide for immunologically determining the ratio of X/Y in the above described method.

### FIGURE LEGENDS

**Figure 1****.** Partial amino acid sequence of amyloid precursor protein (APP) comprising the amino acid sequence of Aβ. The α, β and γ cleavage sites in APP are indicated. Further indicated are the epitopes of some of the monoclonal antibodies used in the methods and diagnostic kits of the present invention.
**Figure 2****.** Level x (pg peptide equivalents/ml) of specific Aβ peptides capable of binding with the monoclonal antibody 3D6. The level is measured in CSF samples obtained from the following patient groups: moderate AD (modAD), severe AD (sevAD), mild AD (mildAD), patients with memory complaints who progressed to AD (Cog-AD), patients with memory complaints who did not develop into AD (Cog), patients suffering from dementia with Lewy bodies (DLB), patients suffering from Parkinson's disease (PD), and control subjects (C).
**Figure 3****.** Level y (pg peptide equivalents/ml) of specific Aβ peptides capable of binding with the monoclonal antibody 6E10. The level is measured in CSF samples obtained from the following patient groups: moderate AD (modAD), severe AD (sevAD), mild AD (mildAD), patients with memory complaints who progressed to AD (Cog-AD), patients with memory complaints who did not develop into AD (Cog), patients suffering from dementia with Lewy bodies (DLB), patients suffering from Parkinson's disease (PD), and control subjects (C).
**Figure 4****.** Level y (pg peptide equivalents/ml) of specific Aβ peptides capable of binding with the monoclonal antibody 4G8. The level is measured in CSF samples obtained from the following patient groups: moderate AD (modAD), severe AD (sevAD), mild AD (mildAD), patients with memory complaints who progressed to AD (Cog-AD), patients with memory complaints who did not develop into AD (Cog), patients suffering from dementia with Lewy bodies (DLB), patients suffering from Parkinson's disease (PD) and control subjects (C).

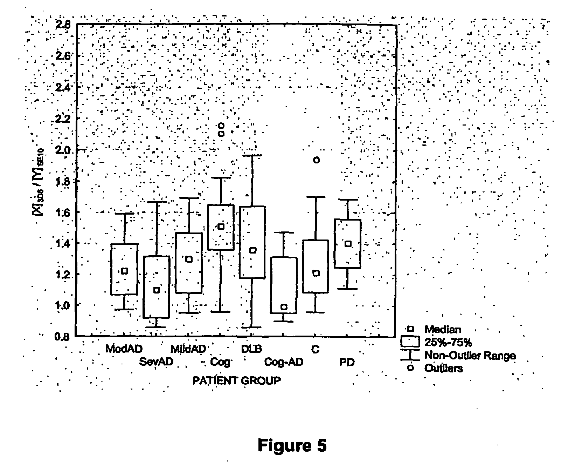

**Figure 5****.** Ratio x/y wherein x is the level of specific Aβ peptides capable of binding with the monoclonal antibody 3D6 and wherein y is the level of specific Aβ peptides capable of binding with the monoclonal antibody 6E10. The ratio x/y is determined in CSF samples obtained from the following patient groups: moderate AD (modAD), severe AD (sevAD), mild AD (mildAD), patients with memory complaints who progressed to AD (Cog-AD), patients with memory complaints who did not develop into AD (Cog), patients suffering from dementia with Lewy bodies (DLB), patients suffering from Parkinson's disease (PD), and control subjects (C).

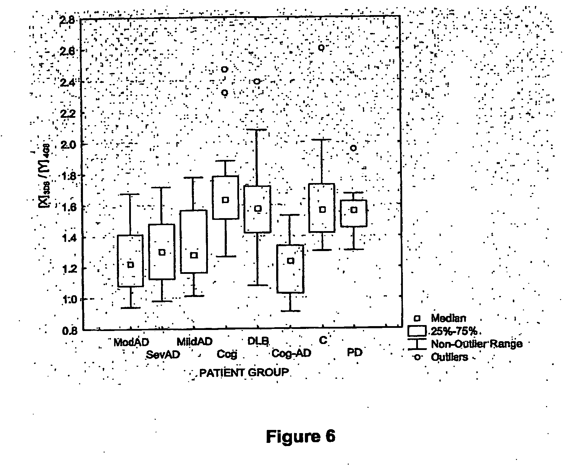

**Figure 6****.** Ratio x/y wherein x is the level of specific Aβ peptides capable of binding with the monoclonal antibody 3D6 and wherein y is the level of specific Aβ peptides capable of binding with the monoclonal antibody 4G8. The ratio x/y is determined in CSF samples obtained from the following patient groups: moderate AD (modAD), severe AD (sevAD), mild AD (mildAD), patients with memory complaints who progressed to AD (Cog-AD), patients with memory complaints who did not develop into AD (Cog), patients suffering from dementia with Lewy bodies (DLB), patients suffering from Parkinson's disease (PD), and control subjects (C).
**Figure 7**. Immunological binding of different Aβ peptides, Aβ₍₁₋₄₂₎, Aβ₍₂₋₄₂₎, Aβ₍₃₋₄₂₎, Aβ₍₄₋₄₂₎, Aβ₍₅₋₄₂₎, Aβ₍₈₋₄₂₎, Aβ₍₉₋₄₂₎, in ELISA with the monoclonal antibody 21F12 as capturing antibody and the monoclonal antibody 3D6 as detector antibody.
**Figure 8****.** Immunological binding of different Aβ peptides, Aβ₍₁₋₄₂₎, Aβ₍₂₋₄₂₎, Aβ₍₃₋₄₂₎, Aβ₍₄₋₄₂₎, Aβ₍₅₋₄₂₎, Aβ₍₈₋₄₂₎, Aβ₍₉₋₄₂₎, in ELISA with the monoclonal antibody 21F12 as capturing antibody and the monoclonal antibody 6E10 as detector antibody.
**Figure 9****.** Immunological binding of different Aβ peptides, Aβ₍₁₋₄₂₎, Aβ₍₂₋₄₂₎, Aβ₍₃₋₄₂₎, Aβ₍₄₋₄₂₎, Aβ₍₅₋₄₂₎, Aβ₍₈₋₄₂₎, Aβ₍₉₋₄₂₎, in ELISA with the monoclonal antibody 21F12 as capturing antibody and the monoclonal antibody 4G8 as detector antibody.
**Figure 10****.** Immunological binding of thet Aβ peptides Aβ₍₁₋₄₂₎ and Aβ₍₂₋₄₂₎ in a multiparameter immuno-assay with the monoclonal antibodies 3D6, 6E10 and 4G8 as capturing antibodies and the monoclonal antibody 21F12 as detector antibody.
**Figure 11****.** Level x (Luminex Units) of specific Aβ peptides capable of binding with the monoclonal antibody 3D6. The level is measured in CSF. samples obtained from subjects suffering from AD and control subjects.
**Figure 12****.** Level y (Luminex Units) of specific Aβ peptides capable of binding with the monoclonal antibody 4G8. The level is measured in CSF samples obtained from subjects suffering from AD and control subjects.
**Figure 13****.** Ratio x/y wherein x is the level of specific Aβ peptides capable of binding with the monoclonal antibody 3D6 and y is the level of specific Aβ peptides capable of binding with the monoclonal antibody 4G8. The ratio x/y is determined in CSF samples obtained from subjects suffering from AD and control subjects.

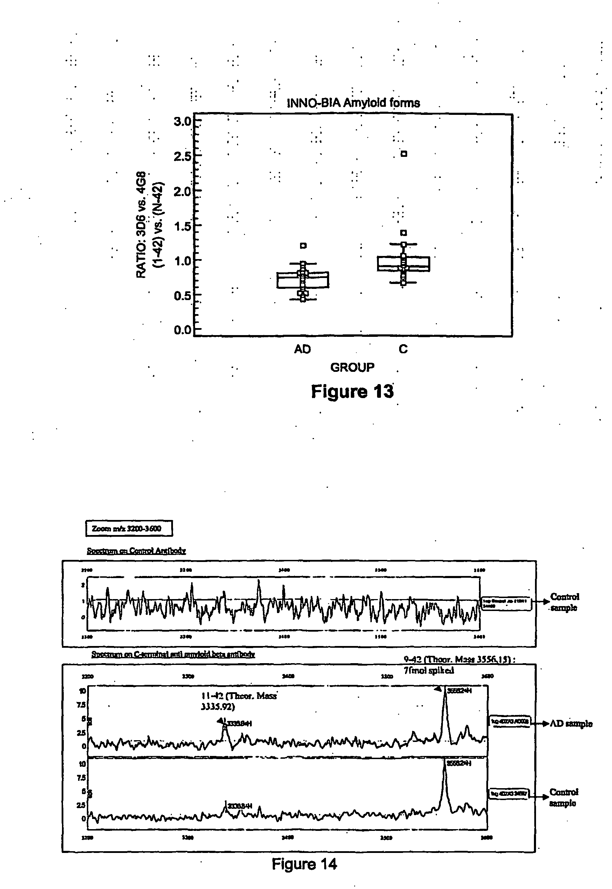

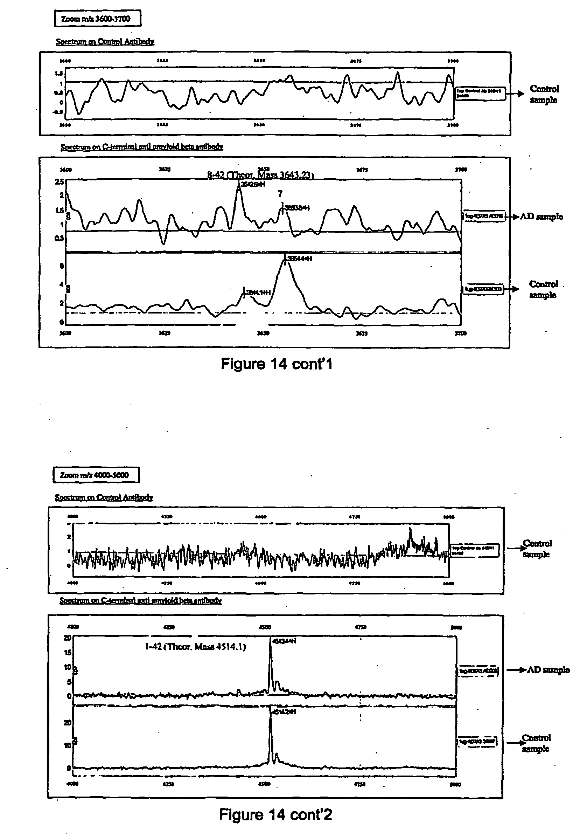

**Figure 14****.** SELDI-TOF spectra of analyzed Aβ₍₄₂₎ peptides in CSF samples of AD and controls. The Aβ₍₄₂₎ peptides were immunocaptured on 4D7A3. The measured molecular masses of peptides are shown. In the experiment external calibration was performed with Dynaphorin (Mr=2147,50 Da), human ACTH₍₁₋₂₄₎ (2933,50), Bovine insulin betha-chain (3495,94), and human insulin (5807,65). On this bases mass accuracy was calculated for the Aβ₍₄₂₎ peptide peak with theoretical mass of 4514,1 Da. The m/z value measured by SELDI-TOF was 4512,069 Da (STDEV 1,193456, %CV 0,02645) giving the accuracy for this experiment of 450 ppm.
**Figure 15****.** SELDI-TOF spectra of analyzed oxidized Aβ₍₄₂₎ peptides in human CSF samples.
**Figure 16**. Level (peak instensity) of Aβ₍₁₋₄₂₎ peptides measured in CSF samples obtained from subjects suffering from AD and control subjects.
**Figure 17****.** Level (peak intensity) of Aβ₍₁₁₋₄₂₎ peptides measured in CSF samples obtained from subjects suffering from AD and control subjects.
**Figure 18****.** Ratio of Aβ₍₁₋₄₂₎/Aβ₍₁₁₋₄₂₎ peptides measured in CSF samples obtained from subjects suffering from AD and control subjects.

### DETAILED DESCRIPTION OF THE INVENTION

The present invention provides methods for the prediction, diagnosis and differential diagnosis of AD. More particularly, the present invention relates to a method to determine whether a subject that does not show any clinical signs of AD has a likelihood to develop AD. The method of the invention comprises the following steps:
(a) Determining, in a body fluid sample obtained from said subject, the ratio x/y, wherein:
   - x is the level of Aβ peptides capable of forming an immunological complex with an antibody that recognizes an epitope of the Aβ peptide containing the first amino acid (D; aspartic acid), the second amino acid (A; alanine), and/or the third amino acid (E; glutamic acid) of the Aβ peptide;
   - y is the level of Aβ peptides capable of forming an immunological complex with an antibody that recognizes an epitope of the Aβ peptide not containing the first amino acid (D; aspartic acid), the second amino acid (A; alanine), and/or the third amino acid (E; glutamic acid) of the Aβ peptide;
(b) Comparing the ratio x/y obtained in (a) with a range of x/y ratios previously defined as characteristic for body fluid samples obtained from subjects that at the time of sampling did not show clinical signs of AD and that later developed AD, and with a range of x/y ratios previously defined as characteristic for body fluid samples obtained from subjects that at the time of sampling did not show clinical signs of AD and that did not develop AD;
(c) Determining, from the comparison in step (b), whether the subject has a likelihood to develop AD, whereby a ratio x/y in a range previously defined as characteristic for body fluid samples obtained from subjects that at the time of sampling did not show clinical signs of AD and that later developed AD, is an indication that said subject has a likelihood to develop AD; and whereby a ratio x/y in a range previously defined as characteristic for body fluid samples obtained from subjects that at the time of sampling did not show clinical signs of AD and that did not develop AD, is an indication that said subject does not have a likelihood to develop AD.

The present invention is based on the finding that this ratio x/y, as defined above, was significantly decreased in body fluid samples obtained from subjects that at the time of sampling did not show any clinical signs of AD and that later developed AD, compared to this x/y ratio in body fluid samples obtained from subjects that at the time of sampling did not show any clinical signs of AD and that did not develop AD. The indication that this ratio x/y is significantly altered in body fluid samples of subjects that will develop AD forms a basis for the development of a diagnostic test for determining whether a subject has a likelihood to develop AD. At present, delineating disease process from "normal aging" still remains difficult. Early diagnosis of a disease process, before clinical signs of dementia are present is, however, highly desirable, since disease modifying therapy is likely to be most effective early in the course of disease

The subject under diagnosis in the above method can be any subject that does not show clinical signs of dementia. The subject under diagnosis may be a non-human subject such as (but not limited to) a cow, a pig, a sheep, a goat, a horse, a monkey, a rabbit, a hare, a chicken, a dog, a cat, a mouse, a rat, an elk, a deer, a tiger, a zebrafish, a pufferfish, a fly, a worm or *C. elegans.* More preferably, the subject is a primate. Even more preferably, the subject is a human. In a preferred embodiment, the subject is a human who does not show any clinical signs of AD according to the NINCDS-ADRDA criteria (McKhann et al., 1984), the ICD-10 criteria (World Health Organization, 1992), and/or the DSM-IV criteria (American Psychiatric Association, 1994). The term "AD" shall mean Alzheimer's disease.

The above method can be carried out on body fluid samples obtained from subjects that, in addition to the absence of clinical signs of dementia or AD, also do not show any clinical signs of memory impairment or MCI. In a preferred embodiment of the invention, however, the above method is carried out on body fluid samples obtained from subjects suffering from memory impairment or subjects that suffer from MCI. Clinical diagnosis of memory impairment and MCI is currently done according to Petersen et al. (1999), Palmer et al. (2003) and/or Wahlund et al. (2003).

In the present invention, the terms "develop AD", "progress to AD", "will have AD", etc. are used interchangeably and mean that the subject at the time of sampling of the body fluid (on which the method of the present invention is carried out), does not show any clinical signs of AD, but that said subject shows clinical signs of AD within a period of maximum 5 years, preferably maximum 3 years, and most preferably within 1 year after the sampling of said body fluid
The terms. "likelihood", "risk", "susceptibility", "predisposition", "prognosis" or "prediction" are interchangeable and are used with respect to the probability of developing AD.

The present invention also relates to a method for the diagnosis of a subject suffering from AD and/or for the differential diagnosis of subjects suffering from AD versus subjects suffering from other dementias such as DLB. The method of the invention comprises the following steps:
(a) Determining, in a body fluid sample obtained from said subject, the ratio x/y, wherein:
   - x is the level of Aβ peptides capable of forming an immunological complex with an antibody that recognizes an epitope of the Aβ peptide containing the first amino acid (D; aspartic acid), the second amino acid (A; alanine), and/or the third amino acid (E; glutamic acid) of the Aβ peptide;
   - y is the level of Aβ peptides capable of forming an immunological complex with an antibody that recognizes an epitope of the Aβ peptide not containing the first amino acid (D; aspartic acid), the second amino acid (A; alanine), and/or the third amino acid (E; glutamic acid) of the Aβ peptide;
(b) Comparing the ratio x/y obtained in (a) with a range of x/y ratios previously defined as characteristic for body fluid samples obtained from subjects diagnosed as suffering from AD, with a range of x/y ratios previously defined as characteristic for body fluid samples obtained from control subjects, and with a range of x/y ratios previously defined as characteristic for body fluid samples obtained from subjects diagnosed as suffering from another dementia such as DLB;
(c) Determining, from the comparison in step (b), whether or not the subject is suffering from AD or from another dementia such as DLB, whereby a ratio x/y in a range previously defined as characteristic for body fluid samples obtained from subjects diagnosed as suffering from AD is an indication that said subject is suffering from AD; whereby a ratio x/y in a range previously defined as characteristic for body fluid samples obtained from control subjects is an indication that said subject is not suffering from AD; and whereby a ratio x/y in a range previously defined as characteristic for body fluid samples obtained from subjects diagnosed as suffering from another dementia such as DLB is an indication that said subject is suffering from another dementia such as DLB.

The above method is based on the finding that the x/y ratios, as defined above, in body fluid samples obtained from subjects that suffer from AD are significantly decreased compared to the x/y ratios in body fluid samples obtained from control subjects and from subjects that suffer from DLB. The indication that the x/y ratio in body fluids samples obtained from subjects suffering from AD is significantly altered compared to the x/y ratio in body fluid samples obtained from control subjects and from subjects suffering from DLB forms a basis for the development of a diagnostic test for the diagnosis of AD and/or the differential diagnosis of AD versus other dementias such as DLB. The term "diagnosis" means that subjects suffering from a certain neurological disease are discriminated from subjects not suffering from said neurological disease. In the present invention, subjects suffering from AD are discriminated from control subjects. The term "differential diagnosis" means that subjects suffering from a certain neurological disease are discriminated from subjects suffering from another neurological disease. In the present invention, subjects suffering from AD are discriminated from subjects suffering from another dementia such as DLB. Although criteria for the diagnosis of AD (McKhann et al., 1984; World Health Organization, 1992; American Psychiatric . Association, 1994) and other dementias such as DLB (McKeith et al., 1996) are currently available, they may lack sufficient detail to discriminate these dementias such as DLB from AD (McKeith, 2002). The differentiation of subjects suffering from AD from subjects suffering from DLB remains a major problem. Ultimately, only autopsy can unequivocally differentiate between the various dementing disorders. In view of potential differing therapeutic implications for subjects suffering from AD and subjects suffering from DLB, this differentiation is, however, of extreme importance. The method of the present invention provides an additional tool in the differential diagnosis of subjects suffering from AD versus subjects suffering from DLB. The term "DLB" shall mean dementia with Lewy bodies.

The subject under diagnosis in the above method can be any subject that shows clinical signs of dementia. The subject under diagnosis may be a non-human subject such as (but not limited to) a cow, a pig, a sheep, a goat, a horse, a monkey, a rabbit, a hare, a dog, a cat, a mouse, a rat, an elk, a deer or a tiger. More preferably, the subject is a primate. Even more preferably, the subject is a human. Control subjects are subjects without histories, symptoms or signs of psychiatric or neurological disease.

The methods of the present invention are based on the detection of the ratio x/y in body fluid samples obtained from the subject under diagnosis. The term "body fluid" refers to all fluids that are present in the body including but not limited to blood, lymph, urine, and cerebrospinal fluid (CSF), containing Aβ peptides. The blood sample may be a plasma sample or a serum sample. It could be possible that x is determined in a certain body fluid sample while y is determined in another body fluid sample. In a preferred embodiment, however, x and y are determined in the same body fluid sample. In a preferred embodiment of the present invention the x/y ratio is determined in a cerebrospinal fluid sample taken from the subject The term "cerebrospinal fluid" or "CSF" is intended to include whole cerebrospinal fluid or derivatives of fractions thereof well known to those skilled in the art. Thus, a cerebrospinal fluid sample can include various fractionated forms of cerebrospinal fluid or can include various diluents added to facilitate storage or processing in a particular assay. Such diluents are well known to those skilled in the art and include various buffers, preservatives, and the like.

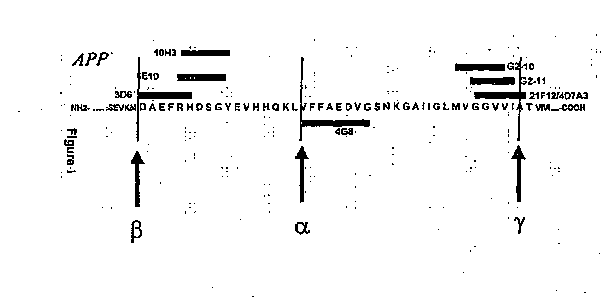

Within the ratio x/y of the present invention, the numerator (x) is defined as the level of Aβ peptides capable of forming an immunological complex with an antibody that recognizes an epitope of the Aβ peptide containing the first amino acid (D; aspartic acid), the second amino acid (A; alanine), and/or the third amino acid (E; glutamic acid) of the Aβ peptide. Within the ratio x/y of the present invention, the denominator (y) is defined as the level of Aβ peptides capable of forming an immunological complex with an antibody that recognizes an epitope of the Aβ peptide not containing the first amino acid (D; aspartic acid), the second amino acid (A; alanine), and/or the third amino acid (E; glutamic acid) of the Aβ peptide. Aβ peptide, Aβ, β-amyloid peptide, or A4 peptide are used interchangeably throughout the present invention and refer to a peptide of 37-43 amino acids (Aβ₃₇, Aβ₃₈, Aβ₃₉, Aβ₄₀, Aβ₄₁, Aβ₄₂ or Aβ₄₃), which is the principal component of characteristic plaques of Alzheimer's disease. Aβ is generated by processing of a larger protein APP by two enzymes, termed β- and γ-secretases (Figure 1; Haass et al. 1992; Seubert et al. 1992). The sequence of the Aβ₄₂ peptide is the following:
DAEFRHDSGYEVHHQKLVFFAEDVGSNKGAIIGLMVGGWIA (SEQ ID NO 1)

Aβ₄₁, Aβ₄₀, Aβ₃₉, Aβ₃₈ and Aβ₃₇ differ from Aβ₄₂ by the omission of Ala (A), Ile-Ala (IA), Val-Ile-Ala (VIA), Val-Val-Ile-Ala (VVIA) and Gly-Val-Val-Ile-Ala (GWIA) respectively, from the C-terminal end. Aβ₄₃ differs from Aβ₄₂ by the presence of a threonine residue at the C-terminus. In the methods of the present invention Aβ peptides with any C-terminal ending are detected. In a preferred embodiment of the present invention, the level of Aβ peptides ending at Ala42 (Aβ₄₂) and/or Thr43 (Aβ₄₃) is determined. In another preferred embodiment, the level of Aβ peptides ending at Ala42 (Aβ₄₂) is determined. The Aβ peptides that are detected in the methods of the present may also include isomerized peptides. Aspartic acid-bond isomerization has, for example, been described by Szandrei et al. (1996). The Aβ peptides that are detected in the methods of the present invention (x and y) are also termed "specific Aβ peptide" or "specific Aβ peptides" and refer to Aβ peptides capable of forming an immunological complex with an antibody that recognizes a certain epitope of the Aβ peptide. The term "capable of forming an immunological complex with", "(specifically) recognizing", "(specifically) binding with", "(specifically) reacting with", or "(specifically) forming an immunological reaction with" refers to a binding reaction by the antibody to the specific Aβ peptide which is determinative of the presence of said specific Aβ peptide in the sample in the presence of a heterogeneous population of other peptides, proteins, and/or other biologicals. Thus, under the designated immunassay conditions, the specified antibody preferentially binds to the specific Aβ peptide, while binding to other peptides or proteins does not occur in significant amounts. The binding of an antibody to an Aβ peptide depends on the epitope available on said Aβ peptide. The term "epitope" refers to that portion of the antigen (i.e. the Aβ peptide) that is specifically bound by an antibody-combining site. Epitopes may be determined by any of the techniques known in the art or may be predicted by a variety of computer prediction models known in the art.
For the numerator of the ratio x/y (x), the epitope recognized by the antibody that is capable of binding with the detected "specific Aβ peptide" should contain the first amino acid (D; aspartic acid), the second amino acid (A; alanine), and/or the third amino acid (E; glutamic acid) of the Aβ peptide. In a preferred embodiment, said epitope should contain the first amino acid (D; aspartic acid) of the Aβ peptide. Accordingly, in this preferred embodiment, x is the level of Aβ peptides capable of forming an immunological complex with an antibody that recognizes an epitope of the Aβ peptide containing the first amino acid (D; aspartic acid) of the Aβ peptide. Examples of antibodies recognizing an epitope of the Aβ peptide containing the first amino acid (D; aspartic acid) of the Aβ peptide include (but are not limited to) the monoclonal antibody 3D6, the monoclonal antibody BAN-50 and the monoclonal antibody Anti-N1(D) (Table 1). Accordingly, in a preferred embodiment, x is the level of Aβ peptides capable of forming an immunological complex with the monoclonal antibody 3D6, BAN-50, and/or Anti-N1(D).
For the denominator of the ratio x/y (y), the epitope recognized by the antibody that is capable of binding with the detected "specific Aβ peptide" may not contain the first amino acid (D, aspartic acid), the second amino acid (A; alanine), and/or the third amino acid (E; glutamic acid) of the Aβ peptide.
In a preferred embodiment, for the numerator (x), the epitope recognized by the antibody that is capable of binding with the detected "specific Aβ peptide" contains the first amino acid (D; aspartic acid) of the Aβ peptide and, for the denominator (y), the epitope recognized by the antibody that is capable of binding with the detected "specific Aβ peptide" may not contain the first amino acid (D; aspartic acid) of the Aβ peptide. Accordingly, in this preferred embodiment, y is the level of Aβ peptides capable of forming an immunological complex with an antibody that recognizes an epitope of the Aβ peptide not containing the first amino acid (D; aspartic acid) of the Aβ peptide. Said epitope should thus be different from the 3D6, BAN-50, and/or Anti-N1(D) epitope and y is thus the level of Aβ peptides capable of forming an immunological complex with an antibody that recognizes an epitope different from the 3D6, BAN-50, and/or Anti-N1(D) epitope. Examples of such antibodies that recognize an epitope of the Aβ peptide that does not contain the first amino acid (D; aspartic acid) of the Aβ peptide include (but are not limited to) the antibodies as given in Table 1. Preferred antibodies include 4G8, 6E10, and 10H3. In accordance, in a preferred embodiment, y is the level of Aβ peptides capable of forming an immunological complex with the monoclonal antibody 4G8, with the monoclonal antibody 6E10, and/or with the monoclonal antibody 10H3.
In another embodiment, for the numerator (x), the epitope recognized by the antibody that is capable of binding with the detected "specific Aβ peptide" contains the second amino acid (A; alanine) of the Aβ peptide and, for the denominator (y), the epitope recognized by the antibody that is capable of binding with the detected "specific Aβ peptide" may not contain the first amino acid (D; aspartic acid) and the second amino acid (A; alanine) of the Aβ peptide. Accordingly, in this preferred embodiment, x is the level of Aβ peptides capable of forming an immunological complex with an antibody that recognizes an epitope of the Aβ peptide containing the second amino acid (A; alanine) of the Aβ peptide and y is the level of Aβ peptides capable of forming an immunological complex with an antibody that recognizes an epitope of the Aβ peptide not containing the first amino acid (D; aspartic acid) and the second amino acid (A; alanine) of the Aβ peptide.
In another embodiment, for the numerator (x), the epitope recognized by the antibody that is capable of binding with the detected "specific Aβ peptide" contains the third amino acid (E; glutamic acid) of the Aβ peptide and, for the denominator (y), the epitope recognized by the antibody that is capable of binding with the detected "specific Aβ peptide" may not contain the first amino acid (D; aspartic acid), the second amino acid (A; alanine) and the third amino acid (E; glutamic acid) of the Aβ peptide. Accordingly, in this preferred embodiment, x is the level of Aβ peptides capable of forming an immunological complex with an antibody that recognizes an epitope of the Aβ peptide containing the third amino acid (E; glutamic acid) of the Aβ peptide and y is the level of Aβ peptides capable of forming an immunological complex with an antibody that recognizes an epitope of the Aβ peptide not containing the first amino acid (D; aspartic acid), the second amino acid (A; alanine) and the third amino acid (E; glutamic acid) of the Aβ peptide.

The specific Aβ peptides detected in the numerator (x) of the ratio x/y are capable of forming an immunological complex with an antibody that recognizes an epitope of the Aβ peptide containing the first amino acid (D; aspartic acid), the second amino acid (A; alanine), and/or the third amino acid (E; glutamic acid) of the Aβ peptide. Said specific Aβ peptides should thus at least contain a first amino acid (D; aspartic acid), second amino acid (A; alanine), and/or third amino acid (E; glutamic acid) of the Aβ peptides that is accessible for said antibody. "Accessible" means that said antibody is capable of forming an immunological complex with said epitope containing the first amino acid (D; aspartic acid), the second amino acid (A; alanine), and/or the third amino acid (E; glutamic acid) of the Aβ peptide.
The specific Aβ peptides detected in the denominator (y) of the ratio x/y are capable of forming an immunological complex with an antibody that recognizes an epitope of the Aβ peptide not containing the first amino acid (D; aspartic acid), the second amino acid (A; alanine), and/or the third amino acid (E; glutamic acid) of the Aβ peptide. Said specific Aβ peptides thus lack an accessible first amino acid (D; aspartic acid), second amino acid (A; alanine), and/or third amino acid (E; glutamic acid) of the Aβ peptide. This lack of accessibility could be caused, for example, by the formation of aggregates or oligomers by said specific Aβ peptides wherein the first amino acid (D; aspartic acid), the second amino acid (A; alanine), and/or the third amino acid (E; glutamic acid) of the Aβ peptide are masked and thus not accessible anymore. Aggregated Aβ is identified as a mixture of oligomers in which the monomeric units are held together by non-covalent bonds. The lack of an accessible first amino acid (D; aspartic acid), second amino acid (A; alanine), and/or third amino acid (E; glutamic acid) of the Aβ peptide can also be caused by the presence of modifications on the N-terminal end of the Aβ peptide. Modification on the N-terminal end of the Aβ peptide may prevent the accessibility of the epitope comprising the first amino acid (D; aspartic acid), the second amino acid (A; alanine), and/or the third amino acid (E; glutamic acid) of the Aβ peptide. An example of such modification includes (but is not limited to) acetylation.
The lack of an accessible first amino acid (D; aspartic acid), second amino acid (A; alanine), and/or third amino acid (E; glutamic acid) of the Aβ peptide can also be caused by the simple deletion of said N-terminal amino acids, resulting in N-terminally truncated Aβ peptides. N-terminally truncated Aβ peptides may start their amino acid sequence at amino acid 2 (A; Alanine), 3 (E; glutamic acid), 4 (F; phenylalanine), 5 (R; arginine), 6 (H; histidine), 7 (D; aspartic acid), 8 (S; serine), 9 (G; glycine), 10 (Y; tyrosine), 11 (E; glutamic acid), 12 (V; valine), 13 (H; histidine), 14 (H; histidine), 15 (Q; glutamine), 16 (K; lysine), or 17 (L; leucine) of the Aβ peptide. Some of the N-terminal truncated Aβ peptides that have been identified in human and animal cells, brains and/or CSF are shown in Table 2. In this specific embodiment, wherein the lack of an accessible epitope is caused by the deletion of one or more N-terminal amino acids, x may be the level of Aβ peptides that comprise said N-terminal amino acids of Aβ, also termed Aβ_{(1-C)}, wherein C means any possible C-terminal ending (see above). Preferably, C can be 42 and/or 43 and x is thus the level of Aβ₍₁₋₄₂₎ and/or Aβ₍₁₋₄₃₎ peptides. More preferably Aβ peptides ending at Ala42, i.e. Aβ₍₁₋₄₂₎, are detected and x is thus the level of Aβ₍₁₋₄₂₎ peptides. In the same specific embodiment (wherein the lack of an accessible epitope is caused by the deletion of one or more N-terminal amino acids), the specific Aβ peptides detected in the denominator (y) of the x/y ratio are then any Aβ peptide, starting at any amino acid of the Aβ peptide, referred to as Aβ_{(N-C)}, wherein N means any possible N-terminal ending (i.e., amino acid 1, 2, 3, 4, 5, 6, 7, 8, 9, 10, 11, 12, 13, 14, 15, 16, 17 of Aβ). As indicated above, preferably, C can be 42 and/or 43 and y is the level of Aβ_{(N-42)} and/or Aβ_{(N-43)} peptides. More preferably, peptides ending at Ala42 of Aβ, i.e. Aβ_{(N-42)}, are detected. Accordingly, in these preferred embodiments, x is the level of Aβ₍₁₋₄₂₎ and/or Aβ₍₁₋₄₃₎ peptides and y is the level of Aβ_{(N-42)} and/or Aβ_{(N-43)} peptides. In another preferred embodiment, x is the level of Aβ₍₁₋₄₂₎ peptides and y is the level of Aβ_{(N-42)} peptides. In another preferred embodiment, N is 11 and y is the level of Aβ_{(11-C)} peptides. Preferably y is the level of Aβ₍₁₁₋₄₂₎ and/or Aβ₍₁₁₋₄₃₎ peptides. More preferably, y is the level of Aβ₍₁₁₋₄₂₎.

The term "level" or "levels", as used in the present invention, refers to the amount of specific Aβ peptide present in the body fluid sample. The levels of specific Aβ peptide (x and y) obtained upon analyzing the body fluid samples and their ratio x/y will depend on the particular analytical protocol and detection technique that is used. Accordingly, those skilled in the art will understand that any laboratory, based on the present description, can establish a suitable reference range for the ratio x/y, characteristic (1) for the group of subjects that, at the time of sampling does not show any clinical signs of AD and that later develops AD, (2) for the group of subjects that, at the time of sampling does not show any clinical signs of AD and that later does not develop AD, (3) for the group of subjects that suffer from AD, (4) for the group of control subjects and (5) for the group of subjects that suffer from another dementia such as DLB. The ratio x/y obtained for the subject under diagnosis can then be compared with these reference ranges and, based on this comparison, a conclusion can be drawn as to which of the above groups the subject under diagnosis might belong.

The levels of the specific Aβ peptides may be determined by any method known to those skilled in the art. They can be identified by their structure, by partial amino acid sequence determination, by functional assay, by enzyme assay, by various immunological methods, or by biochemical methods such as capillary electrophoresis, high performance liquid chromatography (HPLC), thin layer chromatography (TLC), hyper diffusion chromatography, two-dimensional liquid phase electrophoresis (2D-LPE; Davidsson et al., 1999), or by their migration pattern in gel electrophoreses. Sodium dodecyl sulfate-polyacrylamide gel electrophoresis (SDS-PAGE) is a widely used approach for separating proteins from complex mixtures (Patterson and Aebersold, 1995). It can be performed in a one- or two-dimensional (2D) configuration. Simultaneous analysis of different variants of Aβ peptides is provided by the Surface Enhanced Laser Desorption/Ionization (SELDI) ProteinChip™ Array (Ciphergen Biosystems Inc., Palo Alto, CA, USA; Davies et al., 1999; Austen et al., 2000). In a preferred embodiment, the level of specific Aβ peptide is detected by an immunoassay. As used herein, an "immunoassay" is an assay that utilizes an antibody to specifically bind to the antigen (i.e., the specific Aβ peptide). The immunoassay is thus characterized by detection of specific binding of the specific Aβ peptide to antibodies. Immunoassays for detecting specific Aβ peptides may be either competitive or noncompetitive. Noncompetitive immunoassays are assays in which the amount of captured analyte (i.e., the specific Aβ peptide) is directly measured. In competitive assays, the amount of analyte (i.e., the specific Aβ peptide) present in the sample is measured indirectly by measuring the amount of an added (exogenous) analyte displaced (or competed away) from a capture agent (i.e., the antibody) by the analyte (i.e., the specific Aβ peptide) present in the sample. In one competition assay, a known amount of the (exogenous) specific Aβ peptide is added to the sample and the sample is then contacted with the antibody. The amount of added (exogenous) specific Aβ peptide bound to the antibody is inversely proportional to the concentration of the specific Aβ peptide in the sample before the specific Aβ peptide is added. In one preferred "sandwich" assay, for example, the antibodies can be bound directly to a solid substrate where they are immobilized. These immobilized antibodies (capturing antibodies) then capture the specific Aβ peptide of interest present in the test sample. Other immunological methods include, but are not limited to, fluid or gel precipitation reactions, immunodiffusion (single or double), agglutination assays, immunoelectrophoresis, radioimmunoassays (RIA), enzyme-linked immunosorbent assays (ELISA), Western blots, liposome immunoassays (LIA; Monroe et al., 1986), complement-fixation assays, immunoradiometric assays, fluorescent immunoassays, protein A immunoassays, or immunoPCR. An overview of different immunoassays is given in Wild (2001), Ghindilis et al. (2002) and Price and Newman (1997). Particularly advantageous are systems in which the levels of the different Aβ peptides, or the levels of the specific Aβ peptides, possibly together with other biological markers, can be detected simultaneously. In this multiparameter aproach, antibodies may be coupled to microspheres or chips. An example of an immunoassay that provides for such simultaneous detection includes (but is not limited to) the xMap™ technology (Luminex 100 IS, Austin, Texas, USA).

In a preferred embodiment, the level of the specific Aβ peptide is determined by an immunoassay comprising at least the following steps:
(a) contacting the specific Aβ peptides with antibody that specifically recognizes the specific Aβ peptide, under conditions suitable for producing an antigen-antibody complex; and
(b) detecting the immunological binding that has occurred between the antibody and the specific Aβ peptide.

In the methods of the present invention, the ratio x/y is thus determined immunologically making use of two antibodies (a set of antibodies), i.e. a first antibody (detecting x) that specifically recognizes an epitope of the Aβ peptide containing the first amino acid (D; aspartic acid), the second amino acid (A; alanine), and/or the third amino acid (E; glutamic acid) of the Aβ peptide and a second antibody (detecting y) that recognizes an epitope of the Aβ peptide not containing the first amino acid (D; aspartic acid), the second amino acid (A; alanine), and/or the third amino acid (E; glutamic acid) of the Aβ peptide.

In another preferred embodiment, the specific Aβ peptide can be detected by a sandwich ELISA comprising the following steps:
(a) bringing said specific Aβ peptide into contact with an antibody (capturing antibody) recognizing said specific Aβ peptide, under conditions being suitable for producing an antigen-antibody complex;
(b) bringing the complex formed between said specific Aβ peptide and said capturing antibody into contact with another antibody (detector antibody) specifically recognizing said specific Aβ peptide or said Aβ peptide capturing antibody complex, under conditions being suitable for producing an antigen-antibody complex;
(c) bringing the antigen-antibody complex into contact with a marker or label either for specific tagging or coupling with said detector antibody, with said marker being any possible marker known to the person skilled in the art;
(d) possibly also, for standardization purposes, bringing the antibodies in contact with a purified specific Aβ peptide reactive with both antibodies.

Advantageously, the detector antibody itself carries a marker or a group for direct or indirect coupling with a marker.
In the method of the present invention, the ratio x/y can thus be determined immunologically making use of two capturing antibodies (or a set of antibodies), i.e. a first (capturing) antibody (detecting x) that specifically recognizes an epitope of the Aβ peptide containing the first amino acid (D; aspartic acid), the second amino acid (A; alanine), and/or the third amino acid (E; glutamic acid) of the Aβ peptide and a second (capturing) antibody (detecting y) that recognizes an epitope of the Aβ peptide not containing the first amino acid (D; aspartic acid), the second amino acid (A; alanine), and/or the third amino acid (E; glutamic acid) of the Aβ peptide. In a preferred embodiment of the present invention, the first antibody specifically recognizes an epitope of the Aβ peptide containing the first amino acid (D; aspartic acid) of the Aβ peptide and the second antibody recognizes an epitope of the Aβ peptide not containing the first amino acid (D; aspartic acid), the second amino acid (A; alanine), and/or the third amino acid (E; glutamic acid) of the Aβ peptide. In another preferred embodiment, the first antibody is the monoclonal antibody 3D6, BAN-50, or Anti-N1(D) and the second antibody recognizes an epitope of the Aβ peptide not containing the first amino acid (D; aspartic acid), the second amino acid (A; alanine), and/or the third amino acid (E; glutamic acid) of the Aβ peptide. In another preferred embodiment of the present invention, the first antibody specifically recognizes an epitope of the Aβ peptide containing the first amino acid (D; aspartic acid), the second amino acid (A; alanine), and/or the third amino acid (E; glutamic acid) of the Aβ peptide and the second antibody recognizes an epitope of the Aβ peptide not containing the first amino acid (D; aspartic acid) of the Aβ peptide. In another preferred embodiment of the present invention, the first antibody specifically recognizes an epitope of the Aβ peptide containing the first amino acid (D; aspartic acid) of the Aβ peptide and the second antibody recognizes an epitope of the Aβ peptide not containing the first amino acid (D; aspartic acid) of the Aβ peptide. In another preferred embodiment of the present invention, the first antibody is the monoclonal antibody 3D6, BAN-50, or Anti-N1(D) and the second antibody recognizes an epitope of the Aβ peptide not containing the first amino acid (D; aspartic acid) of the Aβ peptide. In another preferred embodiment of the present invention, the first antibody specifically recognizes an epitope of the Aβ peptide containing the first amino acid (D; aspartic acid), the second amino acid (A; alanine), and/or the third amino acid (E; glutamic acid) of the Aβ peptide and the second antibody recognizes an epitope of the Aβ peptide different from the 3D6, BAN-50, and/or Anti-N1(D) epitope. In another preferred embodiment of the present invention, the first antibody specifically recognizes an epitope of the Aβ peptide containing the first amino acid (D; aspartic acid) of the Aβ peptide and the second antibody recognizes an epitope of the Aβ peptide different from the 3D6, BAN-50, and/or Anti-N1(D) epitope. In another preferred embodiment of the present invention, the first antibody is the monoclonal antibody 3D6, BAN-50 or Anti-N1(D) and the second antibody recognizes an epitope of the Aβ peptide different from the 3D6, BAN-50, and/or And-N1(D) eptiope. Any antibody that specifically recognizes the specific Aβ peptides under examination can be used in the above method. Examples of antibodies known in the art are provided in Table 1.
The detector antibody to be used in the sandwich ELISA as discussed above, can be any antibody that recognizes an epitope on the Aβ peptide or on the Aβ peptide-capturing antibody complex, not masked by the capturing antibody. Examples of antibodies that can be used as detector antibody are given in Table 3. In a preferred embodiment, Aβ peptides ending at Ala42 (i.e. Aβ₄₂) or Thr43 (i.e. Aβ₄₃) are detected with an antibody that specifically recognizes Aβ₄₂ and/or Aβ₄₃. In another preferred embodiment, Aβ peptides ending at Ala42 (i.e. Aβ₄₂) are detected.

It is evident that, in the sandwich ELISA of the present invention, the capturing and detector antibodies could change place. Thus, the first and second antibody as discussed above, could als be used as detector antibody, when, as a capturing antibody, an antibody is used that recognizes an epitope on the Aβ peptide different from the epitope recognized by these first and/or second antibody.

The antibodies as discussed above can be used in the preparation of a diagnostic kit for use in the methods of the present invention. Accordingly, the present invention relates to a first antibody and a second antibody (a set of antibodies) as discussed above, for the manufacture of a diagnostic kit for determining whether a subject has a likelihood to develop AD, for the diagnosis of a subject suffering from AD and/or for the differential diagnosis of a subject suffering from AD versus a subject suffering from another dementia such as DLB.

As used in the present invention, an "antibody" refers to a protein consisting of one or more polypeptides substantially encoded by immunoglobulin genes or fragments of immunoglobulin genes. The recognized immunoglobulin genes include the kappa, lambda, alpha, gamma, delta, epsilon and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. The basic immunoglobulin (antibody) structural unit is known to comprise a tetramer or dimer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids, primarily responsible for antigen recognition. The terms "variable light chain (V_{L})" and "variable heavy chain (V_{H})" refer to these variable regions of the light and heavy chains respectively. Optionally, the antibody or the immunological portion of the antibody can be chemically conjugated to, or expressed as, a fusion protein with other proteins.
Antibodies used in the present invention include, but are not limited to polyclonal, monoclonal, bispecific, human, humanized, or chimeric antibodies, single variable fragments (ssFv), single chain fragments (scFv), Fab fragments, F(ab') fragments, fragments produced by a Fab expression library, anti-idiotypic antibodies, and epitope-binding fragments of any of the above, provided that they retain the original binding properties. Also mini-antibodies and multivalent antibodies such as diabodies, triabodies, tetravalent antibodies and peptabodies can be used in a method of the invention. The preparation and use of these fragments and multivalent antibodies has been described extensively in International Patent Application WO 98/29442. The immunoglobulin molecules of the invention can be of any class (i.e. IgG, IgE, IgM, IgD, and IgA) or subclass of immunoglobulin molecule.

The specific Aβ peptides detected in the present invention can be used as an immunogen to generate the antibodies used in the invention which specifically bind such an immunogen. Various host animals can be immunized for injection with said specific Aβ peptides, including but not limited to rabbits, mice, rats, etc. Various adjuvants may be used to enhance the immunological response, depending on the host species, including but not limited to complete or incomplete Freund's adjuvant, a mineral gel such as aluminum hydroxide, surface active substances such as lysolecithin, pluronic polyol, a polyanion, a peptide, an oil emulsion, keyhole limpet hemocyanin, dinitrophenol, or an adjuvant such as BCG (bacille Calmette-Guerin), or corynebacterium parvum. For the preparation of monoclonal antibodies, any technique which provides for the production of antibody molecules by continuous cell lines in culture may be used. Hyperimmunization of an appropriate donor, generally a mouse, with the antigen is undertaken. Isolation of splenic antibody producing cells is then carried out. These cells are fused to a cell characterized by immortality, such as a myeloma cell, to provide a fused cell hybrid (Hybridoma) which can be maintained in culture and which secretes the required monoclonal antibody. The cells are then cultured in bulk and the monoclonal antibodies harvested from the culture media for use. Specific techniques include but are not limited to the hybridoma technique developed by Kohler and Milstein (1975), the human B-cell hybridoma technique (Kozbor et al., 1983), or the EBV-hybridoma technique to produce human monoclonal antibodies (Cole et al., 1985). Screening for the desired antibody can be done by techniques known in the art, such as ELISA. For selection of an antibody that specifically binds a specific Aβ peptides, but that does not specifically bind another protein, selection can be made on the basis of positive binding to the first and the lack of binding to the second.

While various antibody fragments are defined in terms of enzymatic digestion of an intact antibody with papain, pepsin or other proteases, one skilled in the art will appreciate that such antibody fragments as well as full size antibodies may be synthesized de novo either chemically or by utilizing recombinant DNA methodology. Thus, the term antibody, as used herein, also includes antibodies and antibody fragments either produced by the modification of whole antibodies or synthesized de novo using recombinant DNA methodologies. The term "humanized antibody" means that at least a portion of the framework regions of an immunoglobulin is derived from human immunoglobulin sequences. The humanized versions of the mouse monoclonal antibodies can, for example, be made by means of recombinant DNA technology, departing from the mouse and/or human genomic DNA sequences coding for H and L chains or from cDNA clones coding for H and L chains. Humanized forms of mouse antibodies can be generated by linking the CDR regions of non-human antibodies to human constant regions by recombinant DNA techniques (Queen et al., 1989; WO 90/07861). Alternatively, the monoclonal antibodies used in the method of the invention may be human monoclonal antibodies. Human antibodies can be obtained, for example, using phage-display methods (WO 91/17271; WO 92/01047). In these methods, libraries of phage are produced in which members display different antibodies on their outersurfaces. Antibodies are usually displayed as Fv or Fab fragments. Human antibodies against specific Aβ peptides can also be produced from non-human transgenic mammals having transgenes encoding at least a segment of the human immunoglobulin locus and an inactivated endogenous immunoglobulin locus (WO 93/12227; WO 91/10741). Human antibodies can be selected by competitive binding experiments, or otherwise to have the same epitope specificity as a particular mouse antibody. Such antibodies are particularly likely to share the useful functional properties of the mouse antibodies. Human polyclonal antibodies can also be provided in the form of serum from humans immunized with an immunogenic agent. Optionally, such polyclonal antibodies can be concentrated by affinity purification using specific Aβ peptides as an affinity reagent. Monoclonal antibodies can be obtained from serum according to the technique described in WO 99/60846. Also useful in the above methods might be the heavy chain variable domains (VHH) produced as part of the humoral immune response of camelids. Recombinant VHH selected from 'camelised' human VH libraries could consitute excellent ligands for the detection of the specific Aβ peptides of the present invention (Spinelli et al., 2000; Muyldermans, 2001; Cortez-Retamozo et al., 2002).

The antibodies used in the methods of the present invention may be labeled by an appropriate marker or label. The particular label or detectable group used in the assay is not a critical aspect of the invention, as long as it does not significantly interfere with the specific binding of the antibody used in the assay. The detectable group can be any material having a detectable physical or chemical property. Such detectable labels have been well developed in the field of immunoassays and, in general, almost any label useful in such methods can be applied to the methods of the present invention. Thus, a label is any composition detectable by spectroscopic, photochemical, biochemical, immunochemical, electrical, optical, radiological or chemical means. Useful labels in the present invention include but are not limited to magnetic beads (e.g. Dynabeads™), fluorescent dyes (e.g. fluorescein isothiocyanate, Texas Red, rhodamine, phycoerythrin, Alexa 532, cyanine 3), radiolabels (e.g. ³H, ¹²⁵I, ³⁵S, ¹⁴C, or ³²P), enzymes (e.g. horseradish peroxidase, alkaline phosphatase, and others commonly used in an ELISA), and colorimetric labels such as colloidal gold, colored glass, or plastic (e.g., polystyrene, polypropylene, latex, etc.) beads.
The label may be coupled directly or indirectly to the desired component of the assay according to methods well known in the art. As indicated above, a wide variety of labels may be used, with the choice of label depending on the sensitivity required, the ease of conjugation with the compound, stability requirements, the available instrumentation and disposal provisions. Non-radioactive labels are often attached by indirect means. Generally, a ligand molecule (e.g., biotin) is covalently bound to the antibody. The ligand then binds to an anti-ligand (e.g., streptavidin) molecule, which is either inherently detectable or covalently bound to a signal system, such as a detectable enzyme, a fluorescent compound, or a chemiluminescent compound. A number of ligands and anti-ligands can be used. Where a ligand has a natural anti-ligand, for example, biotin, thyroxine, and cortisol, it can be used in conjunction with the labeled, naturally occurring anti-ligands. Alternatively, a haptenic or antigenic compound can be used in combination with an antibody. The antibodies can also be conjugated directly to signal generating compounds; for example, by conjugation with an enzyme or fluorophore. Enzymes of interest as labels will primarily be hydrolases, particularly phosphatases, esterases and glycosidases, or oxidoreductases, particularly peroxidases. Fluorescent compounds include fluorescein and its derivatives, rhodamine and its derivatives, dansyl, umberlliferone, etc. Chemiluminescent compounds include luciferin, and 2,3-dihydrophthalazinediones, for example, luminol. A review of other labeling or signal-producing system is available in US patent No. 4,391,904.
Means for detecting labels are well known in the art Thus, for example, where the label is a radioactive label, means for detection include a scintillation counter or photographic film as in autoradiography. Where the label is a fluorescent label, it may be detected by exciting the fluorophore with the appropriate wavelength of light and detecting the resulting fluorescence. The fluorescence may be detected visually, by means of a photographic film, by the use of electronic detectors such as charge coupled devices (CCDs) or photomultipliers and the like. Similarly, enzyme labels may be detected by providing the appropriate substrates for the enzyme and detecting the resulting reaction product Finally simple colorimetric labels may be detected simply by observing the color associated with the label.

Some assay formats do not require the use of labeled components. For instance, agglutination assays can be used to detect the presence of the target antibodies. In this case, antigen-coated particles are agglutinated by samples comprising the target antibodies. In this format, none of the components need be labeled and the presence of the target antibody is detected by simple visual inspection.

The present invention also provides the use of diagnostic kits comprising the antibodies as referred to above. The invention thus provides the use of a diagnostic kit for determining whether a subject has a likelihood to develop AD, for the diagnosis of a subject suffering from AD and/or for the differential diagnosis of a subject suffering from AD versus a subject suffering from another dementia such as DLB, comprising at least a first antibody and a second antibody (a set of antibodies) as discussed above.
A preferred kit for carrying out the methods of the present invention comprises:
- a first and a second antibody (capturing antibodies) which form an immunological complex with the specific Aβ peptides to be detected;
- an antibody (detector antibody) which recognizes the specific Aβ peptides (or the specific Aβ peptide- capturing antibody complex) to be detected, and not recognizing an epitope recognized by the first or the second antibody;
- a marker or label either for specific tagging or coupling with said detector antibody;
- appropriate buffer solutions for carrying out the immunological reaction between the capturing antibody and the specific Aβ peptide, between the detector antibody and the capturing antibody-specific Aβ peptide complex and/or between the bound detector antibody and the marker or label;
- possibly, for standardization purposes, purified specific Aβ peptides.

The present invention thus provides the use of a first and second antibody (a set of antibodies) or a diagnostic kit, as defined above, in the determination of whether a subject has a likelihood to develop AD, for the diagnosis of a subject suffering from AD and/or for use in the differential diagnosis of a subject suffering from AD versus a subject suffering from another dementia such as DLB.

In the methods of the present invention, the detection of at least the ratio x/y, may optionally be combined with the detection of one or more additional known biomarkers for neurological diseases, including but not limited to other Aβ peptides, tau, phospho-tau, synuclein, Rab3a, cytokines, glutamine synthase (GS) and neural thread protein. Combination of relevant biological markers may increase the sensitivity and specificity of the diagnosis. The methods of the invention can also be used for further confirmation of a diagnosis previously made with one or more other biological markers.

The methods, diagnostic kits and/or set of antibodies of the present invention can also be used for monitoring the effect of therapy administered to a subject, also called therapeutic monitoring or treatment follow up, and patient management. Accordingly, the present invention is also related to the methods as described above for use in the treatment follow up of a subject that has a likelihood to develop AD or of a subject that is diagnosed as suffering from AD. Changes in the x/y ratio can be used to evaluate the response of a subject to drug treatment. In this way, new treatment regimes can also be developed by examining the x/y ratio in a subject. The method of the present invention can thus assist in monitoring a clinical study, for example, for evaluation of a certain therapy for memory impaired subjects, subjects with MCI or subjects suffering from AD. In this case, a chemical compound is tested for its ability to normalize the x/y ratio in a subject diagnosed as developing or suffering from AD.

The methods of the present invention can also be used in animal or cellular models, for example, for drug screening. The animal model on which the method of the present invention can be applied can be any model of an animal in which the body control system is directed by CNS. The animal thus may belong to the Platyhelminthes, Aschelminthes, Annelida, Arthropoda, Mollusca, Echinodermata, Acrania, Cyclostomata, Chondrichthyes, Osteichthyes, Amphibia, Reptilia, Aves, and Mammalia. In a preferred embodiment, the animal in the animal model is a mouse, a rat, a monkey, a rabbit, a worm, a fly, a zebrafish, a pufferfish or *C. elegans.* In another embodiment, the animal is a transgenic animal, possibly modified by one or more determinants causing AD. Cellular models on which the method of the present invention can be applied can be any cell line in which APP is expressed. Examples include APP expressing primary cultures of neurons as described by De Jonghe et al. (2001), CHO (Chinese Hamster Ovarian) cells transfected with human wild type or mutant APP and human neuroblastoma cells (SKNSH-SY5Y) transfected with human wild type or mutant APP as described in WO 02/37118.

Throughout this specification and the claims which follow, unless the context requires otherwise, the word "comprise", and variations such as "comprises" and "comprising" will be understood to imply the inclusion of a stated integer or step or group of stated integers or steps but not to the exclusion of any other integer or step or group of integers or steps.

The present invention will now be illustrated by reference to the following examples that set forth particularly advantageous embodiments. However, it should be noted that these examples are illustrative and can not be construed as to restrict the invention in any way.

### EXAMPLES

### Example 1: Epitope mapping of the monoclonal antibodies used in the present invention

### 1. Determination of the binding specificities of monoclonal antibodies 3D6, 6E10 and 4G8 in ELISA

Three J3-amyloid antibodies, 3D6 (Elan Pharmaceuticals, South San Francisco, CA, USA), 6EI0 and 4G8 (Signet Laboratories, Dedham, MA, USA) were tested in ELISA for immunological binding with different β-amyloid peptides truncated at their N-terminal end: Aβ₍₁₋₄₂₎, Aβ₍₂₋₄₂₎, Aβ₍₃₋₄₂₎, Aβ₍₄₋₄₂₎, Aβ₍₅₋₄₂₎, Aβ₍₈₋₄₂₎, Aβ₍₉₋₄₂₎. Synthetic peptides were obtained from Bachem (Heidelberg, Germany), Neosystems (Strasbourg, France), or AnaSpec (San Jose, California, USA). The ELISA format and its characteristics have previously been described in detail (Vanderstichele et al., 2000). In short, plates were pre-coated with the 3D6, 6E10 or 4G8 monoclonal antibody, specific for the N-terminus of Aβ42, as capturing antibody. To each well, 100 µl of blank or peptide sample were added and incubated for three hours. After several wash steps, the plates were incubated for 1 h with the biotinylated monoclonal antibody 21F12, specific for the carboxy terminus of Aβ42. Biotinylated antibodies were detected via peroxidase-labeled streptavidin. After the final washing step, substrate was added and the reaction was stopped after 30 min by adding 0.2 N sulphuric acid. The optical density (OD) was measured at 450 nm.

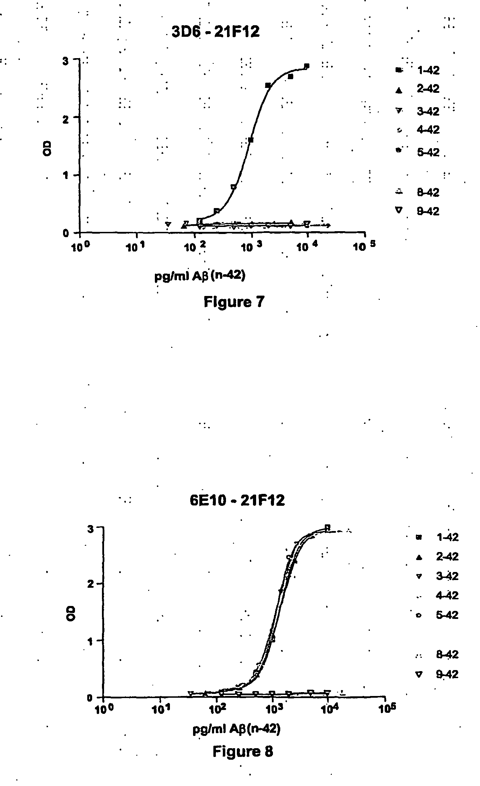

The reactivity of 3D6, 6E10 and 4G8 with the different N-terminally truncated Aβ peptides is shown in Figures 7, 8 and 9 respectively. Peptide concentrations have been normalized according to the reactivity with the 4G8 antibody. 3D6 was only reactive with Aβ₍₁₋₄₂₎, indicating that 3D6 recognizes an epitope of the Aβ peptide containing the first amino acid (D; aspartic acid). 6E10 reacted with Aβ₍₁₋₄₂₎, Aβ₍₂₋₄₂₎, Aβ₍₃₋₄₂₎, Aβ₍₄₋₄₂₎, and Aβ₍₅₋₄₂₎. Accordingly, 6E10 recognizes an epitope of the Aβ peptide not containing the first (D; aspartic acid), second (A;alanine) and third (E; glutamic acid) amino acid. 4G8 was reactive with all Aβ peptides tested. 4G8 therefore should recognize an epitope of Aβ residing beyond amino acid 9. The epitopes recognized by these monoclonal antibodies are indicated in Figure 1.

### 2. Determination of the binding specificities of monoclonal antibodies 3D6, 6E10 and 4G8 in a multiparameter immunoassay

The three β-amyloid antibodies, 3D6, 6E10 and 4G8 were tested in a multiparameter assay for immunological binding with Aβ₍₁₋₄₂₎ and Aβ₍₂₋₄₂₎. Synthetic peptides were obtained from Bachem (Heidelberg, Germany), Neosystems (Strasbourg, France), or AnaSpec (San Jose, California, USA).

The xMAP™-technology (Luminex, Austin, Texas, USA) was used to design a multiparametric bead-based assay. 3D6, 6E10, and 4G8 were covalently coupled onto carboxylated microsphere sets according to a modified protocol supplied by the manufacturer. In short, a mixture of water-soluble 1-ethyl-3-(3-dimethyllaminopropyl)-carbodiimide hydrochloride (EDC; Pierce Chemicals, Erembodegem, Belgium) and N-hydroxy-sulfosuccinimide (Sulfo-NHS; Pierce Chemicals, Erembodegem, Belgium) was used to activate the free carboxyl groups on the beads. The amino groups of the antibodies were subsequently covalently bound to the carboxy groups of the microspheres. The antibody-coupled beads were counted using a haemocytometer. Coupled microspheres were stored in the dark at 2-8 °C.

All incubations were performed at room temperature (25°C). A ninety-six well filter plate (Millipore Corporation, Bedford, MA, US) was first pretreated with 250 µl wash buffer (PBS, 0.05% Tween20). The wash buffer was removed from the plates with a vacuum manifold (Millipore Corporation, Brussels, Belgium). The coupled microspheres were allowed to eliquilibrate at room temperature for at least 15 min, after which 100 µl of sonicated microspheres (30.000 beads/ml for each parameter) were added to the filterplate, after which the plates were covered with aluminium foil. After aspiration of the buffer, 50 µl of the biotinylated detector antibody 21F12 (Innogenetics N.V., Gent, Belgium) and 50 µl of the peptide sample were added to the wells. Incubation was performed overnight (16 hours) by shaking at room temperature on an orbital plate shaker at 1000 rpm. The wells were washed three times by aspiration with 300 µl of wash buffer and incubated with 100 µl of Streptavidin-phycoerythrine (Streptavidin-PE; Caltag, Burlingame, CA, US; Sanbio, Uden, the Netherlands) for 60 min on a plate shaker. The wells were again washed three times by aspiration with wash buffer. Finally, the microspheres were redissolved in PBS. The fluorescence intensity was measured in the Luminex100 instrument using software version 2.1 for analysis.

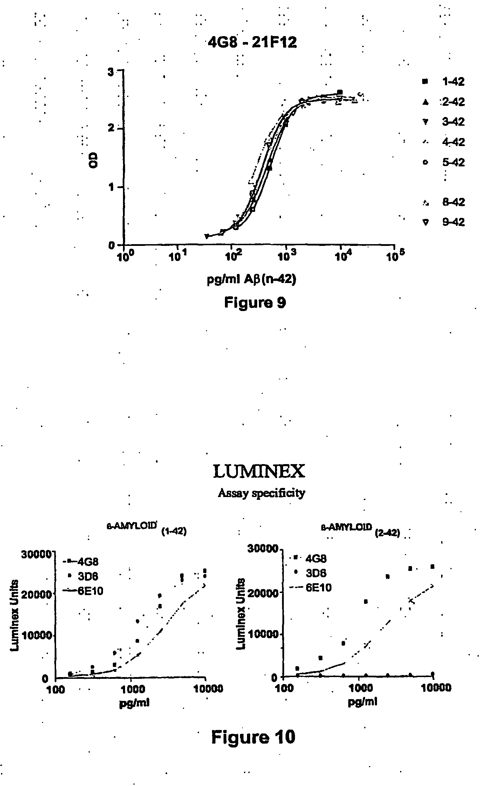

The reactivity of 3D6, 6E10 and 4G8 with the Aβ₍₁₋₄₂₎ and Aβ₍₂₋₄₂₎ peptides is shown in Figure 10. 3D6 was reactive with Aβ₍₁₋₄₂₎ but not with Aβ₍₂₋₄₂₎. This confirms that 3D6 recognizes an epitopes of the Aβ peptide containing the first amino acid (D; aspartic acid). 6E10 and 4G8 reacted with both peptides Aβ₍₁₋₄₂₎ and Aβ₍₂₋₄₂₎, confirming that 6E10 and 4G8 recognize an epitope of the Aβ peptide not containing the first (D; aspartic acid) amino acid. The epitopes recognized by these monoclonal antibodies are indicated in Figure 1.

### Example 2: Analysis of the Aβ peptides binding 3D6 and the Aβ peptides binding 4G8 or 6E10 in CSF samples obtained from subjects suffering from memory impairment or dementia

### 1. Subjects

A study was carried out based on CSF samples archived at the Sahlgren's University Hospital, Göteborg, Sweden, including 166 subjects. For 12 CSF samples, not all parameters were determined. Exclusion of these partial results did not affect the final analysis. The results of 154 samples are discussed here, including the following patient groups: 18 patients with moderate AD (modAD), 21 patients with severe AD (sevAD), 20 patients with mild AD (mildAD), 39 patients with cognitive impairment, 12 patients with dementia with Lewy bodies (DLB), 15 patients with Parkinson's disease (PD) and 29 control subjects (C). All patients with AD satisfied the NINCDS-ADRDA criteria (McKhann et al., 1984). For the patient group with cognitive impairment, no symptom other than memory impairment was reported or identified and none of these patients fulfilled the DSM-IV criteria for dementia. Within a follow up period of 5 years, 14 patients with cognitive impairment had progressed to AD (Cog-AD) whereas in 25 patients with cognitive impairment, the memory problems did not develop into AD (Cog). DLB was diagnosed according to the Consensus guidelines for the clinical and pathological diagnosis of dementia with Lewy bodies (McKeith et al., 1996). PD patients were included according to Langston et al., (1992). The control group (C) consisted of individuals without histories, symptoms, or signs of psychiatric or neurological disease, malignant disease, or systemic disorders (e.g., rheumatoid arthritis, infectious disease).

The Ethics Committees of the University of Göteborg, Sweden, approved the study. All patients (or their nearest relatives) and controls gave informed consent to participate in the study, which was conducted according to the provisions of the Declaration of Helsinki.

### 2. Sampling

CSF samples were taken using atraumatic cannulas placed in the L3/L4 or L4/L5 invertebral space of the subject 12 ml was collected in sterile polypropylene tubes and gently mixed. The CSF was centrifuged for 10 minutes at 4000g. Samples were sent to the Clinical Neurochemistry Laboratory at Sahlgrens's University Hospital in Mölndal. Sweden. After arrival, samples were aliquoted and kept frozen at -80°C. Samples were kept without being thawed and refrozen. In the native CSF, determination of routine chemical parameters was performed, including leucocyte and erythrocyte cell counts as well as glucose and lactate measurements, total protein content, CSF-serum ratios of albumin and immunoglobulin G, and a screening for oligoclonal bands. CSF samples were not included in the study if they contained more than 500 red blood cells per µL.

### 3. Immunoassay

The levels x and y of specific Aβ peptides in the CSF samples were determined using the xMap™ technology (Luminex 100IS, Austin, Texas, US). As capturing antibody, the monoclonal antibody 3D6 (Elan Pharmaceuticals, South San Francisco, CA, US) was used for the detection of x and the monoclonal antibodies 6E10 and 4G8 (Signet Laboratories, Dedham, MA, US) for the detection of y. The monoclonal antibody 21F12 (Innogenetics N.V., Gent, Belgium) was used as detector antibody. The multiparameter assay was carried out as described above. Luminex units were converted to ELISA pg peptide equivalents /ml using synthetic amyloid₍₁₋₄₂₎ peptide as a standard and a sigmoidal fit as a curve fitting model.

### 4. Statistical analysis

Data analysis was based on graphical representation by means of box plots. These plots represent the median values and the 25%-75% range that covers the middle 50% of the data points for every variable. Additional support for the capacity of a variable to discriminate between 2 groups is provided by a formal Mann-Whitney U test This test assesses the hypothesis that the sum of the ranks of data points in each group is the same. Significant p values (<0.05) reject this hypothesis and thus provide strong evidence for the discriminating ability of a variable between the 2 groups. P values <0.10 suggest a trend for the discriminating ability of a variable between the 2 groups.

### 5. Discrimination of memory-impaired subjects that will progress to AD from patients with prolonged memory problems

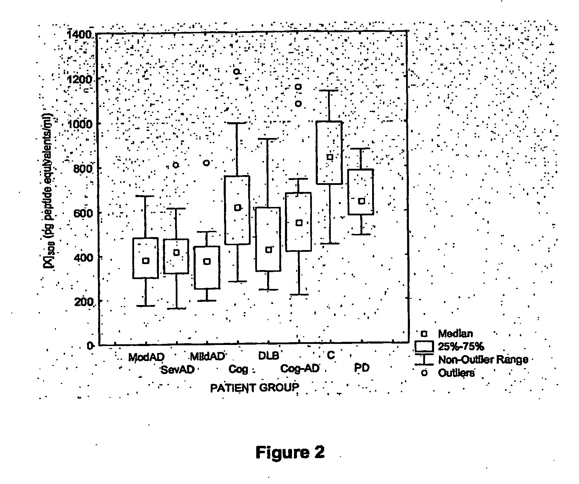

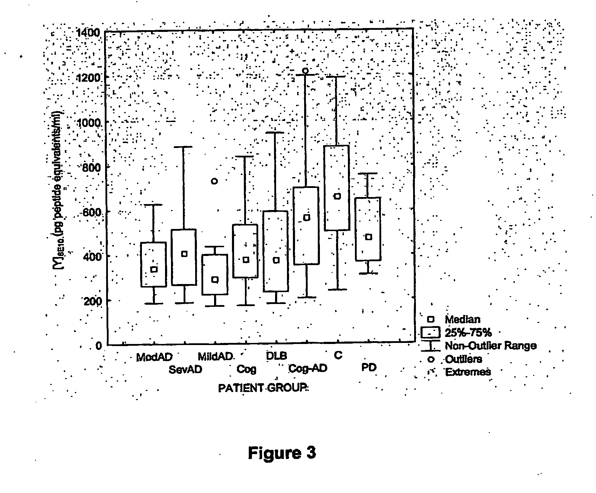

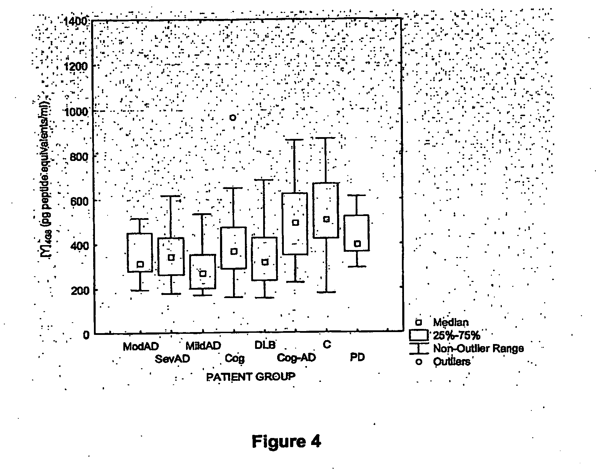

In Figure 2 ([x]_{3D6}), Figure 3 ([y]_{6E10}) and Figure 4 ([y]_{4G8}), the median and the 25%-75% interval are shown for the levels x and y of specific Aβ peptides in the CSF samples of the different subject groups, using 3D6, 6E10 and 4G8 as capturing antibody, respectively. No differences in the level of Aβ peptides binding 3D6 or Aβ peptides binding 6E10 or 4G8 can be observed between the patients with cognitive impairment who progressed to AD (Cog-AD) compared to the patients with cognitive impairment who did not develop AD (Cog). The p values of the Mann-Whitney U test were 0.46, 0.23, and 0.15 for [x]_{3D6}, [y]_{6E10} and [y]_{4G8}, respectively.

In Figure 5 ([x]_{3D6}/[y]_{6E10}) and Figure 6 ([x]_{3D6}/[y]_{4G8}), the median and the 25%-75% interval are shown for the ratios of x/y in the CSF samples of the different subject groups. For this ratio, a clear decrease was observed for patients with cognitive impairment who progressed to AD (Cog-AD) compared to the patients with cognitive impairment who did not develop AD (Cog). The p values of the Mann-Whitney U test were <0.001 and <0.001 for [x]_{3D6}/[y]_{6E10} and [x]_{3D6}/[y]_{4G8}, respectively. This shows that in a population of subjects with memory problems, the ratio of x/y allows an excellent discrimination between subjects developing AD and subjects with prolonged memory problems who do not develop AD.

### 6. Discrimination of subjects suffering from AD from subjects suffering from DLB

In Figures 2 ([x]_{3D6}), 3 ([y]_{6E10}), and 4 ([y]_{4G8}), the median and the 25%-75% interval are shown for the levels x and y of specific Aβ peptides in the CSF samples of the different subject groups using 3D6, 6E10, and 4G8 as capturing antibody, respectively. While a clear decrease can be observed in the level of Aβ peptides binding 3D6 between patients who suffer from any form of AD (ModAD, SevAD, MildAD) compared to control subjects (C), to patients with PD (PD) or to patients suffering from memory impairment (Mem, Mem-AD), the levels x or y did not provide for the discrimination between patients suffering from AD versus patients suffering from DLB. In the comparison of all AD (ModAD, SevAD, and MildAD pooled) to DLB the p values of the Mann-Whitney U test were 0.16, 0.68, and 0.79 for [x]_{3D6}, [y]_{6E10} and [y]_{4G8}, respectively.

In Figures 5 ([x]_{3D6}/[y]_{6E10}) and 6 ([x]_{3D6}/[Y]_{4G8}), the median and the 25%-75% interval are shown for the ratios of x/y in the CSF samples of the different subject groups. For this ratio, a clear decrease can be observed for patients suffering from any form of AD (ModAD, SevAD, MildAD) compared to patients suffering from DLB. The p values of the Mann-Whitney U test were 0.098 and <0.001 for [x]_{3D6}/[y]_{6E10} and [x]_{3D6}/[y]_{4G8}, respectively. This shows that the ratio of x/y allows discrimination between subjects suffering from AD versus subjects suffering from DLB.

### Example 3: Analysis of the levels x and y of specific Aβ peptides in samples obtained from subjects suffering AD

### 1. Subjects

CSF samples from patients with AD (n=22) and non-demented controls (n=20) were provided by the Sahlgren's University Hospital, Göteborg, Sweden. They were collected for the purpose of routine diagnostic procedure from patients diagnosed according to the generally accepted criteria of AD, ICD-10 (World Health Organization, 1992) and National Institute of Neurological and Communicative Disorders and Stroke-Alzheimer's Disease and Related disorder Association (NINCDS-ADRDA criteria; McKann et al., 1984). The control group consisted of individuals without histories, symptoms, or signs of psychiatric or neurological disease.

The Ethics Committees of the Universities of Göteborg, Sweden, approved the study. All patients (or their nearest relatives) and controls gave informed consent to participate in the study, which was conducted according to the provisions of the Declaration of Helsinki.

### 2. Analysis of the levels x and y of specific Aβ peptides by multiparameter immunoassay

The levels x and y of specific Aβ peptides in the CSF samples were determined using the xMAP™-technology (Luminex, Austin, Texas, USA). As capturing antibody, the monoclonal antibody 3D6 was used for detection of x and the monoclonal antibody 4G8 for detection of y. The monoclonal antibody 21F12 was used as detector antibody. The multiparameter assay was carried out as described above.

Data analysis was based on graphical representation by means of box plots. These represent the median values and the 25%-75% range that covers the middle 50% of the data points for every variable. Additional support for the capacity of a variable to discriminate between the 2 groups (AD and controls) was provide by the Student-Newman-Keuls test for pairwise comparisons. A significant p value (<0.05) provides strong support for the discriminating ability of a variable between AD and control patients.

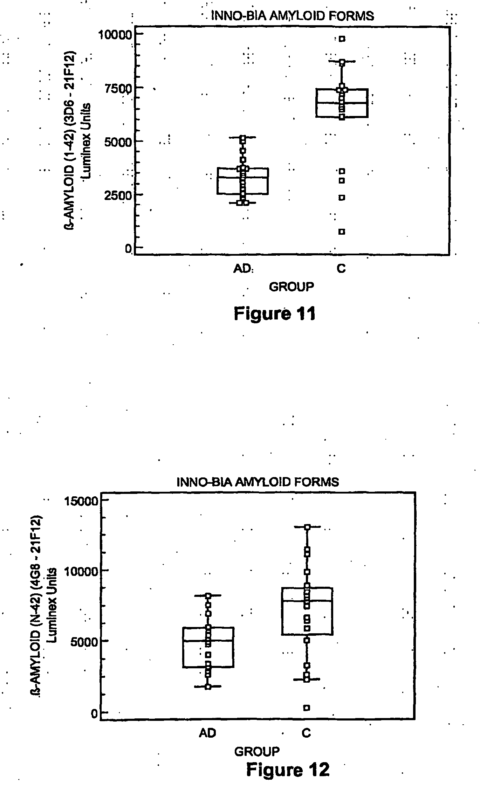

In Figure 11 (3D6-21F12) and Figure 12 (4G8-21F12) the median and 25%-75% interval are shown for respectively the levels x and y of specific Aβ peptides in the CSF samples of the AD patients and the control group. A clear difference in Aβ peptides binding 3D6 or 4G8 was observed between the patients suffering from AD and the control subjects. The p values of the Student-Newman-Keuls test were <0.001 and 0.008 respectively, indicating the discriminating ability of the levels x (specific Aβ peptides binding 3D6) and y (specific Aβ peptides binding 4G8).

In Figure 13 (RATIO: 3D6 vs. 4G8) the median and the 25%-75% interval are shown for the ratios of x/y (wherein x is the level of specific Aβ₄₂₍₄₃₎ peptides binding 3D6 and y is the level of specific Aβ₄₂₍₄₃₎ peptides binding binding 4G8) in the CSF samples of the AD patients and the control group. Also for this ratio x/y a clear difference is observed between the patients suffering from AD and the control subjects. The p value of the Student-Newman-Keuls test was 0.003 indicating the discriminating ability of this ratio.

### 3. Analysis of the levels x and y of specific Aβ peptides by surface-enhanced laser desorption/ionization technology

Surface-enhanced laser desorption/ionization (SELDI) technology was applied to analyze and compare the patterns of Aβ₍₄₂₎ peptide in CSF of 22 individual CSF samples from patients with AD (n=22) and non-demented controls (n=20). The CSF samples were analyzed on the SELDI-TOF (PBS IIc) for the presence of specific Aβ₍₄₂₎ peptides. To allow this, the Aβ peptides were immunologically bound to a ProteinChip by the following immuno-array preparation protocol. A monoclonal antibody (4D7A3; Innogenetics Cat. No. BR032D) directed towards C-terminus of β-amyloid₍₄₂₎ peptide was covalently linked to the PS 10 ProteinChip by applying 1 µg 4D7A3 on the array-spot and incubation in a humidity chamber (3h, RT) to allow covalent binding to the PS10 ProteinChip array. The arrays were washed twice with PBS/0.1% Triton X-100. Additional washing was performed 2x with PBS (pH 8.0). The arrays were blocked with 0.5 M Tris pH 8.0 (during 2h, RT). The arrays were again washed twice with PBS/0.1% Triton X-100. 100 µl CSF in 0.1M urea/0.1 %CHAPS was loaded on a spot using the ProteinChip bioprocessor and incubated by constant shaking, overnight at 4°C. The arrays were again washed twice with PBS/0.1% Triton X-100 and two additional washes with 50 mM Hepes pH 8.0. On the air-dried array spots α-cyano-4-hydroxycinnamic acid in 50% ACN/50 % TFA was applied and mass analysis was performed. The same immune-array was prepared with antibody 31D11 as control antibody to obtain a spectrum without any Aβ peptide (Figure 14).

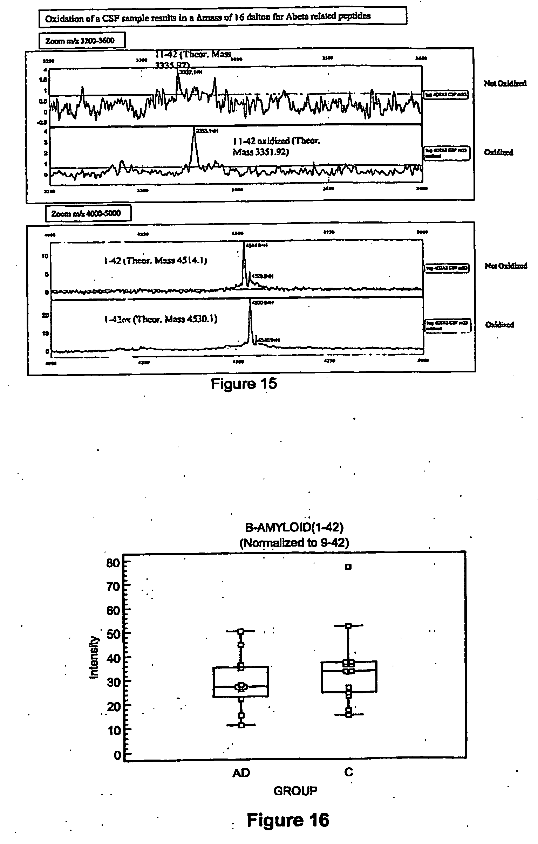

External calibration was performed with Dynorphorin (Mr=2147,50 Da), human ACTH₍₁₋₂₄₎ (2933,50), Bovine insulin beta-chain (3495,94), and human insulin (5807,65). On this basis mass accuracy was calculated for the Aβ₍₄₂₎ peptide peak with theoretical mass of 4514,1 Da. The m/z value measured by SELDI-TOF was 4512,069 Da (STDEV 1,193456, %CV 0,02645) giving the accuracy for this experiment of 450 ppm. Taking this mass accuracy of the SELDI-TOF (450 ppm) into a account, and by using complementary analysis of relevant synthetic β-amyloid peptides, novel N-terminally truncated β-amyloid peptides were assigned on the basis of their molecular masses as follows: 1-42, 11-42, 8-42, 5-42 and 3-42 (Figure 14). Oxidation of the CSF sample resulted in a change in mass of 16 dalton for the Aβ peptides (Figure 15). The theoretical and measured mass and the mass accuracy of the present technology are shown in Table 4. For a number of Aβ peptides, a very high mass accuracy was obtained. The peak intensities of the different specific Aβ peptides in the tested samples are given in Table 5. Peptide peak intensities of Aβ₍₁₋₄₂₎ were specifically decreased in AD CSF if compared with CSF of non-dementing controls. For the measured N-truncated β-amyloid₍₄₂₎ peptides peak intensities, no significant difference between two patients groups was detected. However, when mass peak intensities of detected amyloid species were expressed as ratio of Aβ₍₁₋₄₂₎/Aβ_{(N-42)}, Alzheimer disease patients could be differentiated from the group of controls (data not shown).

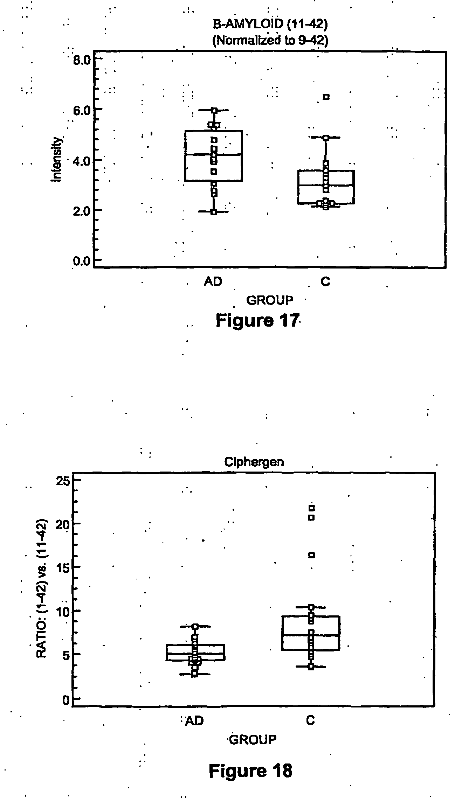

In order to improve measurements, the same samples were exposed to the high-laser intensity and for several samples more accurate data were obtained For the calibration purposes 7 fmol 9-42 β-amyloid peptide (AnaSpec San Jose, CA; cat. No. 60084-1) and 6 fmol bovine insulin (Ciphergene Biosystems Fremont, CA) were applied and were used for data calibration. In Figures 16 and 17, the median and the 25%-75% interval are calculated with normalized data for the specific Aβ₍₁₋₄₂₎ and Aβ₍₁₁₋₄₂₎ peptide in the CSF samples of the AD and control group. The capacity of Aβ₍₁₋₄₂₎ and Aβ₍₁₁₋₄₂₎ levels to discriminate between AD and control subjects was further estimated by the Students Newman-Keuls test for pairwise comparison. The values for the Aβ₍₁₁₋₄₂₎ level was 0.041 indicating the discriminating ability of the Aβ₍₁₁₋₄₂₎ level. In Figure 18, the median and the 25%-75% interval are calculated with normalized data for the ratio Aβ₍₁₋₄₂₎/Aβ₍₁₁₋₄₂₎ in the CSF samples of the AD and control group. For this ratio also a clear difference is observed between the patients suffering from AD and the control subjects. The p value calculated by the Students Newman-Keuls test was 0.003, indicating the discriminating ability of this ratio Aβ₍₁₋₄₂₎/Aβ₍₁₁₋₄₂₎. If normalized data were used for the ratio analysis; the degree of differentiation between AD and control group was improved.

### TABLES

**Table 1. Antibodies capable of forming an immunological complex with the specific Aβ peptides detected in the methods of the invention.**

| **Antibody** | **Epitope of Aβ** | **Reference** |
|---|---|---|
| **Detecting specific Aβ peptides of numerator of x/y ratio (i.e. x) [first antibody]** | | |
| 3D6 | 1-5 | Games et al., 1995; Bard et al., 2000; Vanderstichele et al., 2000; DeMattos et al., 2001; Brayden et al., 2001; Elan Pharmaceuticals, South San Francisco, CA, US |
| BAN-50 | (1-10) | Iwatsubo et al., 1994; Suzuki et al., 1994; Gravina et al., 1995; Tamaoka et al., 1997; Enya et al., 1999; Harigaya et al., 2000; Kawarabayashi et al., 2001; Oshima et al., 2001; EP 0 683 234 A1 |
| Anti-Aβ₁₋₅ Anti-N1(D) | 1-5 | Saido et al., 1995; 1996; Russo et al., 1997; Tekirian et al., 1998 |

| **Detecting specific Aβ peptides of denominator of x/y ratio (i.e. y) [second antibody]** | | |
|---|---|---|
| 6E10 | 4-13. | Kim et al., 1990; Metha et al., 1991;Ghiso et al., 1992; Pirtilla et al., 1994; Kida et al., 1995; Wang et al., 1996; Russo et al., 1997; Kawarabayashi et al., 2001; Oshima et al., 2001; Wiltfang et al., 2002; Senetek, St. Louis, MO, USA; Signet Laboratories , Dedham, MA, US |
| 10H3 | 7-12 | WO 89/06242; Majocha et al., 1992; Friedland et al., 1994 |
| 4G8 | 17-24 | Kim et al., 1988; Bancher et al., 1989; Kim et al., 1990; Spillantini et al., 1990; Metha et al., 1991; Anderson et al,. 1992; Shoji et al., 1992; Harrington et al., 1993; Pirtilla et al., 1994; Gravina et al., 1995; Kida et al., 1995; Wang et al., 1996; Russo et al., 1997; Enya et al., 1999; Harigaya et al., 2000; Kawarabayashi et al., 2001; Oshima et al., 2001; Senetek, St. Louis, MO, US; Signet Laboratories, Dedham, MA, US |
| 266 | 13-28 | Citron et al., 1992; Haass et al., 1992; Seubert et al., 1992; Vigo-Pelfrey et al., 1993 |
| 5E2 | (6-24) | Kim et al., 1988; WO 89/06242 |
| 4D12 | 8-17 | Allsop et al., 1986; 1990 |
| 2F9 | 17-24 | Kim et al., 1988; Bancher et al., 1989; Kim et al., 1990 |
| 1D2 | 8-17 | Allsop et al., 1986 |
| 1G10 | 8-17 | Allsop et al., 1986; Ikeda et al., 1987 |
| 3B6 | 8-17 | Allsop et al., 1986 |
| 4G5 | 17-24 | Kim et al., 1988; Bancher et al., 1989 |
| 4E11 | 17-24 | Kim et al., 1988; Bancher et al., 1989 |
| 2B8 | 17-24 | Kim et al., 1988; Bancher et al., 1989 |
| BNT 77 | 11-16 | Asami-Odaka et al., 1995; Tamaoka et al., 1997; Enya et al., 1999; Kawarabayashi et al., 2001; Oshima et al., 2001 |
| WO2 | 5-8 | Ida et al., 1996; Jensen et al., 2000 |
| 6F/3D | 8-17 | Tanzi et al., 1988; Thal et al., 1999; DakoCytomation N.V., Heverlee, Belgium, Novocastra Laboratories Ltd, Newcastle upon Tyne, UK |
| Hyb310-01 | 10-16 | Antibodyshop, Copenhagen, Denmark |
| Hyb310-03 | 9-10 | Antibodyshop, Copenhagen, Denmark |
| Hyb310-04 | 10-16 | Antibodyshop, Copenhagen, Denmark |
| Hyb310-07 | 5-9 | Antibodyshop, Copenhagen, Denmark |
| Hyb310-08 | 10-16 | Antibodyshop, Copenhagen, Denmark |
| 8E1 | N3pE | IBL-Hamburg, Hamburg, Germany |

**Table 2. N-terminaly truncated Aβ peptides that have been identified.**

| **Aβ peptide** | **Source** | **Reference** |
|---|---|---|
| 2-13 | Transfected (APP) mouse neuroblastoma (N2a) cells | Wang et al., 1996 |
| 2-14 | Transfected (APP) mouse neuroblastoma (N2a) cells | Wang et al., 1996 |
| 2-15 | Transfected (APP) mouse neuroblastoma (N2a) cells | Wang et al., 1996 |
| 2-16 | Brain of AD subject | Sergeant et al., 2003 |
| 2-18 | Transfected (APP) mouse neuroblastoma (N2a) cells | Wang et al., 1996 |
| 2-19 | Transfected (APP) mouse neuroblastoma (N2a) cells | Wang et al., 1996 |
| 2-20 | Transfected (APP) mouse neuroblastoma (N2a) cells | Wang et al., 1996 |
| 2-36 | Aβ deposits in AD | Kalback et al., 2002 |
| 2-37 | Aβ deposits in AD | Kalback et al., 2002 |
| 2-38 | Aβ deposits in AD | Kalback et al., 2002 |
| 2-39 | Aβ deposits in AD | Kalback et al., 2002 |
| 2-40 | Aβ deposits in AD | Kalback et al., 2002 |
| 2-42 | Neuroglioma H4 or primary mouse neurons, transfected | Wiltfang et al., 2001 |
| | Detergent (RIPA) soluble fraction of AD brains | Wiltfang et al., 2001 |
| | Aβ deposits in AD | Kalback et al., 2002 |
| 3-14 | Transfected (APP) mouse neuroblastoma (N2a) cells | Wang et al., 1996 |
| 3-16 | Brain of AD subject | Sergeant et al., 2003 |
| 3-34 | CSF-pool | Vigo-Pelfrey et al., 1993 |
| 3-38 | Aβ deposits in AD | Kalback et al., 2002 |
| 3-40 | Human kidney 293 cells | Haass et al., 1992 |
| | AD brain | Näslund et al., 1994 |
| | Human kidney 293 cells | Cescato et al., 2000 |
| 3p-40 | Aβ deposits | Harigaya et al., 2000 |
| | Pooled brain homogenate | Wiltfang et al., 2001 |
| | Aβ deposits in AD | Kalback et al., 2002 |
| 3-42 | AD brain | Näslund et al., 1994 |
| | Aβ deposits in AD | Kalback et al., 2002 |
| 3p-42 | Aβ deposits | Harigaya et al., 2000 |
| | Pooled brain homogenate | Wiltfang et al., 2001 |
| | Aβ deposits in AD | Kalback et al., 2002 |
| 3p-C | Aβ deposits | Saido et al., 1995; 1996 |
| | Diffuse plaques | Iwatsubo et al., 1996 |
| | Brain extracts | Russo et al., 1997 |
| | AD brain | Tekirian et al., 1998 |
| 4-14 | Transfected (APP) mouse neuroblastoma (N2a) cells | Wang et aL, 1996 |
| 4-16 | Brain of AD subject | Sergeant et al., 2003 |
| 4-18 | Transfected (APP) mouse neuroblastoma (N2a) cells | Wang et aL, 1996 |
| 4-19 | Transfected (APP) mouse neuroblastoma (N2a) cells | Wang et aL, 1996 |
| 4-42 | Cortical brain | Näslund et al., 1994 |
| | Aβ deposits in AD | Kalback et aL, 2002 |
| 5-16 | Brain of AD subject | Sergeant et al., 2003 |
| 5-27 | Transfected (APP) mouse neuroblastoma (N2a) cells | Wang et al., 1996 |
| 5-29 | Transfected (APP) mouse neuroblastoma (N2a) cells | Wang et al., 1996 |
| 5-34 | Non-transfected (APP) mouse neuroblastoma (N2a) cells | Wang et al., 1996 |
| 5-36 | Non-transfected (APP) mouse neuroblastoma (N2a) cells | Wang et al., 1996 |
| 5-40 | Human kidney 293 cells | Cescato et al., 2000 |
| | Aβ deposits in AD | Kalback et al., 2002 |
| 6-27 | CSF-pool | Vigo-Pelfrey et al., 1993 |
| | Transfected (APP) mouse neuroblastoma (N2a) cells | Wang et aL, 1996 |
| | Non-transfected (APP) mouse neuroblastoma (N2a) cells | Wang et al., 1996 |
| 6-34 | CSF-pool | Vigo-Pelfrey et al., 1993 |
| | Transfected (APP) mouse neuroblastoma (N2a) cells | Wang et al., 1996 |
| 6-35 | CSF-pool | Vigo-Pelfrey et al., 1993 |
| | Aβ deposits in AD | Kalback et al., 2002 |
| 6-42 | CSF-pool | Vigo-Pelfrey et al., 1993 |
| | AD brain | Näslund et al., 1994 |
| | Aβ deposits in AD | Kalback et al., 2002 |
| 7-40 | Aβ deposits in AD | Kalback et al., 2002 |
| 7-42 | AD brain | Näslund et al., 1994 |
| | Aβ deposits in AD | Kalback et al., 2002 |
| 8-16 | Brain of AD subject | Sergeant et al., 2003 |
| 8-34 | Transfected (APP) mouse neuroblastoma (N2a) cells | Wang et al., 1996 |
| 8-40 | Aβ deposits in AD | Kalback et al., 2002 |
| 8-42 | Cortical brain | Näslund et al., 1994 |
| | Aβ deposits in AD | Kalback et al., 2002 |
| 9-16 | Brain of AD subject | Sergeant et al., 2003 |
| 9-42 | AD brain | Näslund et al., 1994 |
| | Aβ deposits in AD | Kalback et al., 2002 |
| 10-16 | Brain of AD subject | Sergeant et al., 2003 |
| 10-40 | Aβ deposits in AD | Kalback et al., 2002 |
| 10-42 | Aβ deposits in AD | Kalback et al., 2002 |
| 11-27 | Non-transfected (APP) mouse neuroblastoma (N2a) cells | Wang et al., 1996 |
| 11-28 | Non-transfected (APP) mouse neuroblastoma (N2a) cells | Wang et al., 1996 |
| 11-33 | Non-transfected (APP) mouse neuroblastoma (N2a) cells | Wang et al., 1996 |
| 11-34 | CSF-pool | Vigo-Pelfrey et al., 1993 |
| | Non-transfected (APP) mouse neuroblastoma (N2a) cells | Wang et al., 1996 |
| 11-37 | Non-transfected (APP) mouse neuroblastoma (N2a) cells | Wang et al., 1996 |
| 11-38 | Non-transfected (APP) mouse neuroblastoma (N2a) cells | Wang et al., 1996 |
| 11-39 | Non-transfected (APP) mouse neuroblastoma (N2a) cells | Wang et al., 1996 |
| 11-40 | Human kidney 293 cells | Haass et al., 1992 |
| | Non-transfected (APP) mouse neuroblastoma (N2a) cells | Wang et al., 1996 |
| 11-42 | Cortical brain | Näslund et al., 1994 |
| | Aβ deposits in AD | Kalback et aL, 2002 |
| 1 p-42 | Aβ deposits in AD | Kalback et al., 2002 |
| 11-43 | CSF-pool | Vigo-Pelfrey et al., 1993 |
| 11-C | CSF | Seubert et al., 1992 |
| 11p-C | Brain extracts | Russo et al., 1997 |
| 12-33 | Non-transfected (APP) mouse neuroblastoma (N2a) cells | Wang et al., 1996 |
| 12-34 | Non-transfected (APP) mouse neuroblastoma (N2a) cells | Wang et aL, 1996 |
| 12-37 | Non-transfected (APP) mouse neuroblastoma (N2a) cells | Wang et al., 1996 |
| 12-38 | Non-transfected (APP) mouse neuroblastoma (N2a) cells | Wang et al., 1996 |
| 12-40 | Non-transfected (APP) mouse neuroblastoma (N2a) cells | Wang et aL, 1996 |
| 12-43 | CSF-pool | Vigo-Pelfrey et al., 1993 |
| 14-34 | Transfected (APP) mouse neuroblastoma (N2a) cells | Wang et al., 1996 |
| 14-38 | Transfected (APP) mouse neuroblastoma (N2a) cells | Wang et al., 1996 |
| 14-40 | Transfected (APP) mouse neuroblastoma (N2a) cells | Wang et al., 1996 |
| 14-42 | Transfected (APP) mouse neuroblastoma (N2a) cells | Wang et al., 1996 |
| | Aβ deposits in AD | Kalback et al., 2002 |
| 15-34 | Transfected (APP) mouse neuroblastoma (N2a) cells | Wang et al., 1996 |
| 15-38 | Transfected (APP) mouse neuroblastoma (N2a) cells | Wang et al., 1996 |
| 15-40 | Transfected (APP) mouse neuroblastoma (N2a) cells | Wang et al., 1996 |
| 16-34 | Transfected (APP) mouse neuroblastoma (N2a) cells | Wang et aL, 1996 |
| 16-40 | Aβ deposits in AD | Kalback et al., 2002 |
| 16-42 | Aβ deposits in AD | Kalback et al., 2002 |
| 17-34 | Transfected (APP) mouse neuroblastoma (N2a) cells | Wang et al., 1996 |
| 17-40 | Human kidney 293 cells | Haass et al., 1992 |
| | Transfected (APP) mouse neuroblastoma (N2a) cells | Wang et al., 1996 |
| | Non-transfected (APP) mouse neuroblastoma (N2a) cells | Wang et al., 1996 |
| | Aβ deposits in AD | Kalback et al., 2002 |
| 17-42 | Aβ deposits in AD | Kalback et al., 2002 |
| 18-40 | Human kidney 293 cells | Haass et al., 1992 |
| | Aβ deposits in AD | Kalback et al., 2002 |
| 19-42 | Aβ deposits in AD | Kalback et al., 2002 |

| | | |
|---|---|---|
| p: pyroglutamate; C: C-terminal end of Aβ is not defined. | | |

**Table 3. Antibodies to be used as detector antibody in the methods of the present invention.**

| **Antibody** | **Specificity** | **Reference** |
|---|---|---|
| 21F12 | Aβ₄₂ | Bard et al., 2000; Vanderstichele et al., 2000; Brayden et al., 2001; Elan Pharmaceuticals, South San Fransisco, CA, US; Innogenetics N.V., Ghent, Belgium |
| 4D7A3 | Aβ₄₂ | Innogenetics N.V., Ghent, Belgium |
| 13E9 | Aβ₄₀ | Wiltfang et al., 2002; Shering AG, Berlin, Germany |
| 6D5 | Aβ₄₂ | Wiltfang et al., 2002; Shering AG, Berlin, Germany |
| BA-27 | Aβ₄₂ | Iwatsubo et al., 1994; Suzuki et al., 1994; Tabaton et al., 1994; Gravina et al., 1995; Fukumoto et al., 1996; Tamaoka et al., 1997; Enya et al., 1999; Harigaya et al., 2000; Kawarabayashi et al., 2001; Oshima et al., 2001 |
| BC-05 | Aβ₄₀ | Iwatsubo et al., 1994; Suzuki et al., 1994; Tabaton et al., 1994; Gravina et al., 1995; Fukumoto et al., 1996; Tamaoka et al., 1997; Enya et al., 1999; Harigaya et al., 2000; Kawarabayashi et al., 2001; Oshima et al., 2001 |
| G2-10 | Aβ₄₀ | Ida et al., 1996; Jensen et al., 2000 |
| G2-11 | Aβ₄₂ | Ida et al., 1996; Jensen et al., 2000 |
| 369.2B | Aβ₄₂ | König et al., 1996 |
| 108.1 | Aβ₄₂ | Murphy et al., 1994 |

**Table 4. Molecular masses and mass accuracy of Aβ peptides detected in human CSF.**

| **Suggested peptide** | **Mean mass measured (Da)** | **Theoretical mass (Da)** | **Mass accuracy (ppm)** |
|---|---|---|---|
| Aβ 1-42 | 4513.93 | 4514.1 | 37 |
| Aβ 11-42 | 3335.99 | 3335.9 | 22 |
| Aβ 8-42 | 3643.2 | 3643.2 | 8 |
| Aβ 8-42ox | 3654.05 | 3659.2 | 1415 |
| Aβ 5-42 | 4051.12 | 4051.6 | 126 |

| | | | |
|---|---|---|---|
| ox: oxidized form. | | | |

**Table 5. Aβ₄₂ peptides in CSF of 42 clinical cases. Data present peak heights or intensities detected by SELDI-TOF after immunocapture with monoclonal antibody 4D7A3 (ox; oxidize forms).**

| | **Peak heights** not normalized | | | | | | |
|---|---|---|---|---|---|---|---|
| | 1-42+2H | 11-42 | 8-42ox | 5-42 | 5-42ox | 3-42ox | 1-42 |
| AD 001 | 4,04 | 2,45 | 0,51 | 0,62 | 0,63 | 1,1 | 16,52 |
| AD002 | 1,65 | 1,96 | 0,96 | 0,87 | 0,71 | 0,43 | 11,57 |
| AD 003 | 6,23 | 4,99 | 0,65 | 1,08 | 0,59 | 1,23 | 29,67 |
| AD 004 | 7,1 | 4,04 | 1,04 | 0,61 | 0,18 | 0,43 | 11,73 |
| AD 005 | 4,87 | 2,48 | 0,47 | 0,56 | 0,62 | 0,88 | 14,88 |
| AD 006 | 2,9 | 1,35 | 0,79 | 0,56 | 0,38 | 0,32 | 6,19 |
| AD 007 | 4,81 | 3,98 | 1,28 | 0,88 | 0,65 | 0,85 | 20,93 |
| AD008 | 4.1 | 5,38 | 1,48 | 0,74 | 0,52 | 1,22 | 21,91 |
| AD 009 | 4,02 | 4,01 | 1,59 | 0,71 | 0,4 | 0,99 | 22,45 |
| AD010 | 4,8 | 4,59 | 0,72 | 0,51 | 0,49 | 0,76 | 20,04 |
| AD 011 | 4,73 | 5,18 | 0,75 | 1,12 | 0,45 | 0,83 | 32,31 |
| AD012 | 2,7 | 2,68 | 1,54 | 0,62 | 0,68 | 0,72 | 13,73 |
| AD 013 | 2,35 | 1,97 | 0,67 | 0,53 | 0,78 | 0,88 | 16,1 |
| AD 014 | 1,27 | 0,98 | 0,75 | 0,43 | 0,58 | 0,38 | 3,49 |
| AD 015 | 1,73 | 2,2 | 0,65 | 0,45 | 0,48 | 0,42 | 6,15 |
| AD 016 | 8,45 | 7,9 | 0,94 | 1,49 | 0,71 | 1,36 | 49,83 |
| AD017 | 2,46 | 1,69 | 0,75 | 0,57 | 0,9 | 0,64 | 11,82 |
| AD018 | 2,74 | 2,17 | 1,28 | 0,27 | 0,36 | 0,69 | 8,83 |
| AD 019 | 1,56 | 1,07 | 1,13 | 0,46 | 0,71 | 0,99 | 4,83 |
| AD 020 | 5,08 | 5,07 | 0,33 | 1,2 | 0,68 | 0,65 | 25,3 |
| AD 021 | 1,09 | 0,97 | 1,62 | 0,78 | 0,71 | 0,48 | 4,36 |
| AD 022 | 4,88 | 6,08 | 1,47 | 0,66 | 0,77 | 0,63 | 27 |
| Controle 34384 | 1,06 | 0,91 | 5,58 | 0,52 | 2,24 | 0,46 | 3,25 |
| Controle 34393 | 1,5 | 1,09 | 1,19 | 0,62 | 0,71 | 0,26 | 3,94 |
| Controle 34411 | 1,99 | 1,37 | 1,89 | 0,24 | 0,9 | 0,69 | 7,75 |
| Controle 34417 | 4,92 | 3,07 | 0,89 | 0,76 | 0,94 | 0,69 | 22,92 |
| Controle 34431 | 1,95 | 0,93 | 0,87 | 0,35 | 0,89 | 0,43 | 8,75 |
| Controle 9002163 | 2.85 | 1,71 | 1,49 | 0.46 | 0,55 | 0.36 | 8.52 |
| Controle 34460 | 3,78 | 0.76 | 2,09 | 0.67 | 0.83 | 0,55 | 16,53 |
| Controle 34490 | 9,3 | 4,19 | 1,25 | 0,71 | 0,44 | 1,5 | 38,38 |
| Controle 34493 | 6,59 | 2.67 | 1.05 | 0.79 | 0.42 | 0,92 | 27,55 |
| Controle 34500 | 1.81 | 1,23 | 1,82 | 0.24 | 1.41 | 0,61 | 7,07 |
| Controls 34610 | 2,29 | 1.56 | 0.64 | 0.49 | 0.23 | 0.7 | 8.35 |
| Controle 34667 | 4.31 | 1,82 | 1,21 | 0.44 | 0.53 | 0,71 | 15,99 |
| Controls 34681 | 3.24 | 2.6 | 0,72 | 0.87 | 0.54 | 0,94 | 16,27 |
| Controls 34699 | 2,69 | 1.56 | 1,92 | 0,75 | 0,32 | 0,69 | 10,08 |
| Controls 34861 | 7,61 | 2,73 | 1,63 | 0,37 | 0,58 | 0,88 | 44,65 |
| Controle 34951 | 4,82 | 5,35 | 0,95 | 0,66 | 0,29 | 1,47 | 25,17 |
| Controle 34993 | 4,04 | 3,32 | 0,3 | 0,48 | 0,8 | 0,63 | 29,61 |
| Controle 35007 | 2,11 | 1,12 | 0,33 | 0,87 | 0,99 | 0,41 | 7,83 |
| Controle 34401 | 3,67 | 0,8 | 1,37 | 0,18 | 0,68 | 0,63 | 16,5 |
| Controle 99002240 | 4,37 | 2,37 | 1,89 | 0,63 | 0,46 | 4,01 | 21,52 |

### REFERENCES

Allsop D., Landon M., Kidd M., Lowe J.S., Reynolds G.P., Gardner A. (1986) Monoclonal antibodies raised against a subsequence of senile plaque core protein react with plaque cores, plaque periphery and cerebrovascular amyloid in Alzheimer's disease. Neurosci. Lett. 68: 252-256.
Allsop D., Haga S., Bruton C., Ishii T., Roberts G.W. (1990) Neurofibrillary tangles in some cases of dementia pugilistica share antigens with amyloid beta-protein of Alzheimer's disease. Am. J. Pathol. 136: 255-260.
American Psychiatric Association (1994) Diagnostic and statistical manual of mental disorders, 5th edn. Washington, DC: American Psychiatric Association.
Anderson J.P., Chen Y., Kim K.S., Robakis N.K. (1992) An alternative secretase cleavage produces soluble Alzheimer amyloid precursor protein containing a potentially amyloidogenic sequence. J. Neurochem. 59: 2328-2331.
Andreasen N., Vanmechelen E., Van de Voorde A., Davidsson P., Hesse C., Tarvonen S., Raiha L, Sourander L., Winblad B., Blennow K. (1998) Cerebrospinal fluid tau protein as a biochemical marker for Alzheimer's disease: a community based follow up study. J. Neurol. Neurosurg. Psychiatry 64: 298-305.
Andreasen N., Hesse C., Davidsson P., Minthon L., Wallin A., Winblad B., Vanderstichele H., Vanmechelen E., Blennow K. (1999a) Cerebrospinal fluid beta-amyloid(1-42) in Alzheimer disease: differences between early- and late-onset Alzheimer disease and stability during the course of disease. Arch. Neurol. 56: 673-680.
Andreasen N., Minthon L., Vanmechelen E., Vanderstichele H., Davidsson P., Winblad B., Blennow K. (1999b) Cerebrospinal fluid tau and Aβ42 as predictors of development of Alzheimer's disease in patients with mild cognitive impairment. Neurosci. Lett. 273: 5-8.
Andreasen N., Vanmechelen E., Vanderstichele H., Davidsson P., Blennow K. (2003) Cerebrospinal fluid levels of total-tau, phospho-tau and Aβ42 predicts development of Alzheimer's disease in patients with mild cognitive impairment Acta Neurol. Scand. 107 (Suppl. 179): 47-51.
Arai H., Ishiguro K., Ohno H., Moriyama M., Itoh N., Okamura N., Matsui T., Morikawa Y., Horikawa E., Kohno H., Sasaki H., Imahori K. (2000) CSF phosphorylated tau protein and mild cognitive impairment: a prospective study. Exp. Neurol. 166: 201-203.
Asami-Odaka A., Ishibashi Y., Kikuchi T., Kitada C., Suzuki N. (1995) Long amyloid beta-protein secreted from wild-type human neuroblastoma IMR-32 cells. Biochemistry 34: 10272-10278.
Austen B.M., Frears E.R., Davies H. (2000) The use of seldi proteinchip arrays to monitor production of Alzheimer's betaamyloid in transfected cells. J. Pept. Sci. 6: 459-469.
Bancher C., Grundke-Iqbal I., Iqbal K., Kim K.S., Wisniewski H.M. (1989) Immunoreactivity of neuronal lipofuscin with monoclonal antibodies to the amyloid beta-protein. NeurobioL Aging 10: 125-132.
Bard F., Cannon C., Barbour R., Burke R.L., Games D., Grajeda H., Guido T., Hu K., Huang J., Johnson-Wood K., Khan K., Kholodenko D., Lee M., Lieberburg I., Motter R., Nguyen M., Soriano F., Vasquez N., Weiss K., Welch B., Seubert P., Schenk D., Yednock T. (2000) Peripherally administered antibodies against amyloid beta-peptide enter the central nervous system and reduce pathology in a mouse model of Alzheimer disease. Nat. Med. 6: 916-919.
Blennow K., Wallin A., Agren H., Spenger C., Siegfried J., Vanmechelen E. (1995) Tau protein in cerebrospinal fluid: a biochemical marker for axonal degeneration in Alzheimer's disease? Mol. Chem. Neuropathol. 26: 231-235.
Brayden D.J., Templeton L., McClean S., Barbour R, Huang J., Nguyen M., Ahern D., Motter R, Johnson-Wood K., Vasquez N., Schenk D., Seubert P. (2001) Encapsulation in biodegradable microparticles enhances serum antibody response to parenterally-delivered beta-amyloid in mice. Vaccine 19: 4185-4193.
Buerger K., Teipel S.J., Zinkowski R, Blennow K., Arai H., Engel R, Hofinann-Kiefer K., McCulloch C., Ptok U., Heun R., Andreasen N., DeBernardis J., Kerkman D., Moeller H.-J., Davies P., Hampel H. (2002) CSF tau protein phsophorylated at threonine 231 correlates with cognitive decline in MCI subjects. Neurology 59: 627-629.
Cescato R, Dumermuth E., Spiess M., Paganetti P.A. (2000) Increased generation of alternatively cleaved beta-amyloid peptides in cells expressing mutants of the amyloid precursor protein defective in endocytosis. J. Neurochem. 74: 1131-1139.
Chertkow H. (2002) Mild cognitive impairment. Curr. Opin. Neurol. 15: 401-407.
Citron M., Oltersdorf T., Haass C., McConlogue L., Hung A.Y., Seubert P., Vigo-Pelfrey C., Lieberburg I., Selkoe D.J. (1992) Mutation of the beta-amyloid precursor protein in familial Alzheimer's disease increases beta-protein production. Nature 360: 672-674.
Cole et al. (1985) In: Monoclonal Antibodies and Cancer Therapy. Alan R Liss, Inc. pp. 77-96.
Conde S. (2002) β-amyloid peptide as a target for treatment of Alzheimer's disease. Expert Opin. Ther. Patents 12: 503-512.
Cortez-Retamozo V., Lauwereys M., Hassanzadeh Gh. G., Gobert M., Conrath K., Muyldermans S., De Baetselier P., Revets H. (2002) Efficient tumor targeting by single-domain antibody fragments of camels. Int. J. Cancer 98: 456-462.
Davidsson P., Westman A., Puchades M., Nilsson C.L., Blennow K. (1999) Characterization of Proteins from Human Cerebrospinal Fluid by a Combination of Preparative Two-Dimensional Liquid-Phase Electrophoresis and Matrix-Assisted Laser Desorption/Ionization Time-of-Flight Mass Spectrometry. Anal. Chem. 71: 642-647.
Davies H., Lomas .L, Austen B. (1999) Profiling of amyloid beta peptide variants using SELDI Protein Chip arrays. Biotechniques 27: 1258-1261.
De Jonghe C., Esselens C., Kumar-Singh S., Craessaerts K., Serneels S., Checler F., Annaert W., Van Broeckhoven C., De Strooper B. (2001) Pathogenic APP mutations near the gamma-secretase cleavage site differentially affect Abeta secretion and APP C-terminal fragment stability. Hum. Mol. Genet. 10: 1665-1671.
DeMattos R.B., Bales K.R, Cummins D.J., Dodart J.C., Paul S.M., Holtzman D.M. (2001) Peripheral anti-A beta antibody alters CNS and plasma A beta clearance and decreases brain A beta burden in a mouse model of Alzheimer's disease. Proc. Natl. Acad. Sci. USA 98: 8850-8855.
Enya M., Morishima-Kawashima M., Yoshimura M., Shinkai Y., Kusui K., Khan K., Games D., Schenk D., Sugihara S., Yamaguchi H., Ihara Y. (1999) Appearance of sodium dodecyl sulfate-stable amyloid beta-protein (Abeta) dimer in the cortex during aging. Am. J. Pathol. 154: 271-279.
Friedland R.P., Majocha R.E., Reno J.M., Lyle L.R., Marotta C.A. (1994) Development of an anti-A beta monoclonal antibody for in vivo imaging of amyloid angiopathy in Alzheimer's disease. Mol. Neurobiol. 9: 107-113.
Fukumoto H., Asami-Odaka A., Suzuki N., Iwatsubo T. (1996) Association of A beta 40-positive senile plaques with microglial cells in the brains of patients with Alzheimer's disease and in non-demented aged individuals. Neurodegeneration 5: 13-17.
Galasko D., Chang L., Motter R., Clark C.M., Kaye J., Knopman D., Thomas R., Kholodenko D., Schenk D., Lieberburg I., Miller B., Green R., Basherad R., Kertiles L., Boss M.A., Seubert P. (1998) High cerebrospinal fluid tau and low amyloid beta42 levels in the clinical diagnosis of Alzheimer disease and relation to apolipoprotein E genotype. Arch. Neurol. 55: 937-945.
Games D., Adams D., Alessandrini R., Barbour R., Berthelette P., Blackwell C., Carr T., Clemens J., Donaldson T., Gillespie F., et al. (1995) Alzheimer-type neuropathology in transgenic mice overexpressing V717F beta-amyloid precursor protein. Nature 373: 523-527.
Ghindilis A.L., Pavlov A.R, Atanassov P.B. (eds.) (2002) Immunoassay Methods and Protocols. Humana Press, Totowa, NJ, US.
Ghiso J., Rostagno A., Gardella J.E., Liem L., Gorevic P.D., Frangione B. (1992) A 109-amino-acid C-terminal fragment of Alzheimer's-disease amyloid precursor protein contains a sequence, -RHDS-, that promotes cell adhesion. Biochem. J. 288: 1053-1059.
Glenner G.G., Wong C.W. (1984) Alzheimer's disease: initial report of the purification and characterization of a novel cerebrovascular amyloid protein. Biochem. Biophys. Res. Commun. 120: 885-890.
Goedert M. (1996) Tau protein and the neurofibrillary pathology of Alzheimer's disease. Ann. N Y Acad. Sci. 777: 121-131.
Gravina S.A., Ho L., Eckman C.B., Long K.E., Otvos L. Jr., Younkin L.H., Suzuki N., Younkin S.G. (1995) Amyloid beta protein (A beta) in Alzheimer's disease brain. Biochemical and immunocytochemical analysis with antibodies specific for forms ending at A beta 40 or A beta 42(43). J. Biol. Chem. 270: 7013-7016.
Haass C., Schlossmacher M.G., Hung A.Y., Vigo-Pelfrey C., Mellon A., Ostaszewski B.L., Lieberburg I., Koo E.H., Schenk D., Teplow D.B., Selkoe D.J. (1992) Amyloid beta-peptide is produced by cultured cells during normal metabolism. Nature 359: 322-325.
Harigaya Y., Saido T.C., Eckman C.B., Prada C.-M., Shoji M., Younkin S.G. (2000) Amyloid β Protein Starting Pyroglutamate at Position 3 Is a Major Component of the Amyloid Deposits in the Alzheimer's Disease Brain. Biochemical and Biophysical Research Communications 276: 422-427.
Harrington C.R, Quinn G.B., Hurt J., Day I.N., Wischik C.M., (1993) Characterisation of an epitope specific to the neuron-specific isoform of human enolase recognised by a monoclonal antibody raised against a synthetic peptide corresponding to the C-terminus of beta/A4-protein. Biochim. Biophys. Acta 1158: 120-128.
Harrington C.R., Perry R.H., Perry E.K., Hurt J., McKeith I.G., Roth M., Wischik C.M. (1994) Senile dementia of Lewy body type and Alzheimer type are biochemically distinct in terms of paired helical filaments and hyperphosphorylated tau protein. Dementia 5: 215-228.
Hulstaert F., Blennow K., Ivanoiu A., Schoonderwaldt H.C., Riemenschneider M., De Deyn P.P., Bancher C., Cras P., Wiltfang J., Mehta P.D., Iqbal K., Pottel H., Vanmechelen E., Vanderstichele H. (1999) Improved discrimination of AD patients using β-amyloid(1-42) and tau in CSF: a multicenter study. Neurology 52: 1555-1562.
Ida N., Hartmann T., Pantel J., Schröder J., Zerfass R., Först H., Sandbrink R., Masters C.L., Beyreuther K. (1996) Analysis of heterogeneous βA4 peptides in human cerebrospinal fluid and blood by a newly developed sensitive Western blot assay. J. Biol. Chem. 271: 22908-22914.
Ikeda S., Wong C.W., Allsop D., Landon M., Kidd M., Glenner G.G. (1987) Immunogold labeling of cerebrovascular and neuritic plaque amyloid fibrils in Alzheimer's disease with an anti-beta protein monoclonal antibody. Lab. Invest. 57: 446-449.
Iwatsubo T., Odaka A., Suzuki N., Mizusawa H., Nukina N., Ihara Y. (1994) Visualization of A beta 42(43) and A beta 40 in senile plaques with end-specific A beta monoclonals: evidence that an initially deposited species is A beta 42(43). Neuron 13: 45-53.
Iwatsubo T., Saido T.C., Mann D.M.A., Lee V.M.-Y., Trojanowski J.Q. (1996) Full-length amyloid-β(1-42(43)) and amino terminally modified and truncated amyloid-β42(43) deposit in diffuse plaques. Am. J. Pathol. 149: 1823-1830.
Jensen M., Hartmann T., Engvall B., Wang R., Uljon S.N., Sennvik K., Naslund J., Muehlhauser F., Nordstedt C., Beyreuther K., Lannfelt L. (2000) Quantification of Alzheimer amyloid beta peptides ending at residues 40 and 42 by novel ELISA systems. Mol. Med.6: 291-302.
Kalback W., Watson M.D., Kokjohn, T.A., Kuo Y.-M., Weiss N., Luehrs D.C., Lopez J., Brune D., Sisodia S.S., Staugenbiel M., Emmerling M., Roher A.E. (2002) APP transgenic mice Tg2576 accumulate Abeta peptides that are distinct from the chemically modified and insoluble peptides deposited in Alzheimer's disease senile plaques. Biochemistry 41: 922-928.
Kanai M., Matsubara E., Isoe K., Urakami K., Nakashima K., Arai H., Sasaki H., Abe K., Iwatsubo T., Kosaka T., Watanabe M., Tomidokoro Y., Shizuka M., Mizushima K., Nakamura T., Igeta Y., Ikeda Y., Amari M., Kawarabayashi T., Ishiguro K., Harigaya Y., Wakabayashi K., Okamoto K., Hirai S., Shoji M. (1998) Longitudinal study of cerebrospinal fluid levels of tau, A betal-40, and A betal-42(43) in Alzheimer's disease: a study in Japan. Ann. Neurol. 44: 17-26.
Kawarabayashi T., Younkin L.H., Saido T.C., Shoji M., Ashe K.H., Younkin S.G. (2001) Age-dependent changes in brain, CSF, and plasma amyloid (beta) protein in the Tg2576 transgenic mouse model of Alzheimer's disease. J. Neurosci. 21: 372-381.
Kida E., Wisniewski K.E., Wisniewski H.M. (1995) Early amyloid-beta deposits show different immunoreactivity to the amino- and carboxy-terminal regions of beta-peptide in Alzheimer's disease and Down's syndrome brain. Neurosci. Lett. 193: 105-108.
Kim K.S., Miller D.L., Sapienza V.J., Chen C.-M. J., Bai C., Grundke-Iqbal I., Currie J.R., Wisniewski H.M. (1988) Production and characterization of monoclonal antibodies reactive to synthetic cerebrovascular amyloid peptide. In: Neuroscience Research Communications, published by John Wiley & Sons, Ltd. Vol. 2, No. 3, pages 121-130.
Kim K.S., Wen G.Y., Bancher C., Chen C.M.J., Sapienza V.J., Hong H., Wisniewski H.M. (1990) Detection and quantitation of amyloid b-peptide with 2 monoclonal antibodies. In: Neuroscience Research Communications, published by John Wiley & Sons, Ltd. Vol. 7, No. 2, pages 113-122.
Knopman D., Ritchie K., Polge C., Alafuzoff I., Soininen H. (2002) Alzheimer's disease. In: Qizilbash N. (ed.) Evidence-based Dementia Practice. Oxford: Blackwell Science, p. 228-259.
Kohler G., Milstein C. (1975) Continuous cultures of fused cells secreting antibody of predefined specificity. Nature 256: 495-497.
Konig G., Graham P., Bushnell A., Webster S., Wunderlich D., Perlmutter L.S. (1996) Development and characterization of a monoclonal antibody 369.2B specific for the carboxyl-terminus of the beta A4 peptide. Ann. NY Acad. Sci. 777: 34.4-355.
Kozbor D., Dexter D., Roder J.C. (1983) A comparative analysis of the phenotypic characteristics of available fusion partners for the construction of human hybridomas. Hybridoma 2:7-16.
Langston J.W., Widner H., Goetz C.G., Brooks D., Fahn S., Freeman T., Watts R. (1992) Core assessment program for intracerebral transplantations (CAPIT). Mov. Disord .7: 2-13.
Lamer A.J. (1999) Hypothesis: amyloid β-peptides truncated at the N-terminus contribute to the pathogenesis of Alzheimer's disease. Neurobiol. Aging 20: 65-69.
Lewczuk P., Esselmann H., Meyer M., Wollscheid V., Neumann M., Otto M., Maler J.M., Ruther E., Kornhuber J., Wiltfang J. (2003) The amyloid-beta (Abeta) peptide pattern in cerebrospinal fluid in Alzheimer's disease: evidence of a novel carboxyterminally elongated Abeta peptide. Rapid Commun. Mass Spectrom. 17: 1291-1296.
Lewczuk P., Esselmann H., Groemer T.W., Bibl M., Maler J.M., Steinacker P., Otto M., Kornhuber J., Wiltfang J. (2004) Amyloid beta peptides in cerebrospinal fluid as profiled with surface enhanced laser desorption/ionization time-of-flight mass spectrometry: evidence of novel biomarkers in Alzheimer's disease. Biol. Psychiatry 55: 524-530.
Leys D., Englund E., Erkinjuntii T. (2002) Vascular dementia. In: Qizilbash.N. (ed.) Evidence-based Dementia Practice. Oxford: Blackwell Science, p. 260-287.
Lowe J. (2001) The pathological diagnosis of neurodegenerative diseases causing dementia. Curr. Top. Pathol. 95: 149-177.
Majocha RE., Reno J.M., Friedland RP., VanHaight C., Lyle L.R., Marotta C.A. (1992) Development of a monoclonal antibody specific for beta/A4 amyloid in Alzheimer's disease brain for application to in vivo imaging of amyloid angiopathy.J Nucl. Med 33: 2184-2189.
Marin D.B., Sewell M.C., Schlechter A. (2002) Alzheimer's disease: Accurate and early diagnosis in the primary care setting. Geriatrics 57: 36-40.
Masters C.L., Simms G., Weinman N.A., Multhaup G., McDonald B.L., Beyreuther K. (1985) Amyloid plaque core protein in Alzheimer disease and Down syndrome. Proc. Natl. Acad. Sci. USA 82: 4245-4249.
Masur D.M., Sliwinski M., Lipton R.B., Blau A.D., Crystal H.A. (1994) Neuropsychological prediction of dementia and the absence of dementia in healthy elderly persons. Neurology 44: 1427-1432.
McKeith I.G., Galasko D., Kosaka K., Perry E.K., Dickson D.W., Hansen L.A., Salmon D.P., Lowe J., Mirra S.S., Byrne E.J., Lennox G., Quinn N.P., Edwardson J.A., Ince P.G., Bergeron C., Burns A., Miller B.L., Loverstone S., Collerton D., Jansen E.N.H., Ballard C., d Vos R.A.I., Wilcock G.K., Jellinger K.A., Perry RH. (1996) Consensus guidelines for the clinical and pathologic diagnosis of dementia with Lewy bodies (DLB): Report of the consortium on DLB international workshop. Neurology 47: 1113-1124.
McKeith I.G. (2002) Dementia with Lewy bodies. Br. J. Psychiatry 180: 144-147.
McKhann G., Drachman D.A., Folstein M.F., Katzman R, Price D.L., Stadlan E. (1984) Clinical diagnosis of Alzheimer's disease: report of the NINCDS-ADRDA Work Group under the auspices of the Department of Health and Human Services Task Force on Alzheimer's disease. Neurology 34: 939-944.
Metha P.D., Kim K.S., Wisniewski H.M. (1991) ELISA as a laboratory test to aid the diagnosis of Alzheimer's disease. In: Bullock et al., Techniques in diagnostic pathology, Vol. 2, published by Academic Press (London), pages 100-112.
Miller D.L., Papayannopoulos LA., Styles J., Bobin S.A., Lin Y.Y., Biemann K., Iqbal K. (1993) Peptide compositions of the cerebrovascular and senile plaque core amyloid deposits of Alzheimer's disease. Arch. Biochem. Biophys. 15: 41-52.
Monroe et al. (1986) Amer. Clin. Prod. Rev. 5: 34-41.
Mori H., Takio K., Ogawara M., Selkoe D.J. (1992) Mass spectrometry of purified amyloid β protein in Alzheimer's disease. J. Biol. Chem. 267: 17082-17086.
Motter R, Vigo-Pelfrey C., Kholodenko D., Barbour R., Johnson-Wood K., Galasko D., Chang L., Miller B., Clark C., Green R et al. (1995) Reduction of β-amyloid peptide 42 in the cerebrospinal fluid of patients with Alzheimer's disease. Ann. Neurol 38: 643-648.
Murphy G.M. Jr., Forno L.S., Higgins L., Scardina J.M., Eng L.F., Cordell B. (1994) Development of a monoclonal antibody specific for the COOH-terminal of beta-amyloid 1-42 and its immunohistochemical reactivity in Alzheimer's disease and related disorders. Am. J. Pathol. 144: 1082-1088.
Muyldermans S. (2001) Single domain camel antibodies: current status. J. Biotechnol. 74: 277-302.
Näslund J., Schierhom A., Hellman U., Lannfelt L., Roses A.D., Tjernberg L.O., Silberring J., Gandy S.E., Winblad B., Greengard P., Nordstedt C., Terenius L. (1994) Relative abundance of Alzheimer Aβ amyloid peptide variants in Alzheimer disease and normal aging. Proc. Natl. Acad. Sci. USA 91: 8378-8382.
Okamura N., Arai H., Maruyama M., Higuchi M., Matsui T., Tanji H., Seki T., Hirai H., Chiba H., Itoh M., Sasaki H. (2002) Combined analysis of CSF tau levels and [123I]Iodamphetamine SPECT in Mild Cognitive Impairment: implications for a novel predictor of Alzheimer's disease. Am. J. Psychiatry 159: 474-476.
Oshima N., Morishima-Kawashima M., Yamaguchi H., Yoshimura M., Sugihara S., Khan K., Games D., Schenk D., Ihara Y. (2001) Accumulation of amyloid beta-protein in the low-density membrane domain accurately reflects the extent of beta-amyloid deposition in the brain. Am. J. Pathol. 158: 2209-2218.
Palmer K., Fratiglioni L., Winblad B. (2003) What is mild cognitive impairment? Variations in definitions and evolution of nondemented persons with cognitive impairment. Acta Neurol. Scand. 107 (Suppl. 179): 14-20.
Parnetti L., Lanari A., Amici S., Gallai V., Vanmechelen E., Hulstaert F. (2001) CSF phosphorylated tau is a possible marker for discriminating Alzheimer's disease from dementia with Lewy bodies. Phospho-Tau International Study Group. Neurol. Sci.22: 77-78.
Patterson S.D., Aebersold R. (1995) Mass spectrometric approaches for the identification of gel-separated proteins. Electrophoresis 16: 1791-814.
Petersen RC., Smith G.E., Ivnik R.J., Kokmen E., Tangelos E.G. (1994) Memory function in very early Alzheimer's disease. Neurology 44: 867-872.
Petersen R.C., Stevens J.C., Ganguli M., Tangalos E.G., Cummings J.L., DeKosky S.T. (2001) Practice parameter: Early detection of dementia: Mild cognitive impairment (an evidence-based review). Report of the Quality Standards Subcommittee of the American Academy of Neurology. Neurology 56: 1133-1142.
Pirttila T., Kim K.S., Mehta P.D., Frey H., Wisniewski H.M. (1994) Soluble amyloid beta-protein in the cerebrospinal fluid from patients with Alzheimer's disease, vascular dementia and controls. J. Neurol .Sci. 127: 90-95.
Price C.P., Newman D.J. (1997) Principles and practice of immunoassay. 2nd Edition. McMillan Reference Ltd.
Queen C., Schneider W.P., Selick H.E., Payne P.W., Landolfi N.F., Duncan J.F., Avdalovic N.M., Levitt M., Junghans R.P., Waldmann T.A. (1989) A humanized antibody that binds to the interleukin 2 receptor. Proc. Natl. Acad. Sci. USA. 86: 10029-10033.
Riemenschneider M., Lautenschlager N., Wagenpfeil S., Diehl J., Drzezga A., Kurz A. (2002) Cerebrospinal fluid tau and β-amyloid 42 proteins identify Alzheimer disease in subjects with Mild Cognitive Impairment. Arch. Neurol. 59: 1729-1734.
Roher A.E., Lowenson J.D., Clarke S., Woods A.S., Cotter R.J., Gowing E., Ball M. (1993) β-amyloid-(1-42) is a major component of cerebrovascular amyloid deposits: Implications for the pathology of Alzheimer's disease. Proc. Natl. Acad. Sci. USA 90: 10836-10840.
Russo C., Saido T.C., DeBusk L.M., Tabaton M., Gambetti P., Teller J.K. (1997) Heterogeneity of water-soluble amyloid β-peptide in Alzheimer's disease and Down's syndrome brains. FEBS Lett. 409: 411-416.
Saido T.C., Iwatsubo T., Mann DM.A., Shimada H., Ihara Y., Kawashima S. (1995) Dominant and differential deposition of distinct beta-amyloid peptide species, A beta N3(pE); in senile plaques. Neuron 14: 457-466.
Saido T.C., Yamao-Harigaya W., Iwatsubo T., Kawashima S. (1996) Amino- and carboxyl-terminal heterogeneity of beta-amyloid peptides deposited in human brain. Neurosci. Lett. 215: 173-176.
Saido T.C. (2000) Degradation of amyloid-β peptide: a key to Alzheimer pathogenesis, prevention and therapy. Neurosci. News 3: 52-62.
Selkoe D.J. (1991) The molecular pathology of Alzheimer's disease. Neuron 6: 487-498.
Sergeant N., Bombois S., Ghestem A., Drobeck H., Kostanjevecki V., Missiaen C., Wattez A., David J.-P., Vanmechelen E., Sergheraert C., Delacourte A. (2003) Truncated Abeta species in preclinical Alzheimer's disease as new targets for the vaccination approach. J. Neurochem. Accepted.
Seubert P., Vigo-Pelfrey C., Esch F., Lee M., Dovey H., Davis D., Sinha S., Schlossmacher M., Whaley J., Swindlehurst C., et al. (1992) Isolation and quantification of soluble Alzheimer's beta-peptide from biological fluids. Nature 359: 325-327.
Shoji M., Golde T.E., Ghiso J., Cheung T.T., Estus S., Shaffer L.M., Cai X.-D., McKay D.M., Tintner R., Frangione B., Younkin S.G. (1992) Production of the Alzheimer amyloid β protein by normal proteolytic processing. Science 258: 126-129.
Skoog I., Davidsson P., Aevarsson O., Vanderstichele H., Vanmechelen E., Blennow K. (2003) Cerebrospinal Fluid Beta-Amyloid 42 Is Reduced before the Onset of Sporadic Dementia: A Population-Based Study in 85-Year-Olds. Dement Geriatr Cogn Disord. 15: 169-176.
Spillantini M.G., Goedert M., Jakes R, Klug A. (1990) Different configurational states of beta-amyloid and their distributions relative to plaques and tangles in Alzheimer disease. Proc. Natl. Acad. Sci. USA 87: 3947-3951.
Spinelli S., Frenken L.G., Hermans P., Verrips T., Brown K., Tegoni M., Cambillau C. (2000) Camelid heavy-chain variable domains provide efficient combining sites to haptens. Biochemistry.39: 1217-1222.
Sramek J.J., Cutler N.R. (2000) Ongoing trials in Alzheimer's disease. Expert Opin. Investig. Drugs 9: 899-915.
Sunderland T., Wolozin B., Galasko D., Levy J., Dukoff R, Bahro M., Lasser R, Motter R, Lehtimaki T., Seubert P. (1999) Longitudinal stability of CSF tau levels in Alzheimer patients. Biol. Psychiatry 46:750-755.
Sunderland T., Linker G., Mirza N., Putnam K.T., Friedman D.L., Kimmel L.H., Bergeson J., Manetti G.J., Zimmermann M., Tang B., Bartko J.J., Cohen RM. (2003) Decreased β-amyloid1-42 and increased tau levels in cerebrospinal fluid of patients with Alzheimer disease. JAMA 289: 2094-2103.
Suzuki N., Cheung T.T., Cai X.D., Odaka A., Otvos L. Jr., Eckman C., Golde T.E., Younkin S.G. (1994) An increased percentage of long amyloid beta protein secreted by familial amyloid beta protein precursor (beta APP717) mutants. Science. 264: 1336-1340.
Szendrei G.I., Prammer K.V., Vasko M., Lee V.M., Otvos L. Jr. (1996) The effects of aspartic acid-bond isomerization on in vitro properties of the amyloid beta-peptide as modeled with N-terminal decapeptide fragments. Int. J. Pept. Protein Res. 47: 289-296.
Tabaton M., Nunzi M.G., Xue R., Usiak M., Autilio-Gambetti L., Gambetti P. (1994) Soluble amyloid beta-protein is a marker of Alzheimer amyloid in brain but not in cerebrospinal fluid. Biochem. Biophys. Res. Commun. 200: 1598-1603.
Tamaoka A., Sawamura N., Fukushima T., Shoji S., Matsubara E., Shoji M., Hirai S., Furiya Y., Endoh R, Mori H. (1997) Amyloid β protein 42(43) in cerebrospinal fluid of patients with Alzheimer's disease. J Neurol. Sci. 148: 41-45.
Tanzi R.E., McClatchey A.I., Lamperti E.D., Villa-Komaroff L., Gusella J.F., Neve R.L. (1988) Protease inhibitor domain encoded by an amyloid protein precursor mRNA associated with Alzheimer's disease. Nature 331: 528-530.
Tapiola T., Overmyer M., Lehtovirta M., Helisalmi S., Ramberg J., Alafuzoff I., Riekkinen P. Sr., Soininen H. (1997) The level of cerebrospinal fluid tau correlates with neurofibrillary tangles in Alzheimer's disease. Neuroreport. 8: 3961-3963.
Tekirian T.L., Saido T.C., Markesbery W.R, Russell M.J., Wekstein D.R, Patel E., Geddes J.W. (1998) N-terminal heterogeneity ofparenchymal and cerebrovascular Aβ deposits. J. Neurpathol. Exp. Neurol. 57: 76-94.
Thal D.R., Sassin I., Schultz C., Haass C., Braak E., Braak H. (1999) Fleecy amyloid deposits in the internal layers of the hupman enthorhinal cortex are comprised of N-terminal truncated fragments of Aβ. J. Neuropathol. Exp. Neurol. 58: 210-216.
Thal L.J. (2000) Trials to slow progression and prevent disease onset. J. Neural. Transm. Suppl. 59: 243-249.
The Ronald and Nancy Reagan Research Institute of the Alzheimer's Association of the National Institute on Aging Working Group. (1998) Consensus Report of the Working Group on Molecular and Biochemical Markers of Alzheimer's Disease. Neurobiol. Aging 19: 109-116.
Vanderstichele H., Van Kerschaver E., Hesse C., Davidsson P., Buyse M.A., Andreasen N., Minthon L., Wallin A., Blennow K., Vanmechelen E. (2000) Standardization of measurement of beta-amyloid(1-42) in cerebrospinal fluid and plasma. Amyloid 7: 245-58.
Vanmechelen E., Van Kerschaver E., Blennow K., De Deyn P.P., Galasko D., Parnetti L., Sindic C.J. M., Arai H., Riemenschneider M., Hampel H., Pottel H., Valgaeren A., Hulstaert F., Vanderstichele H. (2001) CSF-phospho-tau (181P) as a promising marker for discriminating Alzheimer's disease from dementia with Lewy bodies. In: Iqbal K, Sisodia SS, Winblad B. Alzheimer's disease: Advances in etiology, pathogenesis and therapeutics. Chichester: John Wiley & Sons; p. 285-291.
Vigo-Pelfrey C., Lee D., Keim P., Lieberburg I., Schenk D.B. (1993) Characterization of β-amyloid peptide from human cerebrospinal fluid J. Neurochem. 61: 1965-1968.
Vigo-Pelfrey C., Seubert P., Barbour R, Blomquist C., Lee M., Lee D., Coria F., Chang L., Miller B., Lieberburg I., et al. (1995) Elevation of microtubule-associated protein tau in the cerebrospinal fluid of patients with Alzheimer's disease. Neurology 45: 788-793.
Wahlund L.-O., Pihlstrand E., Eriksdotter Jönhagen M. (2003) Mild cognitive impairment: experience from a memory clinic. Acta Neurol. Scand. 107 (Suppl. 179): 21-24.
Wang R, Sweeney D., Gandy S.E., Sisodia S.S. (1996) The profile of soluble amyloid beta protein in cultured cell media. Detection and quantification of amyloid beta protein and variants by immunoprecipitation-mass spectrometry. J. Biol. Chem. 271:31894-31902.
Welsh K.A., Butters N., Hughes J., Mohs R., Heyman A. (1991) Detection of abnormal memory decline in mild cases of Alzheimer's disease using CERAD neuropsychological measures. Arch. Neurol. 48: 278-281.
Wild D. (ed.) (2001) The Immunoassay Handbook 2nd edition. Nature Pr., London, UK.
Wiltfang J., Esselmann H., Cupers P., Neumann M., Kretzschmar H., Beyermann M., Schleuder D., Jahn H., Ruther E., Kornhuber J., Annaert W., De Strooper B., Saftig P. (2001) Elevation of beta-amyloid peptide 2-42 in sporadic and familial Alzheimer's disease and its generation in PS1 knockout cells. J. Biol. Chem. 276: 42645-42657.
Wiltfang J., Esselmann H., Bibl M., Smirnov A., Otto M., Paul S., Schmidt B., Klafki H.-W., Maler M., Dyrks T., Bienert M., Beyermann M., Rüther E., Kornhuber J. (2002) Highly conserved and disease-specific patterns of carboxyterminally truncated Aβ peptides 1-37/38/39 in addition to 1-40/42 in Alzheimer's disease and in patients with chronic neuroinflammation. J. Neurochem. 81: 481-496.
World Health Organization (1992) The ICD-10 Classification of Mental and Behavioral Disorders. Geneva, World Health Organization.
Yaffe K., Gregg E.W. (2002) Diabetes mellitus, cognitive function, and risk of dementia in older adults. Available at: http://www.harrisonsonline.com/server-java/Arknoid/amed/harrisons/ex editorials/ed12566 p01.html. Accessed April 26, 2002.

## Claims

1. A method to determine whether a subject has a likelihood to develop Alzheimer's disease (AD) comprising the following steps:
(a) Determining, in a body fluid sample obtained from said subject, the ratio x/y, wherein:
• x is the level of Aβ peptides capable of forming an immunological complex with an antibody that recognizes an epitope of the A β peptide containing the first amino acid (D; aspartic acid), the second amino acid (A; alanine), and/or the third amino acid (E; glutamic acid) of the Aβ peptide;
• y is the level of A β peptides capable of forming an immunological complex with an antibody that recognizes an epitope of the A β peptide not containing the first amino acid (D; aspartic acid), the second amino acid (A; alanine), and/or the third amino acid (E; glutamic acid) of the Aβ peptide;
(b) Comparing the ratio x/y obtained in (a) with a range of x/y ratios previously defined as characteristic for body fluid samples obtained from subjects that at the time of sampling did not show clinical signs of AD and that later developed AD, and with a range of x/y ratios previously defined as characteristic for body fluid samples obtained from subjects that at the time of sampling did not show clinical signs of AD and that did not develop AD;
(c) Determining, from the comparison in step (b), whether the subject has a likelihood to develop AD, whereby a ratio x/y in a range previously defined as characteristic for body fluid samples obtained from subj ects that at the time of sampling did not show clinical signs of AD and that later developed AD, is an indication that said subject has a likelihood to develop AD; and whereby a ratio x/y in a range previously defined as characteristic for body fluid samples obtained from subjects that at the time of sampling did not show clinical signs of AD and that did not develop AD, is an indication that said subject does not have a likelihood to develop AD.

2. The method according to claim1, further **characterized** that the subject is a memory-impaired individual.

3. The method according to claim 2, further **characterized** that the memory impaired individual is suffering from Mild cognitive impairment (MCI).

4. A method for the diagnosis of a subject suffering from AD and/or for the differential diagnosis of a subject suffering from AD versus a subject suffering from another dementia such as Dementia with Lewy bodies (DLB) comprising the following steps:
(a) Determining, in a body fluid sample obtained from said subject, the ratio x/y, wherein:
• x is the level of Aβ peptides capable of forming an immunological complex with an antibody that recognizes an epitope of the Aβ peptide containing the first amino acid (D; aspartic acid), the second amino acid (A; alanine), and/or the third amino acid (E; glutamic acid) of the Aβ peptide;
• y is the level of A β peptides capable of forming an immunological complex with an antibody that recognizes an epitope of the Aβ peptide not containing the first amino acid (D; aspartic acid), the second amino acid (A; alanine), and/or the third amino acid (E; glutamic acid) of the Aβ peptide;
(b) Comparing the ratio x/y obtained in (a) with a range of x/y ratios previously defined as characteristic for body fluid samples obtained from subjects diagnosed as suffering from AD, with a range of x/y ratios previously defined as characteristic for body fluid samples obtained from control subjects and with a range of x/y ratios previously defined as characteristic for body fluid samples obtained from subjects diagnosed as suffering from another dementia such as DLB;
(c) Determining, from the comparison in step (b), whether or not the subject is suffering from AD or from another dementia such as DLB, whereby a ratio x/y in a range previously defined as characteristic for body fluid samples obtained from subjects diagnosed as suffering from AD is an indication that said subject is suffering from AD; whereby a ratio x/y in a range previously defined as characteristic for body fluid samples obtained from control subjects is an indication that said subject is not suffering from AD; and whereby a ratio x/y in a range previously defined as characteristic for body fluid samples obtained from subjects diagnosed as suffering from another dementia such as DLB is an indication that said subject is suffering from another dementia such as DLB.

5. The method according to any of claims 1 to 4 further **characterized** that x is the level of A β₄₂ peptides or A β₄₃ peptides capable of forming an immunological complex with an antibody that recognizes an epitope of the Aβ peptide containing the first amino acid (D; aspartic acid), the second amino acid (A; alanine), and/or the third amino acid (E; glutamic acid) of the Aβ peptide and y is the level of A β₄₂ peptides or A β₄₃ peptides capable of forming an immunological complex with an antibody that recognizes an epitope of the A β peptide not containing the first amino acid (D; aspartic acid), the second amino acid (A; alanine), and/or the third amino acid (E; glutamic acid) of the Aβ peptide.

6. The method according to any of claims 1 to 5 further **characterized** that x is the level of A β peptides capable of forming an immunological complex with an antibody that recognizes an epitope of the A β peptide containing the first amino acid (D; aspartic acid) of the Aβ peptide.

7. The method according to any of claims 1 to 6 further **characterized** that x is the level of A β peptides capable of forming an immunological complex with the monoclonal antibody 3D6, BAN-50, and/or Anti-N1(D).

8. The method according to any of claims 1 to 7 further **characterized** that y is the level of A β peptides capable of forming an immunological complex with an antibody that recognizes an epitope of the A β peptide not containing the first amino acid (D; aspartic acid) of the Aβ peptide.

9. The method according to any of claims 1 to 8 further **characterized** that y is the level of A β peptides capable of forming an immunological complex with an antibody that recognizes an epitope different from the 3D6, BAN-50, and/or Anti-N1(D) epitope.

10. The method according to any of claims 1 to 9 further **characterized** that y is the level of A β peptides capable of forming an immunological complex with the monoclonal antibody 4G8, with the monoclonal antibody 6E10 and/or with the monoclonal antibody 10H3.

11. The method according to any of claims 1 to 10, further **characterized** that x is the level of Aβ_{(1-C)} peptides and y is the level of A β_{(N-C)} peptides.

12. The method according to claim 11, further **characterized** that x is the level of A β₍₁₋₄₂₎ and/or Aβ₍₁₋₄₃₎ peptides and y is the level of A β_{(N-42)} and/or A β_{(N-43)} peptides.

13. The method according to claim 12, further **characterized** that x is the level of A β₍₁₋₄₂) peptides and y is the level of A β_{(N-42)} peptides.

14. The method according to any of claims 1 to 11, further **characterized** that x is the level of Aβ_{(1-C)} peptides and y is the level of A β_{(11-C)} peptides.

15. The method according to claim 14, further **characterized** that x is the level of A β₍₁₋₄₂₎ and/or Aβ₍₁₋₄₃₎ peptides and y is the level of A β₍₁₁₋₄₂₎ and/or Aβ₍₁₁₋₄₃₎ peptides.

16. The method according to claim 15, further **characterized** that x is the level of A β₍₁₋₄₂) peptides and y is the level of A β₍₁₁₋₄₂₎ peptides.

17. The method according to any of claims 1 to 16, further **characterized** that the body fluid sample is a cerebrospinal fluid sample or a plasma or serum sample.

18. The method according to any of claims 1 to 17 for use in the treatment follow up of a subject that has a likelihood to develop AD or of a subject that is diagnosed as suffering from AD.

19. The method according to any of claims 1 to 18, further **characterized** that the ratio x/y is determined immunologically making use of a first antibody that specifically recognizes an epitope of the A β peptide containing the first amino acid (D; aspartic acid), the second amino acid (A; alanine), and/or the third amino acid (E; glutamic acid) of the A β peptide and making use of a second antibody that recognizes an epitope of the Aβ peptide not containing the first amino acid (D; aspartic acid), the second amino acid (A; alanine), and/or the third amino acid (E; glutamic acid) of the Aβ peptide.

20. Use of a set of antibodies for immunologically determining the ratio of X/Y in the methods of claims 1 and 4 comprising at least a first antibody that specifically recognizes an epitope of the Aβ peptide containing the first amino acid (D; aspartic acid), the second amino acid, (A; alanine) and/or the third amino acid (E; glutamic acid) of the Aβ peptide for detecting X and a second antibody that recognizes an epitope of the A peptide not containing the first amino acid (D; aspartic acid), the second amino acid (A; alanine), and/or the third amino acid (E; glutamic acid) of the Aβ peptide for determining Y.

21. Use of a diagnostic kit comprising a first antibody that specifically recognizes an epitope of the Aβ peptide containing the first amino acid (D; aspartic acid), the second amino acid (A; alanine), and/or the third amino acid (E; glutamic acid) of the Aβ peptide and a second antibody that recognizes an epitope of the Aβ peptide not containing the first amino acid (D; aspartic acid), the second amino acid (A; alanine), and/or the third amino acid (E; glutamic acid) of the Aβ peptide for immunologically determining the ratio of X/Y in the methods of claims 1 and 4.

22. Use of a diagnostic kit according to claim 21, further **characterized** that it is a multiparameter assay for the simultaneous detection of the levels of different Aβ peptides.

23. Use of a diagnostic kit according to claim 22, further **characterized in that** it uses the xMap™ technology.

24. The method according to claim 19, the use of a set of antibodies according to claim 20, or the use of a diagnostic kit according to any of claims 21 to 23, further **characterized** that the first antibody specifically recognizes an epitope of the Aβ peptide containing the first amino acid (D; aspartic acid) of the Aβ peptide.

25. The method according to claim 19, the use of a set of antibodies according to claim 20, or the use of a diagnostic kit according to any of claims 21 to 23, further **characterized** that the first antibody is the monoclonal antibody 3D6, BAN-50 or Anti-N1(D).

26. The method according to claim 19, the use of a set of antibodies according to claim 20, or the use of a diagnostic kit according to any of claims 21 to 23, further **characterized** that the second antibody recognizes an epitope of the Aβ peptide not containing the first amino acid (D; aspartic acid) of the Aβ peptide.

27. The method according to claim 19, the use of a set of antibodies according to claim 20, or the use of a diagnostic kit according to any of claims 21 to 23, further **characterized** that the first antibody specifically recognizes an epitope of the Aβ peptide containing the first amino acid (D; aspartic acid) of the Aβ peptide and the second antibody recognizes an epitope of the Aβ peptide not containing the first amino acid (D; aspartic acid) of the Aβ peptide.

28. The method according to claim 19, the use of a set of antibodies according to claim 20, or the use of a diagnostic kit according to any of claims 21 to 23, further **characterized** that the first antibody is the monoclonal antibody 3D6, BAN-50 or Anti-N1(D) and the second antibody recognizes an epitope of the Aβ peptide not containing the first amino acid D; aspartic acid) of the Aβ peptide.

29. The method according to claim 19, the use of a set of antibodies according to claim 20, or the use of a diagnostic kit according to any of claims 21 to 23, further **characterized** that the second antibody recognizes an epitope of the Aβ peptide different from the 3D6, BAN-50, and/or Anti-N1(D) epitope.

30. The method according to claim 19, the use of a set of antibodies according to claim 20, or the use of a diagnostic kit according to any of claims 21 to 23, further **characterized** that the first antibody specifically recognizes an epitope of the Aβ peptide containing the first amino acid (D; aspartic acid) of the Aβ peptide and the second antibody recognizes an epitope of the Aβ peptide different from the 3D6, BAN-50, and/or Anti-N1(D) epitope.

31. The method according to claim 19, the use of a set of antibodies according to claim 20, or the use of a diagnostic kit according to any of claims 21 to 23, further **characterized** that the first antibody is the monoclonal antibody 3D6, BAN-50, or Anti-N1(D) and the second antibody recognizes an epitope of the Aβ peptide different from the 3D6, BAN-50, and/or Anti-N1(D) epitope.

## Patentansprüche

1. Verfahren zur Bestimmung, ob ein Patient eine Wahrscheinlichkeit zur Entwicklung von AD (Morbus Alzheimer) aufweist, welches die folgenden Schritte umfasst:
(a) Bestimmen, in einer aus dem Patienten erhaltenen Körperflüssigkeitsprobe, des Verhältnisses x/y, wobei:
• x der Spiegel von Aβ-Peptiden ist, fähig zur Bildung eines immunologischen Komplexes mit einem Antikörper, der ein Epitop des Aβ-Peptids erkennt, welches die erste Aminosäure (D, Asparaginsäure), die zweite Aminosäure (A; Alanin) und/oder die dritte Aminosäure (E; Glutaminsäure) des Aβ-Peptids enthält;
• y der Spiegel von Aβ-Peptiden ist, fähig zur Bildung eines immunologischen Komplexes mit einem Antikörper, der ein Epitop des Aβ-Peptids erkennt, welches die erste Aminosäure (D, Asparaginsäure), die zweite Aminosäure (A; Alanin) und/oder die dritte Aminosäure (E; Glutaminsäure) des Aβ-Peptids nicht enthält;
(b) Vergleichen des in (a) erhaltenen Verhältnisses x/y mit einem Bereich von x/y-Verhältnissen, welcher zuvor als charakteristisch für Körperflüssigkeitsproben definiert worden war, erhalten aus Patienten, welche zur Zeit der Probennahme keine klinischen Anzeichen von AD zeigten und welche später AD entwickelten, und mit einem Bereich von x/y-Verhältnissen, welcher zuvor als charakteristisch für Körperflüssigkeitsproben definiert worden war, erhalten aus Patienten, welche zur Zeit der Probennahme keine klinischen Anzeichen von AD zeigten und welche AD nicht entwickelten;
(c) Bestimmen, aus dem Vergleich in Schritt (b), ob der Patient eine Wahrscheinlichkeit zur Entwicklung von AD aufweist, wobei ein Verhältnis x/y in einem Bereich, welcher zuvor als charakteristisch für Körperflüssigkeitsproben definiert worden war, erhalten aus Patienten, welche zur Zeit der Probennahme keine klinischen Anzeichen von AD zeigten und welche später AD entwickelten, ein Hinweis ist, dass der Patient eine Wahrscheinlichkeit zum Entwickeln von AD aufweist; und wobei ein Verhältnis x/y in einem Bereich, welcher zuvor als charakteristisch für Körperflüssigkeitsproben definiert worden war, erhalten aus Patienten, welche zur Zeit der Probennahme keine klinischen Anzeichen von AD zeigten und welche AD nicht entwickelten, ein Hinweis ist, dass der Patient keine Wahrscheinlichkeit zum Entwickeln von AD aufweist.

2. Verfahren gemäß Anspruch 1, ferner **gekennzeichnet dadurch, dass** der Patient ein hinsichtlich des Gedächtnisses beeinträchtigtes Individuum ist.

3. Verfahren gemäß Anspruch 2, ferner **gekennzeichnet dadurch, dass** das hinsichtlich des Gedächtnisses beeinträchtigte Individuum an MCI (milde kognitive Beeinträchtigung) leidet.

4. Verfahren für die Diagnose eines Patienten, der an AD leidet und/oder für die differentielle Diagnose eines Patienten, der an AD leidet, gegenüber einem Patienten, der an einer anderen Demenz, wie DLB (Demenz mit Lewis-Körperchen), leidet, welches die folgenden Schritte umfasst:
(a) Bestimmen, in einer aus dem Patienten erhaltenen Körperflüssigkeitsprobe, des Verhältnisses x/y, wobei:
• x der Spiegel von Aβ-Peptiden ist, fähig zur Bildung eines immunologischen Komplexes mit einem Antikörper, der ein Epitop des Aβ-Peptids erkennt, welches die erste Aminosäure (D, Asparaginsäure), die zweite Aminosäure (A; Alanin) und/oder die dritte Aminosäure (E; Glutaminsäure) des Aβ-Peptids enthält;
• y der Spiegel von Aβ-Peptiden ist, fähig zur Bildung eines immunologischen Komplexes mit einem Antikörper, der ein Epitop des Aβ-Peptids erkennt, welches die erste Aminosäure (D, Asparaginsäure), die zweite Aminosäure (A; Alanin) und/oder die dritte Aminosäure (E; Glutaminsäure) des Aβ-Peptids nicht enthält;
(b) Vergleichen des in (a) erhaltenen Verhältnisses x/y mit einem Bereich von x/y-Verhältnissen, welcher zuvor als charakteristisch für Körperflüssigkeitsproben definiert worden war, die aus Patienten erhalten wurden, welche als an AD leidend diagnostiziert wurden, mit einem Bereich von x/y-Verhältnissen, welcher zuvor als charakteristisch für Körperflüssigkeitsproben definiert worden war, die aus Kontroll-Patienten erhalten wurden, und mit einem Bereich von x/y-Verhältnissen, welcher zuvor als charakteristisch für Körperflüssigkeitsproben definiert worden war, die aus Patienten erhalten wurden, welche als an einer anderen Demenz, wie DLB, leidend diagnostiziert wurden;
(c) Bestimmen, aus dem Vergleich in Schritt (b), ob oder ob nicht der Patient an AD oder an einer anderen Demenz, wie DLB, leidet, wobei ein Verhältnis x/y in einem Bereich, welcher zuvor als charakteristisch für Körperflüssigkeitsproben definiert worden war, die aus Patienten erhalten wurden, welche als an AD leidend diagnostiziert wurden, ein Hinweis dafür ist, dass der Patient an AD leidet; wobei ein Verhältnis x/y in einem Bereich, welcher zuvor als charakteristisch für Körperflüssigkeitsproben definiert worden war, die aus Kontroll-Patienten erhalten wurden, ein Hinweis dafür ist, dass der Patient nicht an AD leidet; und wobei ein Verhältnis x/y in einem Bereich, welcher zuvor als charakteristisch für Körperflüssigkeitsproben definiert worden war, die aus Patienten erhalten wurden, welche als an einer anderen Demenz, wie DLB, leidend diagnostiziert wurden, ein Hinweis dafür ist, dass der Patient an einer anderen Demenz, wie DLB, leidet.

5. Verfahren gemäß mindestens einem der Ansprüche 1 bis 4, ferner **dadurch gekennzeichnet, dass** x der Spiegel von Aβ₄₂-Peptiden oder Aβ₄₃-Peptiden ist, fähig zur Bildung eines immunologischen Komplexes mit einem Antikörper, der ein Epitop des Aβ-Peptids erkennt, welches die erste Aminosäure (D, Asparaginsäure), die zweite Aminosäure (A; Alanin) und/oder die dritte Aminosäure (E; Glutaminsäure) des Aβ-Peptids enthält, und y der Spiegel von Aβ₄₂-Peptiden oder Aβ₄₃-Peptiden ist, fähig zur Bildung eines immunologischen Komplexes mit einem Antikörper, der ein Epitop des Aβ-Peptids erkennt, welches die erste Aminosäure (D, Asparaginsäure), die zweite Aminosäure (A; Alanin) und/oder die dritte Aminosäure (E; Glutaminsäure) des Aβ-Peptids nicht enthält.

6. Verfahren gemäß mindestens einem der Ansprüche 1 bis 5, ferner **dadurch gekennzeichnet, dass** x der Spiegel von Aβ-Peptiden ist, fähig zur Bildung eines immunologischen Komplexes mit einem Antikörper, der ein Epitop des Aβ-Peptids erkennt, welches die erste Aminosäure (D, Asparaginsäure) des Aβ-Peptids enthält.

7. Verfahren gemäß mindestens einem der Ansprüche 1 bis 6, ferner **dadurch gekennzeichnet, dass** x der Spiegel von Aβ-Peptiden ist, fähig zur Bildung eines immunologischen Komplexes mit dem monoklonalen Antikörper 3D6, BAN-50 und/oder Anti-N1(D).

8. Verfahren gemäß mindestens einem der Ansprüche 1 bis 7, ferner **dadurch gekennzeichnet, dass** y der Spiegel von Aβ-Peptiden ist, die zur Bildung eines immunologischen Komplexes mit einem Antikörper fähig sind, der ein Epitop des Aβ-Peptids erkennt, welches die erste Aminosäure (D, Asparaginsäure) des A**ß**Peptids nicht enthält.

9. Verfahren gemäß mindestens einem der Ansprüche 1 bis 8, ferner **dadurch gekennzeichnet, dass** y der Spiegel von Aβ-Peptiden ist, fähig zur Bildung eines immunologischen Komplexes mit einem Antikörper, der ein anderes Epitop erkennt als das 3D6-, BAN-50- und/oder Anti-N1(D)-Epitop.

10. Verfahren gemäß mindestens einem der Ansprüche 1 bis 9, ferner **dadurch gekennzeichnet, dass** y der Spiegel von Aβ-Peptiden ist, die zur Bildung eines immunologischen Komplexes mit dem monoklonalen Antikörper 4G8, mit dem monoklonalen Antikörper 6E10 und/oder mit dem monoklonalen Antikörper 10H3 fähig sind.

11. Verfahren gemäß mindestens einem der Ansprüche 1 bis 10, das ferner **dadurch gekennzeichnet ist, dass** x der Spiegel von Aβ_{(1-C)}-Peptiden ist und y der Spiegel von Aβ_{(N-C)}-Peptiden ist.

12. Verfahren gemäß Anspruch 11, das ferner **dadurch gekennzeichnet ist, dass** x der Spiegel von Aβ₍₁₋₄₂₎- und/oder Aβ₍₁₋₄₃₎-Peptiden ist und y der Spiegel von Aβ_{(N-42)}- und/oder Aβ_{(N-43)}-Peptiden ist.

13. Verfahren gemäß Anspruch 12, ferner **dadurch gekennzeichnet, dass** x der Spiegel von Aβ₍₁₋₄₂)-Peptiden ist und y der Spiegel von Aβ_{(N-42)}-Peptiden ist.

14. Verfahren gemäß mindestens einem der Ansprüche 1 bis 11, ferner **dadurch gekennzeichnet, dass** x der Spiegel von Aβ_{(1-C)}-Peptiden ist und y der Spiegel von Aβ_{(11-C)}-Peptiden ist.

15. Verfahren gemäß Anspruch 14, das ferner **dadurch gekennzeichnet ist, dass** x der Spiegel von Aβ₍₁₋₄₂₎- und/oder Aβ₍₁₋₄₃₎-Peptiden ist und y der Spiegel von Aβ₍₁₁₋₄₂₎- und/oder Aβ₍₁₁₋₄₃₎-Peptiden ist.

16. Verfahren gemäß Anspruch 15, das ferner **dadurch gekennzeichnet ist, dass** x der Spiegel von Aβ₍₁₋₄₂₎-Peptiden ist und y der Spiegel von Aβ₍₁₁₋₄₂₎-Peptiden ist.

17. Verfahren gemäß mindestens einem der Ansprüche 1 bis 16, das ferner **dadurch gekennzeichnet ist, dass** die Körperflüssigkeitsprobe eine Liquorprobe oder eine Plasma- oder Serumprobe ist.

18. Verfahren gemäß mindestens einem der Ansprüche 1 bis 17 zur Verwendung in der Behandlungs-Nachuntersuchung eines Patienten, der eine Wahrscheinlichkeit zum Entwickeln von AD aufweist, oder eines Patienten, der als an AD leidend diagnostiziert wurde.

19. Verfahren gemäß mindestens einem der Ansprüche 1 bis 18, ferner **dadurch gekennzeichnet, dass** das Verhältnis x/y immunologisch bestimmt wird, wobei man einen ersten Antikörper verwendet, der spezifisch ein Epitop des Aβ-Peptids erkennt, welches die erste Aminosäure (D, Asparaginsäure), die zweite Aminosäure (A; Alanin) und/oder die dritte Aminosäure (E; Glutaminsäure) des Aß-Peptids enthält, und einen zweiten Antikörper verwendet, der ein Epitop des Aß-Peptids erkennt, welches die erste Aminosäure (D, Asparaginsäure), die zweite Aminosäure (A; Alanin) und/oder die dritte Aminosäure (E; Glutaminsäure) des Aβ-Peptids nicht enthält.

20. Satz von Antikörpern, umfassend mindestens einen ersten Antikörper, der spezifisch ein Epitop des Aβ-Peptids erkennt, welches die erste Aminosäure (D, Asparaginsäure), die zweite Aminosäure (A; Alanin) und/oder die dritte Aminosäure (E; Glutaminsäure) des A**ß**Peptids enthält, und einen zweiten Antikörper, der ein Epitop des Aβ-Peptids erkennt, welches die erste Aminosäure (D, Asparaginsäure), die zweite Aminosäure (A; Alanin) und/oder die dritte Aminosäure (E; Glutaminsäure) des Aβ-Peptids nicht enthält, zur Verwendung bei der Bestimmung, ob ein Patient eine Wahrscheinlichkeit aufweist, AD zu entwickeln, für die Diagnose eines Patienten, der an AD leidet, und/oder für die differentielle Diagnose eines Patienten, der an AD leidet, gegenüber einem Patienten, der an einer anderen Demenz, wie DLB, leidet.

21. Die Verwendung einem diagnostischer Kit umfassend einen ersten Antikörpern, der speczifisch ein Epitop des Aß-Peptids erkennt, welches die erste Aminosäure (D; Asparaginsäure), die zweite Aminosäure (A; Alanin), und/oder die dritte Aminosäure (E; Glutaminsäure) des Aβ-Peptids enthält, und einen zweiten Antikörper, der ein Epitop des Aβ-Peptids erkennt, welches die erste Aminosäure (D; Asparaginsäure), die zweite Aminosäure (A; Alanin), und/oder die dritte Aminosäure (E; Glutaminsäure) des Aβ-Peptids nicht enthält für die immunogischer Ermittlung die Verhaltnis x/y in die Methoden von Patentansprüche 1 und 4.

22. Die Verwendung einem diagnostischer Kit gemäß der Ansprüche 21, ferner **dadurch gekennzeichnet, dass** er ein Multiparameter-Assay für den gleichzeitigen Nachweis der Spiegel von verschiedenen Aβ-Peptiden ist.

23. Die Verwendung einem diagnostischer Kit gemäß Anspruch 22, ferner **dadurch gekennzeichnet, dass** er die xMap™-Technologie anwendet.

24. Verfahren gemäß Anspruch 19, die Verwendung einer Satz von Antikörpern gemäß Anspruch 20 oder die Verwendung einem diagnostischer Kit gemäß mindestens einem der Ansprüche 21 bis 23, ferner **dadurch gekennzeichnet, dass** der erste Antikörper spezifisch ein Epitop des Aβ-Peptids erkennt, welches die erste Aminosäure (D, Asparaginsäure) des Aβ-Peptids enthält.

25. Verfahren gemäß Anspruch 19, die Verwendung einer Satz von Antikörpern gemäß Anspruch 20 oder die Verwendung einem diagnostischer Kit gemäß mindestens einem der Ansprüche 21 bis 23, ferner **dadurch gekennzeichnet, dass** der erste Antikörper der monoklonale Antikörper 3D6, BAN-50 oder Anti-N1(D) ist.

26. Verfahren gemäß Anspruch 19, die Verwendung einer Satz von Antikörpern gemäß Anspruch 20 oder die Verwendung einem diagnostischer Kit gemäß mindestens einem der Ansprüche 21 bis 23, ferner **dadurch gekennzeichnet, dass** der zweite Antikörper ein Epitop des Aβ-Peptids erkennt, welches die erste Aminosäure (D, Asparaginsäure) des Aβ-Peptids nicht enthält.

27. Verfahren gemäß Anspruch 19, die Verwendung einer Satz von Antikörpern gemäß Anspruch 20 oder die Verwendung einem diagnostischer Kit gemäß mindestens einem der Ansprüche 21 bis 23, ferner **dadurch gekennzeichnet, dass** der erste Antikörper spezifisch ein Epitop des Aβ-Peptids erkennt, welches die erste Aminosäure (D, Asparaginsäure) des Aβ-Peptids enthält, und der zweite Antikörper ein Epitop des Aβ-Peptids erkennt, welches die erste Aminosäure (D, Asparaginsäure) des Aβ-Peptids nicht enthält.

28. Verfahren gemäß Anspruch 19, die Verwendung einer Satz von Antikörpern gemäß Anspruch 20 oder die Verwendung einem diagnostischer Kit gemäß mindestens einem der Ansprüche 21 bis 23, ferner **dadurch gekennzeichnet, dass** der erste Antikörper der monoklonale Antikörper 3D6, BAN-50 oder Anti-N1(D) ist, und der zweite Antikörper ein Epitop des Aβ-Peptids erkennt, welches die erste Aminosäure (D, Asparaginsäure) des Aβ-Peptids nicht enthält.

29. Verfahren gemäß Anspruch 19, die Verwendung einer Satz von Antikörpern gemäß Anspruch 20 oder die Verwendung einem diagnostischer Kit gemäß mindestens einem der Ansprüche 21 bis 23, ferner **dadurch gekennzeichnet, dass** der zweite Antikörper ein anderes Epitop des Aβ-Peptids erkennt als das 3D6-, BAN-50- und/oder Anti-N1(D)-Epitop.

30. Verfahren gemäß Anspruch 19, die Verwendung einer Satz von Antikörpern gemäß Anspruch 20 oder die Verwendung einem diagnostischer Kit gemäß mindestens einem der Ansprüche 21 bis 23, ferner **dadurch gekennzeichnet, dass** der erste Antikörper spezifisch ein Epitop des Aβ-Peptids erkennt, welches die erste Aminosäure (D, Asparaginsäure) des Aβ-Peptids enthält, und der zweite Antikörper ein anderes Epitop des Aβ-Peptids erkennt als das 3D6-, BAN-50- und/oder Anti-N1(D)-Epitop.

31. Verfahren gemäß Anspruch 19, die Verwendung einer Satz von Antikörpern gemäß Anspruch 20 oder die Verwendung einem diagnostischer Kit gemäß mindestens einem der Ansprüche 21 bis 23, ferner **dadurch gekennzeichnet, dass** der erste Antikörper der monoklonale Antikörper 3D6, BAN-50 oder Anti-N1(D) ist, und der zweite Antikörper ein anderes Epitop des Aβ-Peptids erkennt als das 3D6-, BAN-50- und/oder Anti-N1(D)-Epitop.

## Revendications

1. Procédé pour déterminer si un sujet présente une probabilité de développer la maladie d'Alzheimer comprenant les étapes suivantes .
(a) Détermination, dans un échantillon de fluide corporel obtenu dudit sujet, du rapport x/y, où
• x est le niveau de peptides Aβ capables de former un complexe immunologique avec un anticorps qui reconnaît un épitope du peptide Aβ contenant le premier acide aminé (D ; acide aspartique), le deuxième acide aminé (A ; alanine), et/ou le troisième acide aminé (E ; acide glutamique) du peptide Aβ ;
• y est le niveau de peptides Aβ capables de former un complexe immunologique avec un anticorps qui reconnaît un épitope du peptide Aβ ne contenant pas le premier acide aminé (D ; acide aspartique), ni le deuxième acide aminé (A ; alanine), et/ou le troisième acide aminé (E ; acide glutamique) du peptide Aβ ;
(b) Comparaison du rapport x/y obtenu dans (a) avec une plage de rapports x/y définie précédemment comme caractéristique pour des échantillons de fluide corporel obtenus de sujets qui, au moment de l'échantillonnage, ne présentaient pas de signes cliniques de la maladie d'Alzheimer et qui ont développé ultérieurement la maladie d'Alzheimer, et avec une plage de rapports x/y définie précédemment comme caractéristique pour des échantillons de fluide corporel obtenus de sujets qui, au moment de l'échantillonnage ne présentaient pas de signes cliniques de la maladie d'Alzheimer et qui n'ont pas développé la maladie d'Alzheimer ;
(c) Détermination, à partir de la comparaison dans l'étape (b), si le sujet présente une probabilité de développer la maladie d'Alzheimer, un rapport x/y dans une plage définie précédemment comme caractéristique pour des échantillons de fluide corporel obtenus de sujets qui, au moment de l'échantillonnage, ne présentaient pas de signes cliniques de la maladie d'Alzheimer et qui ont développé ultérieurement la maladie d'Alzheimer, étant une indication que le sujet présente une probabilité de développer la maladie d'Alzheimer ; et un rapport x/y dans une plage définie précédemment comme caractéristique pour des échantillons de fluide corporel obtenus de sujets qui, au moment de l'échantillonnage ne présentaient pas de signes cliniques de la maladie d'Alzheimer et qui n'ont pas développé la maladie d'Alzheimer étant une indication que ledit sujet ne présente pas de probabilité de développer la maladie d'Alzheimer.

2. Procédé selon la revendication 1, **caractérisé en ce que** le sujet est un individu souffrant de troubles de la mémoire.

3. Procédé selon la revendication 2, **caractérisé en ce que** l'individu souffrant de troubles de la mémoire souffre de troubles cognitifs modérés (TCM).

4. Procédé pour le diagnostic d'un sujet souffrant de la maladie d'Alzheimer et/ou pour le diagnostic différentiel d'un sujet souffrant de la maladie d'Alzheimer par rapport à un sujet souffrant d'une autre forme de démence telle que la démence avec des corps de Lewis (DCL) comprenant les étapes suivantes :
(a) Détermination, dans un fluide corporel obtenu dudit sujet, du rapport x/y, où
• x est le niveau de peptides Aβ capables de former un complexe immunologique avec un anticorps qui reconnaît un épitope du peptide Aβ contenant le premier acide aminé (D ; acide aspartique), le deuxième acide aminé (A ; alanine), et/ou le troisième acide aminé (E ; acide glutamique) du peptide Aβ ;
• y est le niveau de peptides Aβ capables de former un complexe immunologique avec un anticorps qui reconnaît un épitope du peptide Aβ ne contenant pas le premier acide aminé (D ; acide aspartique), ni le deuxième acide aminé (A ; alanine), et/ou le troisième acide aminé (E ; acide glutamique) du peptide Aβ ;
(b) Comparaison du rapport x/y obtenu dans (a) avec une plage de rapports x/y définie précédemment comme caractéristique pour des échantillons de fluide corporel obtenus de sujets diagnostiqués comme souffrant de la maladie d'Alzheimer, avec une plage de rapports x/y définie précédemment comme caractéristique pour des échantillons de fluide corporel obtenus de sujets témoins et avec une plage de rapports x/y définie précédemment comme caractéristique pour des échantillons de fluide corporel obtenus de sujets diagnostiqués comme souffrant d'une autre forme de démence telle que la DCL ;
(c) Détermination, à partir de la comparaison dans l'étape (b), si le sujet souffre ou non de la maladie d'Alzheimer ou d'une autre forme de démence telle que la DCL, un rapport x/y dans une plage définie précédemment comme caractéristique pour des échantillons de fluide corporel obtenus de sujets diagnostiqués comme souffrant de la maladie d'Alzheimer étant une indication que ledit sujet souffre de la maladie d'Alzheimer ; un rapport x/y dans une plage définie précédemment comme caractéristique pour des échantillons de fluide corporel obtenus de sujets témoins étant une indication que ledit sujet ne souffre pas de la maladie d'Alzheimer ; et un rapport x/y dans une plage définie précédemment comme caractéristique pour des échantillons de fluide corporel obtenus de sujets diagnostiqués comme souffrant d'une autre forme de démence telle que la DCL étant une indication que ledit sujet souffre d'une autre forme de démence telle que le DCL.

5. Procédé selon l'une quelconque des revendications 1 à 4, **caractérisé en ce que** x est le niveau de peptides Aβ₄₂ ou de peptides Aβ₄₃ capables de former un complexe immunologique avec un anticorps qui reconnaît un épitope du peptide Aβ contenant le premier acide aminé (D ; acide aspartique), le deuxième acide aminé (A ; alanine), et/ou le troisième acide aminé (E; acide glutamique) du peptide Aβ et y est le niveau de peptides Aβ₄₂ ou de peptides Aβ₄₃ capables de former un complexe immunologique avec un anticorps qui reconnaît un épitope du peptide Aβ ne contenant pas le premier acide aminé (D ; acide aspartique) ni le deuxième acide aminé (A ; alanine), et/ou le troisième acide aminé (E ; acide glutamique) du peptide Aβ.

6. Procédé selon l'une quelconque des revendications 1 à 5, **caractérisé en ce que** x est le niveau de peptides Aβ capables de former un complexe immunologique avec un anticorps qui reconnaît un épitope du peptide Aβ contenant le premier acide aminé (D ; acide aspartique) du peptide Aβ.

7. Procédé selon l'une quelconque des revendications 1 à 6, **caractérisé en ce que** x est le niveau de peptides Aβ capables de former un complexe immunologique avec l'anticorps monoclonal 3D6, BAN-50, et/ou Anti-N1(D).

8. Procédé selon l'une quelconque des revendications 1 à 7, **caractérisé en ce que** y est le niveau de peptides Aβ capables de former un complexe immunologique avec un anticorps qui reconnaît un épitope du peptide Aβ ne contenant pas le premier acide aminé (D ; acide aspartique) du peptide Aβ.

9. Procédé selon l'une quelconque des revendications 1 à 8, **caractérisé en ce que** y est le niveau de peptides Aβ capables de former un complexe immunologique avec un anticorps qui reconnaît un épitope différent de 3D6, BAN-50, et/ou Anti-N1(D).

10. Procédé selon l'une quelconque des revendications 1 à 9, **caractérisé en ce que** y est le niveau de peptides Aβ capables de former un complexe immunologique avec l'anticorps monoclonal 4G8, avec l'anticorps monoclonal 6E10 et/ou avec l'anticorps monoclonal 10H3.

11. Procédé selon l'une quelconque des revendications 1 à 10, **caractérisé en ce que** x est le niveau de peptides Aβ_{(1-C)} et y est le niveau de peptides Aβ_{(N-C)}.

12. Procédé selon la revendication 11, **caractérisé en ce que** x est le niveau de peptides Aβ₍₁₋₄₂₎ et/ou Aβ₍₁₋₄₃₎ et y est le niveau de peptides Aβ_{(N-42)} et/ou Aβ_{(N-43)}.

13. Procédé selon la revendication 12, **caractérisé en ce que** x est le niveau de peptides Aβ₍₁₋₄₂₎ et y est le niveau de peptides Aβ_{(N-42)}.

14. Procédé selon l'une quelconque des revendications 1 à 11, **caractérisé en ce que** x est le niveau de peptides Aβ_{(1-C)} et y est le niveau de peptides Aβ_{(11-C)}.

15. Procédé selon la revendication 14, **caractérisé en ce que** x est le niveau de peptides Aβ₍₁₋₄₂₎ et/ou Aβ₍₁₋₄₃₎ et y est le niveau de peptides Aβ₍₁₁₋₄₂₎ et/ou Aβ₍₁₁₋₄₃₎.

16. Procédé selon la revendication 15, **caractérisé en ce que** x est le niveau de peptides Aβ₍₁₋₄₂₎ et y est le niveau de peptides Aβ₍₁₁₋₄₂₎.

17. Procédé selon l'une quelconque des revendications 1 à 16, **caractérisé en ce que** l'échantillon de fluide corporel est un échantillon de fluide cérébrospinal ou de plasma ou de sérum.

18. Procédé selon l'une quelconque des revendications 1 à 17 destiné à une utilisation dans le suivi du traitement d'un sujet qui présente une probabilité de développer la maladie d'Alzheimer ou d'un sujet qui est diagnostiqué comme souffrant de la maladie d'Alzheimer.

19. Procédé selon l'une quelconque des revendications 1 à 18, **caractérisé en ce que** le rapport x/y est déterminé immunologiquement en utilisant un premier anticorps qui reconnaît spécifiquement un épitope du peptide Aβ contenant le premier acide aminé (D ; acide aspartique), le deuxième acide aminé (A ; alanine), et/ou le troisième acide aminé (E ; acide glutamique) du peptide Aβ et en utilisant un deuxième anticorps qui reconnaît un épitope du peptide Aβ ne contenant pas le premier acide aminé (D ; acide aspartique), ni le deuxième acide aminé (A ; alanine), ni le troisième acide aminé (E ; acide glutamique) du peptide Aβ.

20. L'utilisation d'une série d'anticorps pour déterminer immunologiquement le rapport de x/y dans les méthodes des revendications 1 et 4 comprenant au moins un premier anticorps qui reconnaît spécifiquement un épitope du peptide Aβ contenant le premier acide aminé (D ; acide aspartique), le deuxième acide aminé (A ; alanine), et/ou le troisième acide aminé (E ; acide glutamique) du peptide Aβ pour découvrir x et un deuxième anticorps qui reconnaît un épitope du peptide A ne contenant pas le premier acide aminé (D ; acide aspartique), ni le deuxième acide aminé (A ; alanine), et/ou le troisième acide aminé (E ; acide glutamique) due peptide Aβ pour déterminer y.

21. L'utilisation d'un kit de diagnostic comprenant un premier anticorps qui reconnaît spécifiquement un épitope du peptide Aβ contenant le premier acide aminé (D ; acide aspartique), le deuxième acide aminé (A ; alanine), et/ou le troisième acide aminé (E ; acide glutamique) du peptide Aβ et un deuxième anticorps qui reconnaît un épitope du peptide Aβ ne contenant pas le premier acide aminé (D ; acide aspartique), ni le deuxième acide aminé (A ; alanine), et/ou le troisième acide aminé (E ; acide glutamique) du peptide Aβ pour déterminer immunologiquement le rapport de x/y dans les méthodes des revendications 1 et 4.

22. L'utilisation d'un kit de diagnostic selon la revendication 21, **caractérisé en ce qu'**il s'agit d'un essai à paramètres multiples pour la détection simultanée des niveaux de différents peptides Aβ.

23. L'utilisation d'un kit de diagnostic selon la revendication 22, **caractérisé en ce qu'**il utilise la technologie xMap™.

24. Procédé selon la revendication 19, l'utilisation d'une série d'anticorps selon la revendication 20, ou l'utilisation d'un kit de diagnostic selon l'une quelconque des revendications 21 à 23, **caractérisés en ce que** le premier anticorps reconnaît spécifiquement un épitope du peptide Aβ contenant le premier acide aminé (D ; acide aspartique) du peptide Aβ.

25. Procédé selon la revendication 19, l'utilisation d'une série d'anticorps selon la revendication 20, ou l'utilisation d'un kit de diagnostic selon l'une quelconque des revendications 21 à 23, **caractérisés en ce que** le premier anticorps est l'anticorps monoclonal 3D6, BAN-50 ou Anti-N1(D).

26. Procédé selon la revendication 19, l'utilisation d'une série d'anticorps selon la revendication 20, ou l'utilisation d'un kit de diagnostic selon l'une quelconque des revendications 21 à 23, **caractérisés en ce que** le deuxième anticorps reconnaît un épitope du peptide Aβ ne contenant pas le premier acide aminé (D ; acide aspartique) du peptide Aβ.

27. Procédé selon la revendication 19, l'utilisation d'une série d'anticorps selon la revendication 20, ou l'utilisation d'un kit de diagnostic selon l'une quelconque des revendications 21 à 23, **caractérisés en ce que** le premier anticorps reconnaît un épitope du peptide Aβ contenant le premier acide aminé (D; acide aspartique) du peptide Aβ et le deuxième anticorps reconnaît un épitope du peptide Aβ ne contenant pas le premier acide aminé (D ; acide aspartique) du peptide Aβ.

28. Procédé selon la revendication 19, l'utilisation d'une série d'anticorps selon la revendication 20, ou l'utilisation d'un kit de diagnostic selon l'une quelconque des revendications 21 à 23, **caractérisés en ce que** le premier anticorps est l'anticorps monoclonal 3D6, BAN-50 ou anti-N1(D) et le deuxième anticorps reconnaît un épitope du peptide Aβ ne contenant pas le premier acide aminé (D ; acide aspartique) du peptide Aβ.

29. Procédé selon la revendication 19, l'utilisation d'une série d'anticorps selon la revendication 20, ou l'utilisation d'un kit de diagnostic selon l'une quelconque des revendications 21 à 23, **caractérisés en ce que** le deuxième anticorps reconnaît un épitope du peptide Aβ différent de l'épitope 3D6, BAN-50, et/ou Anti-N1(D).

30. Procédé selon la revendication 19, l'utilisation d'une série d'anticorps selon la revendication 20, ou l'utilisation d'un kit de diagnostic selon l'une quelconque des revendications 21 à 23, **caractérisés en ce que** le premier anticorps reconnaît spécifiquement un épitope du peptide Aβ contenant le premier acide aminé (D ; acide aspartique) du peptide Aβ et le deuxième anticorps reconnaît un épitope du peptide Aβ différent de l'épitope 3D6, BAN-50 et/ou Anti-N1(D).

31. Procédé selon la revendication 19, l'utilisation d'une série d'anticorps selon la revendication 20, ou l'utilisation d'un kit de diagnostic selon l'une quelconque des revendications 21 à 23, **caractérisés en ce que** le premier anticorps est l'anticorps monoclonal 3D6, BAN-50 ou Anti-N1(D) et le deuxième anticorps reconnaît un épitope du peptide Aβ différent de l'épitope 3D6, BAN-50 ou Anti-N1(D).
